# EUROPEAN PATENT SPECIFICATION

(11) **EP 3 234 135 B1**
(45) Date of publication and mention of the grant of the patent: **29.04.2020**
(21) Application number: 15828530.4
(22) Date of filing: 18.12.2015
(51) Int. Cl.: C12N 15/113, A61P 29/00, A61P 37/00, A61P 35/00

(54) **IMMUNOMODULATION BY CONTROLLING ELR+ PROINFLAMMATORY CHEMOKINE LEVELS WITH THE LONG NON-CODING RNA UMLILO**
IMMUNMODULATION DURCH KONTROLLE DER PROINFLAMMATORISCHEN ELR+CHEMOKINSPIEGEL MIT LONG-NON-CODING-RNA-UMLILO
IMMUNOMODULATION PAR CONTRÔLE DES TAUX DE CHIMIOKINES PRO-INFLAMMATOIRES ELR+ AU MOYEN DU LONG ARN NON CODANT UMLILO

(30) Priority: 18.12.2014 ZA 201409351
(43) Date of publication of application: 25.10.2017
(73) Proprietor: CSIR, 0184 Pretoria (ZA)
(72) Inventor: DALLA, Emiliano, 34138 Trieste (TS) (IT); MHLANGA, Musa M, Cape Town (ZA); FANUCCHI, Stephanie, 2007 Johannesburg (ZA); SHIBAYAMA, Youtaro, Kanagawa-ken 230-0062 (JP)
(74) Representative: D Young & Co LLP
(86) International application number: PCT/IB2015/059783
(87) International publication number: WO 2016/098071

(56) References cited:
- WO-A1-2013/082017
- WO-A1-2015/033293
- WO-A2-2011/159685
- US-A1- 2014 056 929
- HACER KARATAS ET AL: "High-Affinity, Small-Molecule Peptidomimetic Inhibitors of MLL1/WDR5 Protein-Protein Interaction", JOURNAL OF THE AMERICAN CHEMICAL SOCIETY, vol. 135, no. 2, 16 January 2013 (2013-01-16), pages 669-682, XP055258413, US ISSN: 0002-7863, DOI: 10.1021/ja306028q
- CAO FANG ET AL: "Targeting MLL1 H3K4 Methyltransferase Activity in Mixed-Lineage Leukemia", MOLECULAR CELL, CELL PRESS, CAMBRIDGE, MA, US, vol. 53, no. 2, 2 January 2014 (2014-01-02), pages 247-261, XP028815036, ISSN: 1097-2765, DOI: 10.1016/J.MOLCEL.2013.12.001 cited in the application
- T. DERRIEN ET AL: "The GENCODE v7 catalog of human long noncoding RNAs: Analysis of their gene structure, evolution, and expression", GENOME RESEARCH, vol. 22, no. 9, 1 September 2012 (2012-09-01), pages 1775-1789, XP055193005, ISSN: 1088-9051, DOI: 10.1101/gr.132159.111 -& "Sequence: HG496980 | dbfetch | EBI", , 23 October 2013 (2013-10-23), XP055258587, Retrieved from the Internet: URL:http://www.ebi.ac.uk/Tools/dbfetch/dbf etch?toolbar=biobar&db=embl&format=default &style=html&id=HG496980 [retrieved on 2016-03-15]
- IMAMURA KATSUTOSHI ET AL: "Long Noncoding RNA NEAT1-Dependent SFPQ Relocation from Promoter Region to Paraspeckle Mediates IL8 Expression upon Immune Stimuli", MOLECULAR CELL, vol. 53, no. 3, 6 February 2014 (2014-02-06), pages 393-406, XP028606229, ISSN: 1097-2765, DOI: 10.1016/J.MOLCEL.2014.01.009
- FLORIAN GREBIEN ET AL: "Pharmacological targeting of the Wdr5-MLL interaction in C/EBP[alpha] N-terminal leukemia", NATURE CHEMICAL BIOLOGY, vol. 11, no. 8, 23 August 2015 (2015-08-23), pages 571-578, XP055258535, GB ISSN: 1552-4450, DOI: 10.1038/nchembio.1859 cited in the application

## Description

### BACKGROUND OF THE INVENTION

The present invention pertains generally to immunomodulation by altering levels of the proinflammatory chemokines. In particular, the invention relates to methods of modulating an immune response with UMLILO, a long non-coding RNA that regulates expression of the ELR+ proinflammatory chemokines by binding to WRD5. The invention further relates to methods of treating inflammatory, autoimmune, and infectious diseases, immunodeficiency, and cancer by reducing or increasing levels of chemokines.

Inflammation is a natural defense against pathogens as well as a hallmark of several chronic diseases. Inflammatory signals are conveyed by cytokines, a diverse group of small proteins secreted by immune and non-immune cells. Chemokines, the largest group of cytokines, induce neutrophil chemotaxis to the site of injury or infection. As this response forms a critical component of the innate immune system, it is precisely calibrated according to the nature and strength of the stimuli. Inadequate calibration may preclude clearing of infection, whereas excessive neutrophil chemotaxis will lead to tissue degradation and eventually chronic inflammatory disorders such as cancer, autoimmune disease, rheumatoid arthritis and severe sepsis.

Within the eukaryotic nucleus, the three-dimensional compaction of chromatin is tightly linked to transcriptional activity. The nucleus is an immensely crowded, yet paradoxically, highly organized environment. Its main constituent, DNA, is folded into chromosomal loops at the completion of mitosis, allowing its compaction in 3D nuclear space. Chromosomal folding and looping has been demonstrated to be causal to transcription bringing co-regulated genes and enhancer-promoter elements together in *cis* and *trans,* to form multigene complexes. These chromosomal contacts are usually identified by the population-based chromosome conformation capture (3C) and derivative techniques (Hi-C, ChlA-PET, 5C, 4C). Analyses of Hi-C data reveal that chromatin is divided into domains enriched in multigene complexes, referred to as topologically associating domains (TADs), with a median length of ∼180 Kbp. In metazoans, TADs are highly conserved and possess well-defined boundaries that are maintained across cell types. However, as 3C-based data sets are typically averaged over millions of cells, these interaction maps most likely represent an ensemble of dynamic chromosomal contacts, rather than revealing that the structure of TADs are identical in every cell across the population. Indeed, single cell Hi-C studies reveal that individual cells within a homogenous population may each possess different 3D chromatin organization. This highlights the absolute necessity of overlaying global interactome data with single cell studies.

Within TADs, or at the interface between neighbouring TADs, chromosomal looping brings genes and enhancer-promoter elements into close proximity, thereby increasing the concentration of transcriptional regulators in multigene complexes. The stability of these contacts remains an open question, with recent evidence showing that these contacts may be formed prior to gene activation, whereas alternative evidence favours the view that these interactions are dynamically established. Seminal studies of the β-globin locus reveal that the locus control region (LCR) forms dynamic chromosomal interactions with the promoter of the β-globin gene. Interestingly, single cell studies using fluorescent in situ hybridization (FISH) assays reveal that the LCR-β-globin chromosomal interactions are confined to a small subset of the population referred to as "jackpot cells", which also exhibit ∼100 fold higher levels of β-globin expression. Transcription is an inherently stochastic process, which is regulated by several factors, including the random collision of transcription factors with upstream promoter and enhancer DNA regions. Thus, the enrichment of chromosomal interactions, such as those observed between the LCR and β-globin, may account for the increased heterogeneity, or all-or-nothing transcriptional response, observed in a subset of an otherwise identical population of cells. However, dynamic loop-mediated regulation, and the resulting stochastic responses in gene expression, may not be ideal for gene classes that need to respond both immediately and uniformly to external stimuli across cell populations. This suggests that to reduce stochasticity in gene expression, the chromosomal contact between rapidly responding genes may exist in a more stable, or pre-formed state. This strongly implicates the influence of chromosomal looping and TAD structure on this sub-class of transcriptional responses.

The syntenic arrangement of co-regulated genes into TADs may enhance the probability of genes engaging in loop-mediated contact within multigene complexes. Recently, Hi-C studies revealed that several classes of innate immune genes are segregated into TADs and interact within multigene complexes. Rapid gene activation is critical to mount a successful innate immune response, which is an evolutionarily ancient system that protects the host organism against invading pathogens and other infectious agents. In particular, the rapid and robust transcriptional induction of the ELR+ CXC class of chemokines (IL8, CXCL1, CXCL2 and CXCL3) is necessary to induce neutrophil chemotaxis, the first line of the mammalian innate immune defense. Critically, a detailed understanding of the molecular mechanism underpinning rapid CXC chemokine transcriptional regulation is lacking. 3C analysis revealed that the promoters of several classes of the innate immune genes engage in chromosomal contact prior to activation by TNF. In addition, strongly emphasizing the central role of the transcriptional cycle in shaping the inflammatory response, the promoters of the innate immune response genes are preoccupied with poised RNA Pol II and are marked with active histone marks, such as trimethylation of lysine-4 of histone H3 (H3K4me3). This suggests that pre-formed chromosomal contact may be a prerequisite to poise an innate immune gene for expression, but, that an additional signal is necessary for complete transcriptional activation. As the transition between inactive and active promoter states is typically a slow, this may be a mechanism that eukaryotes have evolved to reduce the intrinsic noise associated with transcription. Uncontrolled CXC chemokine expression is the etiology of many disorders with an inflammatory basis, such as inflammatory bowel disease, cancer, autoimmune disease and severe sepsis. The latter is responsible for the highest patient mortality in the ICU. Therefore, decrypting the contribution of stable, or pre-formed, chromosomal contact to the poised nature of the innate immune genes would contribute significantly to achieving a full grasp of the molecular mechanism underpinning the uniform, rapid and robust activation of CXC chemokine transcription.

Many critical proinflammatory genes, such as the chemokines, exhibit accessible promoters and are therefore primed for rapid activation. The promoters of these genes are enriched for histone H3 K4 trimethylation (H3K4me3), an active chromatin mark that is catalyzed by the MLL family of methyltransferases. This chromatin modification permits RNA Pol II to access the promoter. The release of "paused" Pol II is facilitated by the recruitment of the transcriptional elongation factor p-TEFb by the chromatin reader, BRD4.

MLL1 is one of six members of the MLL family, which regulates hematopoiesis through the regulation of H3K4 methylation of the Hox genes. MLL1 has been implicated as the H3K4 methyltransferase of the innate immunity genes as it has been found to selectively regulate the activation of TNF- and LPS-induced gene activation (Wang et al. 2012). MLL1 activity is mediated via direct protein-protein interactions with the WAR complex (comprised of WDR5, Ash2L and RbBP5) as well as other regulators. Activity of MLL1 is particularly reliant on its association with WDR5, a multifunctional adapter protein that identifies H3K4 methylation marks on the promoters of genes as well as binds to MLL1. Therefore, small molecule inhibitors such as MM-401, MM-102 or OICR-9429 that prevent the WDR5/MLL1 interaction lead to a significant reduction in MLL1 methyltransferase activity (Cao et al. 2014; Griebien et al. 2015).

WDR5 has been shown to bind directly to long non coding RNAs. Long intergenic non coding RNA (lincRNA) transcripts range from ≤200nt to over 10kb in length and may even be spliced and polyadenylated. A subset of lncRNAs has been shown to regulate gene activity by binding to active or repressive chromatin remodeling complexes. Specifically, the lncRNA Xist achieves silencing of the X chromosome and dosage compensation by interacting with the polycomb repressive complex 2 (PRC2) leading to H3K27me3 and gene silencing. In contrast, two lncRNAs have been identified that bind to WDR5 and facilitate epigenetic H3K4me3 activation. HOTTIP is lncRNA of the human HOXA locus that binds directly binds to the WDR5 protein to recruit the MLL H3K4 methylase complex to maintain H3K4me3 (Wang et al. 2011). In addition, recent research has shown that the NeST lncRNA upregulates IFN-γ expression through interactions with WDR5 (Gomez et al. 2013, patent publication number US 2014/0056929 A1).

By acting as intermediates that link nuclear organization to gene regulation, long noncoding RNAs (lncRNAs) are emerging to be key modulators of gene activity. Despite lacking full protein coding potential, lncRNAs have been demonstrated to be essential during development with alterations in lncRNA activity shown to be associated with a multitude of disease states, such as cancer and inflammation. Although thousands of lncRNAs have been identified, very few have been assigned a function. A large portion of the characterised lncRNAs appear to act as a scaffold for chromatin remodeling complexes that read, write (Wang et al. 2011; Gomez et al. 2013) or erase histone modifications. In this way, lncRNAs may act in *cis* or *trans* to create local 'nuclear compartments' of high lncRNA-protein concentration which are able to modulate the epigenome, and as a consequence, the activity of target genes (Wang et al. 2011; Gomez et al. 2013). This reveals that the appropriate genomic location of IncRNAs is critical to their function.

3D chromatin compaction has been shown to influence how several IncRNAs are able to direct their protein partners to target loci across the genome. LncRNA-protein complexes may act in concert with DNA elements and enhancer-promoter looping interactions to mediate the assembly of multigene complexes. A remarkable recent observation is the transcription of a novel class of noncoding RNAs arising from enhancer loci throughout the genome. These enhancer-derived ncRNAS, termed enhancer RNA (eRNA) or activating RNA, display stimulus-dependent activation and are correlated with the transcriptional activation of nearby coding genes. eRNAs have been demonstrated to exert their activity through interaction with subunits of the Mediator complex, whose depletion abrogates enhancer-promoter loops leading to diminished transcription of target genes. A second subset of lncRNAs displaying enhancer-like activity exert their effect by acting as a scaffold for chromatin remodeling complexes that catalyze the trimethylation of histone H3 on lysine 4 (Wang et at. 2011; Gomez et al. 2013) in "pre-configured" chromatin environments. This suggests that lncRNAs may utilize pre-formed 3D chromosomal compaction to direct protein-binding partners to prime interacting genes for gene activation. A number of lncRNAs have been identified that may regulate innate immune gene transcription by directing the necessary proteins to target gene promoters. However, it remains unknown whether similar IncRNA-based mechanisms modulate the function of the CXC chemokines and how 3D nuclear architecture influences uniformity and rapidity of transcriptional activation.

Here we identify and mechanistically characterise a new enhancer-like lncRNA, UMLILO (Upstream Master LncRNA of the Inflammatory chemokine LOcus), which is conserved in higher vertebrates, but lacks a homolog in rats and mice. Using multiple genome-wide and single cell data sets we demonstrate that UMLILO is transcribed within the ∼500 Kbp chemokine TAD and arises from within a previously identified enhancer-dense region or super-enhancer (SE). Functional studies reveal that both super-enhancer and UMLILO activity act independently to drive chemokine transcription. Further, by combining a novel single cell genome-editing approach with population-based assays, we show the mechanism by which UMLILO acts in *cis,* to coordinate chemokine gene regulation by acting as a guide or scaffold to recruit WDR5 to the chemokine TAD, maintaining H3K4me3 across the chemokine gene promoters. Further, we show that the UMLILO-WDR5 complex is brought in close proximity to the chemokine genes by pre-formed chromosomal loops, which form upstream of UMLILO-mediated activity. Finally -and without precedent- we show that by replacing UMLILO with HOTTIP, a IncRNA which shared molecular partners with UMLILO, in a similar genomic location we are able to restore chemokine gene activation. Collectively, these data reveal that pre-formed chromosomal contact in the chemokine TAD provides the platform that facilitates the formation of a lncRNA-WDR5 complex in close proximity to the chemokine gene promoters. This in turn coordinates the hyper-responsive nature of the chemokine genes leading to robust and uniform transcriptional responses across diverse cell types. Remarkably, IncRNAs able to recruit the WR5-MLL1 complex to the appropriate genomic location can functionally substitute for each other. In sum, it creates a new paradigm for how nuclear architecture and IncRNA regulation assists in the rapid transcription of innate immune genes in inflammatory responses.

### SUMMARY OF INVENTION

The invention relates to compositions and methods of modulating the innate immune response by controlling levels of chemokines secreted by immune (macrophages, dendritic cells, neutrophils, basophils, eosinophils) and non-immune cells (fibroblasts, osteoblasts, epithelial, endothelial cells).

In a first aspect of the invention there is provided for an UMLILO IncRNA inhibitor for use in a method of treating an inflammatory condition, an autoimmune disorder or cancer in a subject by decreasing the production of ELR+ proinflammatory chemokines in the subject, the method comprising administering an effective amount of the UMLILO IncRNA inhibitor to the subject, wherein the UMLILO IncRNA inhibitor is selected from the group consisting of antisense oligonucleotides, miRNAs, siRNAs, piRNAs, snRNAs, CAS9/CRISPR, TALENS, zinc finger nucleases and BUD1 nucleases.

Adjustment of chemokine levels is achieved by increasing or decreasing the activity of UMLILO, a novel long non-coding RNA that induces expression of the proinflammatory chemokines (IL-8, CXCL1, CXCL3 and CXCL2). Further, it will be appreciated that replacing the genomic UMLILO IncRNA gene with a IncRNA gene which encodes a different IncRNA (such as HOTTIP) which is capable of binding WDR5 also leads to the modulation of chemokine levels. In the invention, the UMLILO IncRNA is encoded by the sequence provided in SEQ ID NO:1.

In one embodiment of the invention the ELR+ proinflammatory chemokines are IL8, CXCL1, CXCL2 and CXCL3.

According to the present invention, the UMLILO IncRNA inhibitor is selected from the group consisting of antisense oligonucleotides, miRNAs, siRNAs, piRNAs, snRNAs, CAS9/CRISPR, TALENS, zinc finger nucleases and BUD1 nucleases. Preferably, the siRNA is an siRNA selected from the group consisting of SEQ ID NOs:54 to 57.

In particular, the invention relates to the use of inhibitors of UMLILO, recombinant nucleic acids encoding UMLILO, or recombinant UMLILO incorporating nucleoside analogs wherein the recombinant UMLILO does not bind to WDR5, to modulate levels of chemokines for treatment of inflammatory conditions, autoimmune diseases, infections by pathogens (e.g., viruses, bacteria, fungi, and protists or other eukaryotic parasites), immunodeficiency, and cancer.

Exemplary UMLILO inhibitors may include antisense oligonucleotides, inhibitory RNA molecules, such as miRNAs, siRNAs, piRNAs, and snRNAs, and ribozymes. In certain embodiments, the UMLILO inhibitor is a recombinant polynucleotide comprising a promoter operably linked to a polynucleotide encoding an inhibitor of UMLILO.

It will be appreciated that in the instances where the UMLILO IncRNA inhibitor is a polynucleotide that such polynucleotide may be provided by a recombinant polynucleotide comprising a promoter operably linked to a polynucleotide encoding the UMLILO lncRNA inhibitor. Preferably, when the UMLILO lncRNA inhibitor is a polynucleotide or is encoded by a polynucleotide then the UMLILO IncRNA inhibitor may be provided by a lentiviral vector-carrying short hairpin RNA (shRNA) selected from the group consisting of SEQ ID NOs:54 to 57.

In a preferred embodiment the subject displays reduced inflammation after treatment with the UMLILO IncRNA inhibitor. In the invention, the subject has an inflammatory condition, an autoimmune disorder or cancer.

In certain embodiments, the subject has an inflammatory condition or an autoimmune disorder, such as, but not limited to multiple sclerosis, rheumatoid arthritis, stomatitis, lupus erythematosus, ischemic heart disease, atherosclerosis, cancer, fibrosis, autoimmune thyroid disease (AITD), inflammatory bowel disease, inflammatory myopathy, giant cell arteritis (GCA), asthma, allergy, Parkinson's disease, or Alzheimer's disease. In another embodiment, the subject has damaged tissue or a wound.

Typically, the inflammatory condition may be select from the group consisting of allergy, acne vulgaris, appendicitis, asthma, atherosclerosis, bursitis, cancer, celiac disease, chronic prostatitis, colitis, cystitis, dermatitis, glomerulonephritis, hay fever, viral infection, bacterial infection, fungal infection, archeal infection, inflammatory bowel disease, pelvic inflammatory disease, periodontitis, phlebitis, reperfusion injury, rhinitis, rheumatoid arthritis, sarcoidosis, sepsis, tendonitis, tonsillitis, transplant rejection, type 1 hypersensitivity and vasculitis.

Alternatively, in instances where the subject has an autoimmune disorder, the disorder may be selected from the group consisting of acute disseminated encephalomyelitis, acute motor axonal neuropathy, Addison's disease, adiposis dolorosa, adult-onset still's disease, alopecia areata, ankylosing spondylitis, anti-glomerular basement membrane nephritis, anti-neutrophil cytoplasmic antibody-associated vasculitis, anti-N-methyl-D-aspartate receptor encephalitis, antiphospholipid syndrome, antisynthetase syndrome, aplastic anemia, autoimmune angioedema, autoimmune enteropathy, autoimmune hemolytic anemia, autoimmune hepatitis, autoimmune inner ear disease, autoimmune lymphoproliferative syndrome, autoimmune neutropenia, autoimmune oophoritis, autoimmune orchitis, autoimmune pancreatitis, autoimmune polyendocrine syndrome, autoimmune polyendocrine syndrome type 2, autoimmune polyendocrine syndrome type 3, autoimmune progesterone dermatitis, autoimmune retinopathy, autoimmune thrombocytopenic purpura, autoimmune thyroiditis, autoimmune urticarial, autoimmune uveitis, balo concentric sclerosis, Behçet's disease, Bickerstaff's encephalitis, bullous pemphigoid, celiac disease, chronic inflammatory demyelinating polyneuropathy, Churg-Strauss syndrome, cicatricial pemphigoid, Cogan syndrome, cold agglutinin disease, crest syndrome, Crohn's disease, dermatitis herpetiformis, dermatomyositis, diabetes mellitus type 1, discoid lupus erythematosus, drug-induced lupus, endometriosis, enthesitis-related arthritis, eosinophilic fasciitis, epidermolysis bullosa acquisita, erythema nodosum, essential mixed cryoglobulinemia, Evans syndrome, Felty syndrome, fibromyalgia, gestational pemphigoid, giant cell arteritis, Graves' disease, Graves ophthalmopathy, Guillain-Barré syndrome, Hashimoto's encephalopathy, Henoch-Schonlein purpura, hidradenitis suppurativa, idiopathic inflammatory demyelinating diseases, Igg4-related systemic disease, inclusion body myositis, intermediate uveitis, interstitial cystitis, juvenile arthritis, Kawasaki's disease, Lambert-Eaton myasthenic syndrome, leukocytoclastic vasculitis, lichen planus, lichen sclerosus, ligneous conjunctivitis, linear IgA disease, lupus nephritis, lupus vasculitis, Lyme disease, Ménière's disease, microscopic colitis, microscopic polyangiitis, mixed connective tissue disease, Mooren's ulcer, morphea, Mucha-Habermann disease, multiple sclerosis, myasthenia gravis, myocarditis, myositis, neuromyelitis optica, neuromyotonia, opsoclonus myoclonus syndrome, optic neuritis, Ord's thyroiditis, palindromic rheumatism, paraneoplastic cerebellar degeneration, paroxysmal nocturnal hemoglobinuria, Parry Romberg syndrome, Parsonage-Turner syndrome, pediatric autoimmune neuropsychiatric disorder associated with streptococcus, pemphigus vulgaris, pernicious anemia, pityriasis lichenoides et varioliformis acuta, polyarteritis nodosa, polymyalgia rheumatica, polymyositis, postmyocardial infarction syndrome, primary biliary cirrhosis, primary sclerosing cholangitis, progressive inflammatory neuropathy, psoriasis, psoriatic arthritis, pure red cell aplasia, reactive arthritis, relapsing polychondritis, restless leg syndrome, retroperitoneal fibrosis, rheumatic fever, rheumatoid arthritis, rheumatoid vasculitis, sarcoidosis, Schnitzler syndrome, scleritis, Sjogren's syndrome, stiff person syndrome, subacute bacterial endocarditis, Susac's syndrome, Sydenham chorea, sympathetic ophthalmia, systemic lupus erythematosus, systemic scleroderma, thrombocytopenia, Tolosa-Hunt syndrome, transverse myelitis, ulcerative colitis, undifferentiated connective tissue disease, urticarial vasculitis, vasculitis and vitiligo.

In a second aspect of the invention there is provided for an UMLILO lncRNA for use in a method of treating an immunodeficiency in a subject by increasing the production of ELR+ proinflammatory chemokines in the subject, the method comprising administering an effective amount of an UMLILO lncRNA to the subject, wherein transcription of the ELR+ proinflammatory chemokines is initiated in a cell when WRD5 is present with the UMLILO lncRNA.

It will be appreciated that in the UMLILO lncRNA inhibitor may be provided to the subject or a cell by a recombinant polynucleotide comprising a promoter operably linked to a polynucleotide encoding the UMLILO lncRNA.

In a preferred embodiment, the subject has an immunodeficiency and the immunodeficiency results in the subject having an increased risk of a pathogenic infection. Preferably, the pathogenic infection is a viral, bacterial, fungal or parasitic infection.

There is also described herein methods of decreasing the production of ELR+ proinflammatory chemokines in a subject, comprising administering an effective amount of an UMLILO lncRNA inhibitor to the subject.

It will be appreciated that the method disclosed results in a subject displaying reduced inflammation after treatment with the UMLILO IncRNA inhibitor.

The method of the disclosure finds application in instances where the subject has an inflammatory condition, an autoimmune disorder or cancer.

There is also described herein a method of increasing the production of ELR+ proinflammatory chemokines in a subject, the method comprising administering an effective amount of an UMLILO lncRNA to the subject, wherein transcription of the ELR+ proinflammatory chemokines is initiated in a cell when WRD5 is present with the UMLILO lncRNA. Typically, the method of increasing the production of proinflammatory cytokines will be used instances where the subject has an immunodeficiency and wherein the immunodeficiency results in the subject having an increased risk of a pathogenic infection.

There is also described herein a method of increasing transcription of an UMLILO lncRNA in a cell, comprising administering a compound selected from the group consisting of calcium, nuclear factor of activated T-cells (NF-AT) or activator protein 1 (AP1) to the cell, wherein transcription of UMLILO in the cell is increased after administration of the compound.

There is also described herein a method of decreasing production of ELR+ chemokines by immune and non-immune cells in a subject, the method comprising administering an effective amount of a UMLILO inhibitor or a vector encoding a UMLILO inhibitor to the subject.

There is also described herein a method for treating an infectious disease comprising administering to a subject in need thereof a therapeutically effective amount of UMLILO.

There is also described herein a method for treating an inflammatory condition or autoimmune disorder comprising administering to a subject in need thereof a therapeutically effective amount of at least one UMLILO inhibitor.

There is also described herein a method for inhibiting UMLILO in a subject comprising administering an effective amount of a UMLILO inhibitor to the subject.

### BRIEF DESCRIPTION OF THE FIGURES

Non-limiting embodiments of the invention will now be described by way of example only and with reference to the following figures:

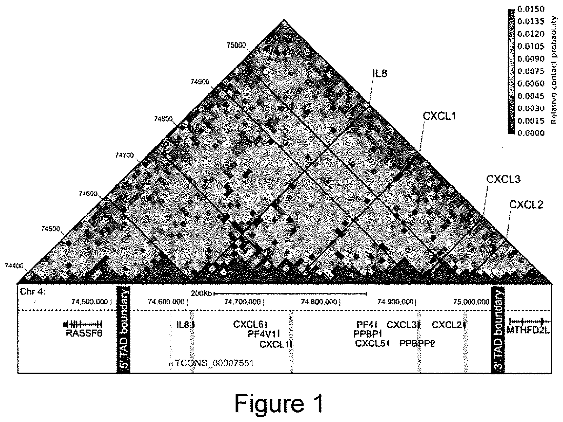

**Figure 1****:** UMLILO is a new super-enhancer resident IncRNA that is transcribed within the ELR+ CXC chemokine TAD: Hi-C analysis across the CXC chemokine locus reveals that the chemokine TAD spans a region of -500 Kbp.

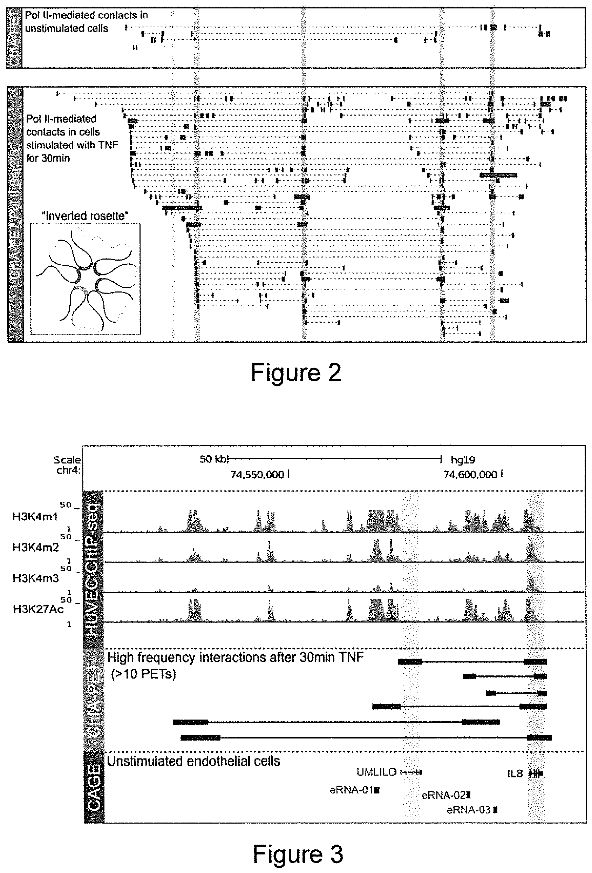

**Figure 2****:** UMLILO is a new super-enhancer resident lncRNA that is transcribed within the ELR+ CXC chemokine TAD: ChlA-PET data from a library constructed in HUVECs with an antibody that enriches for actively transcribing Pol II. Unstimulated HUVECs displayed relatively few PETs/chromosomal contacts. Upon chemokine gene induction with TNF for 30 mins numerous PETs/contacts were observed, occurring between the super-enhancer region and the chemokine genes, resulting in the formation of a putative 'inverted rosette' structure. Contacts detected by ChlA-PET between interacting chemokine genes, were confined exclusively to the region encompassing the chemokine TAD.
**Figure 3****:** UMLILO is a new super-enhancer resident IncRNA that is transcribed within the ELR+ CXC chemokine TAD: ChlPseq analysis across the chemokine TAD revealed that the SE is atypically large (∼80 Kbp) and highly enriched for H3K4me1 and H3K27Ac epigenetic marks. The UMLILO IncRNA was identified from CAGE data to be expressed in unstimulated endothelial cells. UMLILO does not possess the typical eRNA histone modifications, H3K4me1 and H3K27Ac.

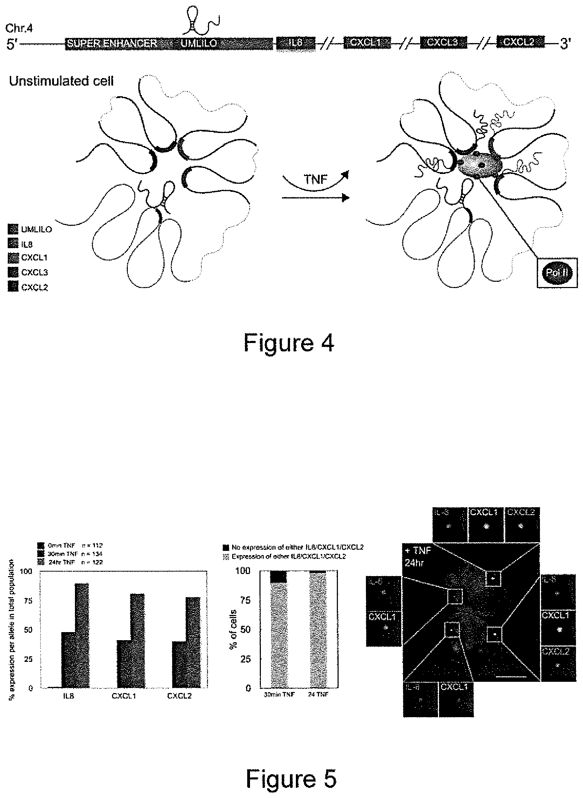

**Figure 4****:** UMLILO transcription precedes chemokine gene activation: The chemokine TAD structure is formed prior to gene activation by TNF and UMLILO is brought in close proximity to the chemokine genes by pre-formed chromosomal loops.
**Figure 5****:** UMLILO transcription precedes chemokine gene activation: Intronic smFISH reveals that the chemokines are only induced in HUVECs following TNF induction. Intronic RNA FISH foci of co-expressed chemokine genes always co-localize (n, number of alleles; scale bar, 5 µm; cells were counterstained with DAPI.).

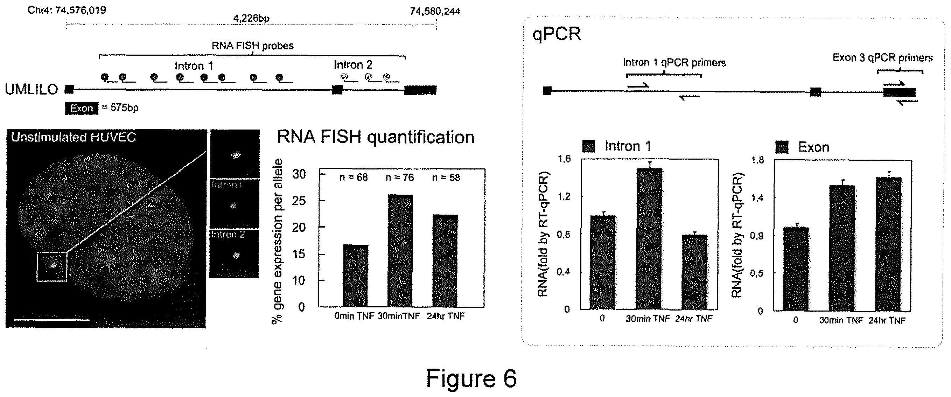

**Figure 6****:** UMLILO transcription precedes chemokine gene activation: RNA FISH on intron 1 and intron 2 of UMLILO reveal UMLILO is expressed in HUVECs prior to TNF stimulation. The signal from both introns consistently colocalized. Levels of intronic UMLILO were elevated shortly after TNF treatment, and then dropped back down to resting UMLILO levels after longer durations of TNF stimulation (n, number of alleles; scale bar, 5 µm; cells were counterstained with DAPI.).

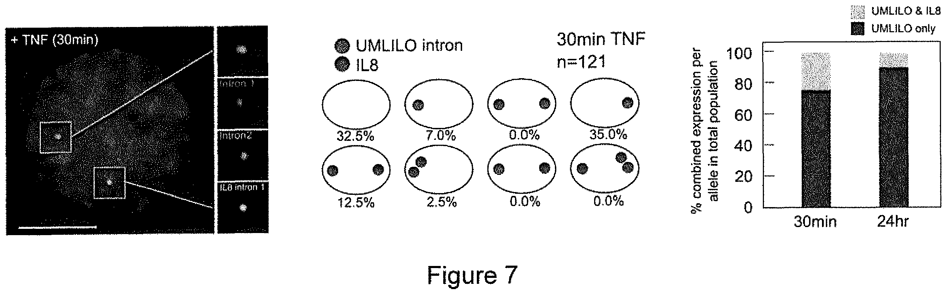

**Figure 7****:** UMLILO transcription precedes chemokine gene activation: Simultaneous intronic RNA FISH on UMLILO and the chemokine genes revealed that co-localization between intronic UMLILO and the chemokine genes was rarely observed (n, number of alleles; scale bar, 5 µm; cells were counterstained with DAPI.).

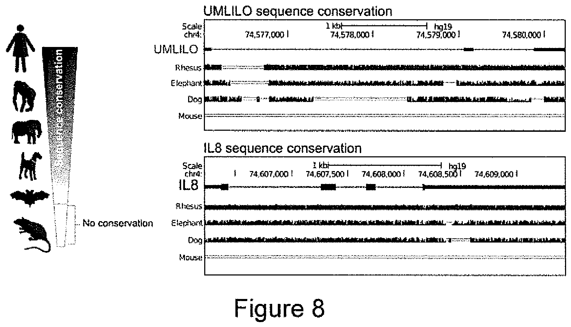

**Figure 8****:** UMLILO transcription precedes chemokine gene activation: UMLILO is highly conserved in higher vertebrates with neutrophil rich blood, but no homolog of UMLILO or IL8 exists in mice.

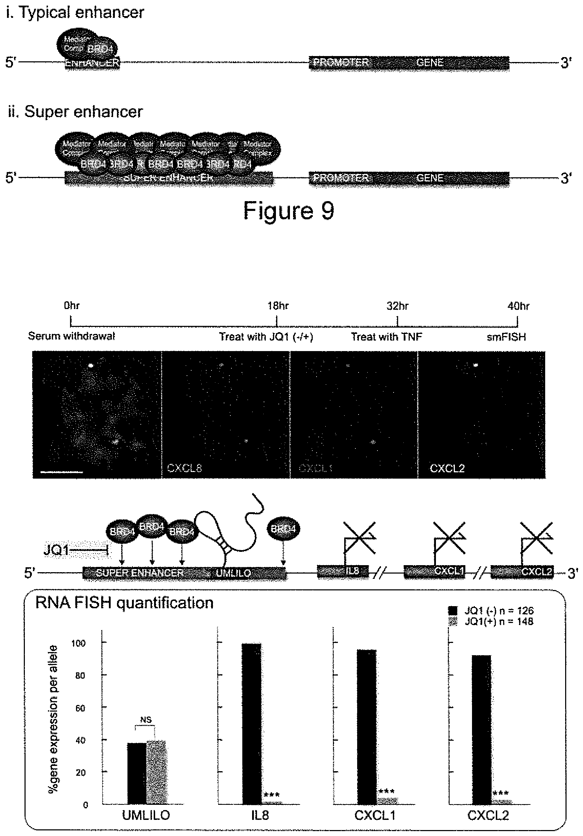

**Figure 9****:** UMLILO transcription is not influenced by perturbing transcriptional regulators that densely occupy the super-enhancer, but is necessary for chemokine transcription: The super-enhancer is densely occupied by chromatin regulators, including the mediator subunit, MED12, and the chromatin reader BRD4.

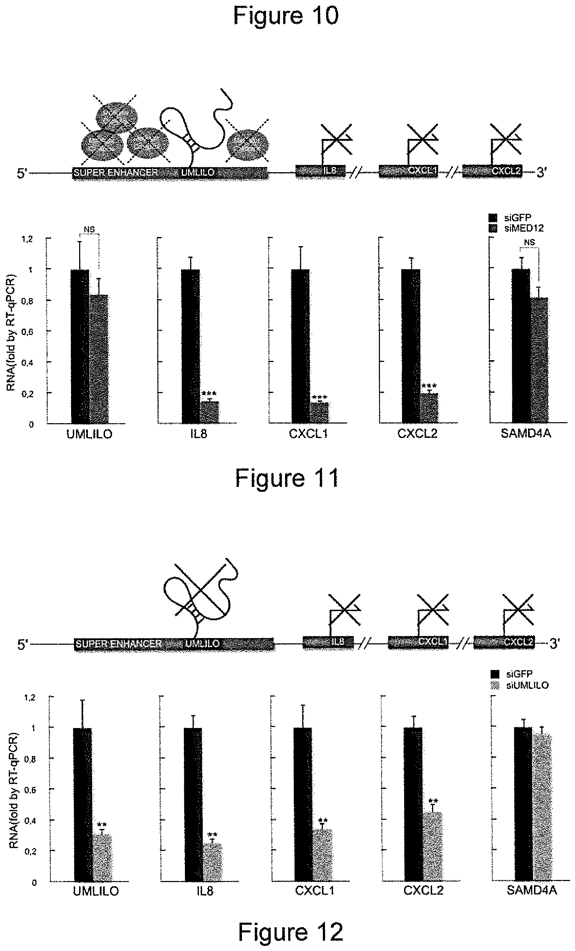

**Figure 10****:** UMLILO transcription is not influenced by perturbing transcriptional regulators that densely occupy the super-enhancer, but is necessary for chemokine transcription: Chemokine expression is sensitive to BRD4 depletion. (+) JQ1 but not its enantiomer (-) JQ1 abrogated chemokine transcriptional activation, but did not influence UMLILO transcription (cells were counterstained with DAPI; scale bar, 5 µm).
**Figure 11****:** UMLILO transcription is not influenced by perturbing transcriptional regulators that densely occupy the super-enhancer, but is necessary for chemokine transcription: Chemokine expression is sensitive to MED12 depletion. Depleting MED12 abrogates the expression of the CXC chemokines, but did not significantly alter the expression of UMLILO (Mean +/- SD; **p < 0.05, *** p < 0.01; two-tailed unpaired Student's T test).
**Figure 12****:** UMLILO transcription is not influenced by perturbing transcriptional regulators that densely occupy the super-enhancer, but is necessary for chemokine transcription:UMLILO is necessary for chemokine expression. siRNA knockdown of UMLILO was sufficient to significantly abrogate chemokine expression, but not another TNF-induced gene located in a different TAD (Mean +/- SD; **p < 0.05, *** p < 0.01; two-tailed unpaired Student's T test).

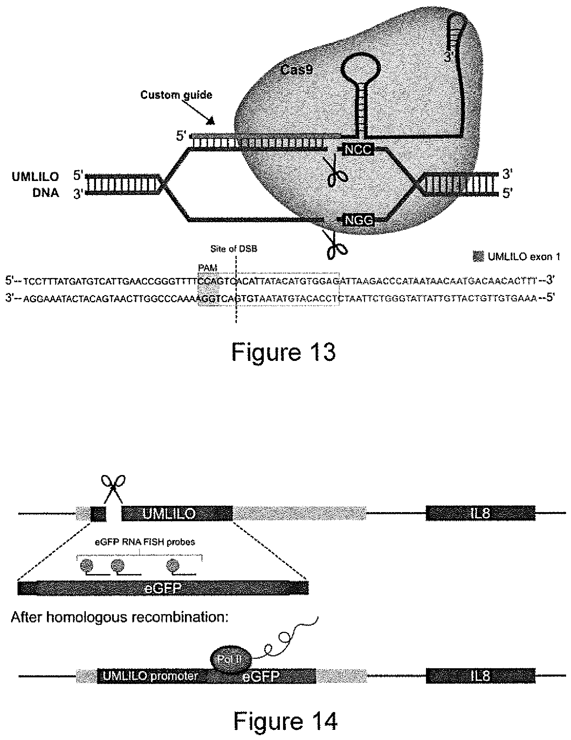

**Figure 13****:** UMLILO acts in *cis* to regulate chemokine expression: The CRISPR-Cas9 system was used to erase the genomic sequence encoding UMLILO and replace it with an eGFP reporter sequence in primary HUVEC cells.
**Figure 14****:** UMLILO acts in *cis* to regulate chemokine expression: Successful repair was detected using RNA FISH probes targeting eGFP RNA, driven by the endogenous UMLILO promoter.

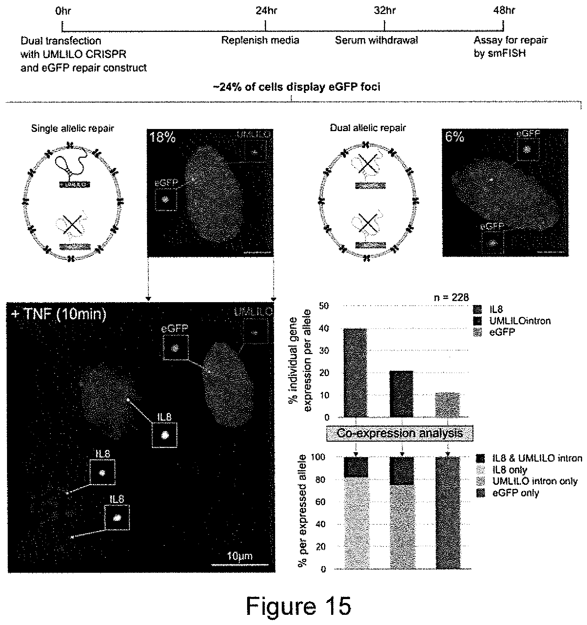

**Figure 15****:** UMLILO acts in *cis* to regulate chemokine expression: Successful repair was observed in ∼26% of cells, with ∼19% of cells displaying a single foci, and ∼7% of cells displaying dual eGFP foci. Cells displaying a single eGFP focus were still able to express UMLILO from the opposite allele. The deletion of UMLILO abrogated chemokine expression in cis. Co-localization between eGFP foci and IL8 was never observed (Two-tailed Fisher's exact test; *p < 0.01; cells were counterstained with DAPI; Scale bar, 5 µm).

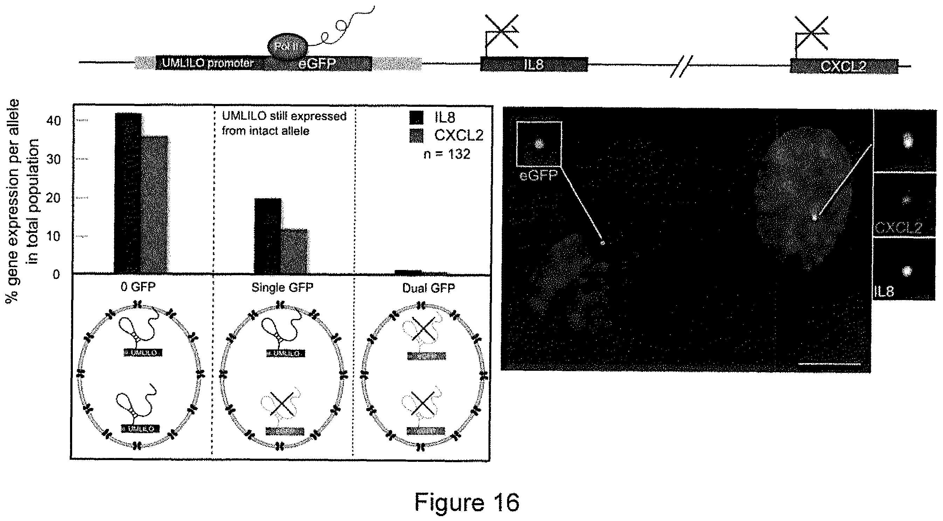

**Figure 16****:** UMLILO acts in *cis* to regulate chemokine expression: UMLILO exerts its effect in *cis.* In cells displaying no eGFP foci, expression of IL8 and CXCL2 was comparable to the mock-transfected cells. In cells displaying a single eGFP focus, the expression of IL8 and CXCL2 was reduced significantly. Chemokine expression was never observed in cells displaying dual eGFP foci (Two-tailed Fisher's exact test; *p < 0.01; cells were counterstained with DAPI; Scale bar, 5 µm).

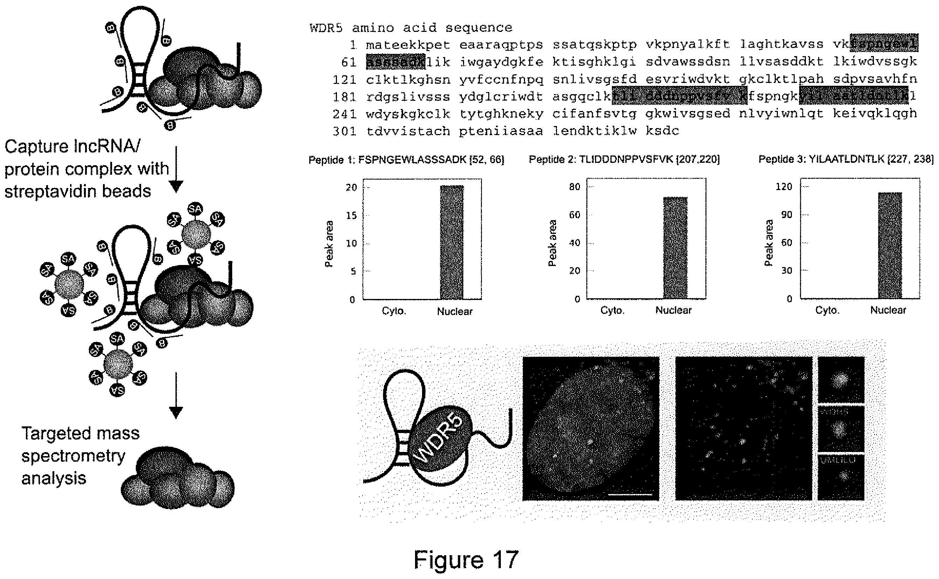

**Figure 17****:** UMLILO interacts with the WAR complex to maintain H3K4me3 epigenetic regulation of chemokine genes: UMLILO interacts with WDR5. UMLILO was recovered from the nuclear fraction of cell lysates using biotinylated single stranded DNA probes tiling the exonic portion of the lncRNA. Using the MRM^{HR} mass spectrophotometry approach, we identified WDR5, but not Ash2L, RbBP5 or DY30, in the UMLILO pull downs. Immunofluoresence revealed that WDR5 is a low abundant protein located in the nucleus. Immuno-RNA FISH revealed that distinct foci of WDR5 colocalize with UMLILO. Cyto.= cytoplasmic fraction (*p < 0.05, ** p < 0.01, two-tailed unpaired Student's T test; Scale bar, 5 µm).

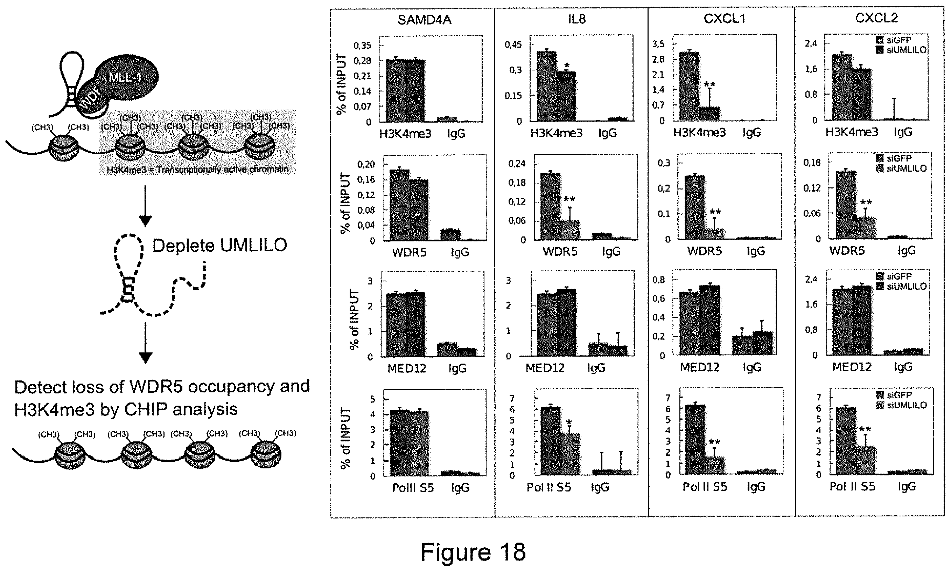

**Figure 18****:** UMLILO interacts with the WAR complex to maintain H3K4me3 epigenetic regulation of chemokine genes: UMLILO directs WDR5 to the chemokine promoters to facilitate their epigenetic activation. siUMLlLO modulates WDR5 binding as well as Pol II-Ser5 and H3K4me3 occupancy on chemokine promoters (*p < 0.05, ** p < 0.01, two-tailed unpaired Student's T test).

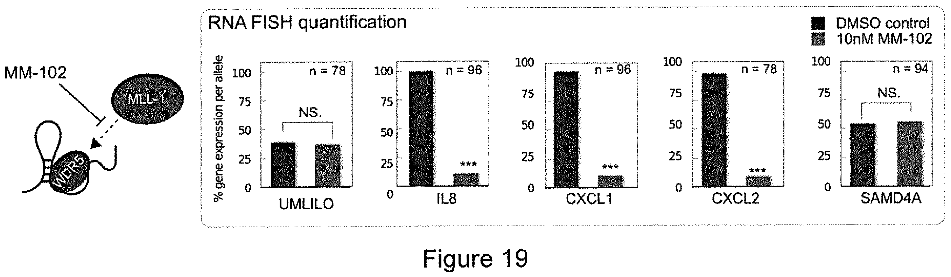

**Figure 19****:** UMLILO interacts with the WAR complex to maintain H3K4me3 epigenetic regulation of chemokine genes: Targeting MLL1-WDR5 interactions abrogated chemokine transcription. MM102 led to a significant reduction in chemokine gene transcription (*p < 0.05, ** p < 0.01, two-tailed unpaired Student's T test).

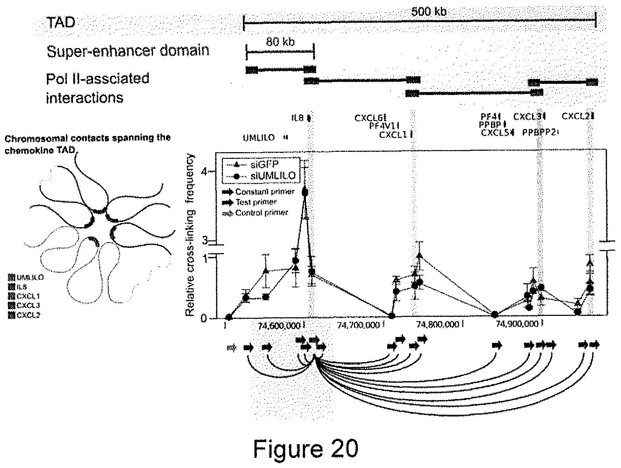

**Figure 20****:** UMLILO does not regulate chromosomal contact across the chemokine TAD: Depleting UMLILO using siRNA does not significantly alter chromosomal contact across the chemokine TAD. ChlA-PET data reveals the sites of active Pol II-mediated chromosomal contacts.

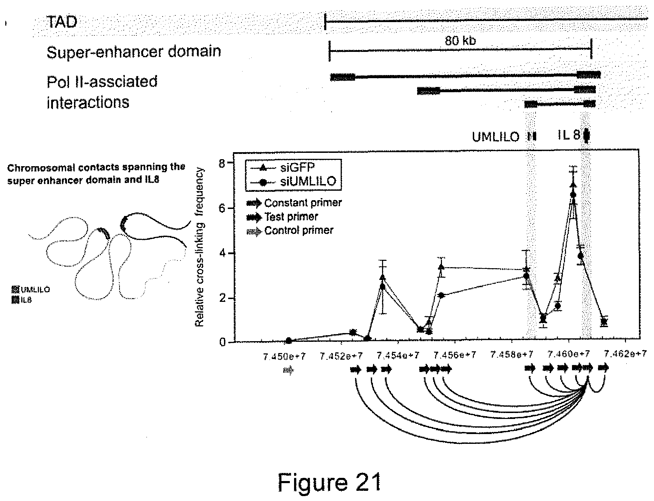

**Figure 21****:** UMLILO does not regulate chromosomal contact across the chemokine TAD: siUMLlLO did not significantly alter the chromosomal contacts between UMLILO and IL8. siGFP was used as a control for the detection of baseline chromosomal contacts.

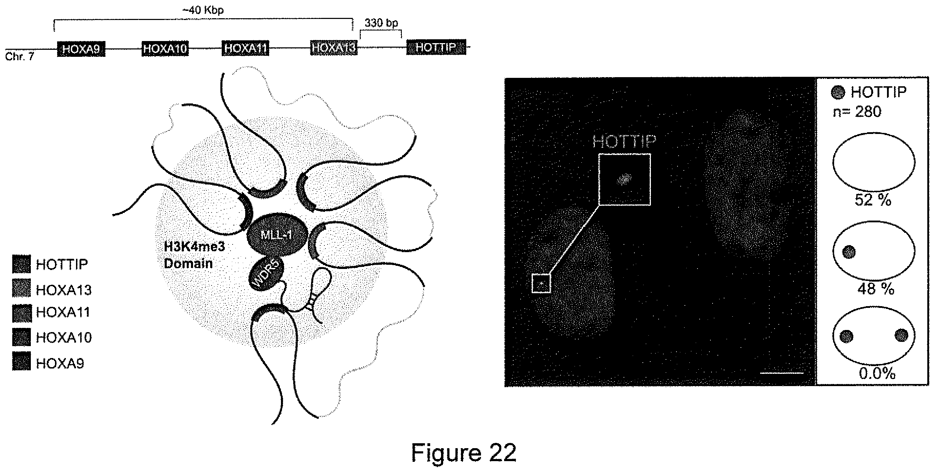

**Figure 22****:** The activity of UMLILO can be substituted with a different WDR5-interacting enhancer-like lncRNA: HOTTIP FISH spots were observed at approximately 25% of alleles and only single allelic expression of HOTTIP per HeLa cell was observed (n, number of cells; cells were counterstained with DAPI; Scale bar, 5 µm).

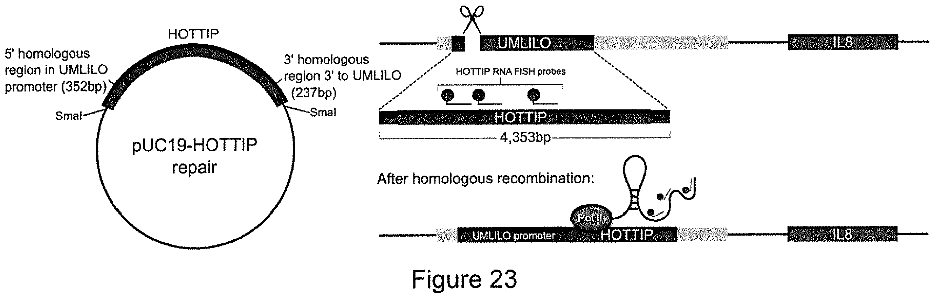

**Figure 23****:** The activity of UMLILO can be substituted with a different WDR5-interacting enhancer-like lncRNA: Graphical representation of the repair strategy. The repair construct consists of the sequence of HOTTIP flanked by homologous arms that flank UMLILO.

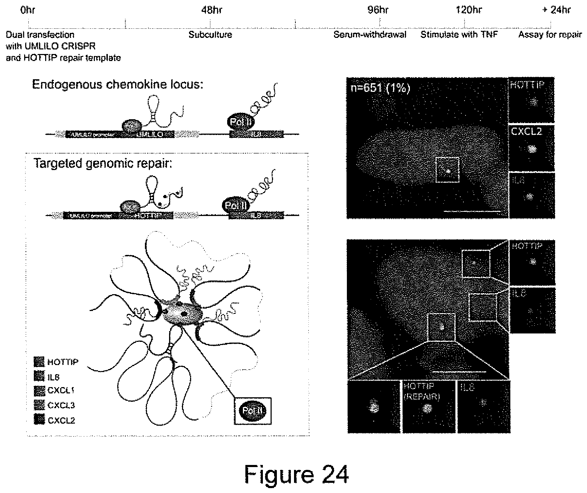

**Figure 24****:** The activity of UMLILO can be substituted with a different WDR5-interacting enhancer-like lncRNA: Replacing endogenous UMLILO with HOTTIP restores chemokine transcription. Overlapping HOTTIP and chemokine foci were observed in ∼1% of HeLas dual transfected with the CRISPR and HOTTIP repair construct for 120 hr and stimulated with TNF for 24 hrs (n, number of cells; cells were counterstained with DAPI; Scale bar, 5 µm).

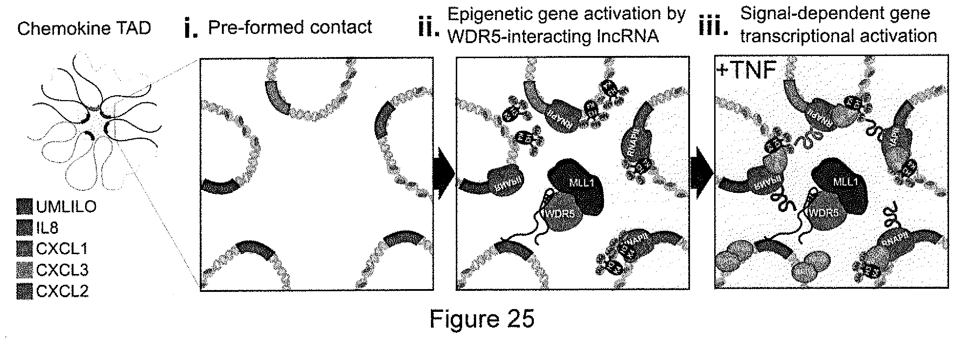

**Figure 25****:** The activity of UMLILO can be substituted with a different WDR5-interacting enhancer-like IncRNA: WDR5-interacting IncRNAs exploit pre-formed 3D chromatin folding to coordinate rapid chemokine gene activation. By acting in *cis*, WDR5-interacting lncRNAs use the local chromatin compaction of the pre-formed chemokine TAD to direct the WDR5 protein across the chemokine promoters. Upon activation by TNF, the chemokine super-enhancer becomes loaded with BRD4 and other transcriptional regulators, to induce the rapid and robust transcriptional induction of the chemokine genes.

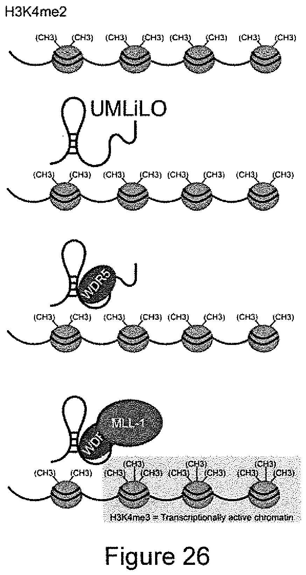

**Figure 26****:** UMLILO is a novel lncRNA of the human ELR+ chemokine locus that binds directly to the WDR5 protein to recruit the MLL H3K4 methylase complex, maintaining H3K4me3 on the chemokine promoters.

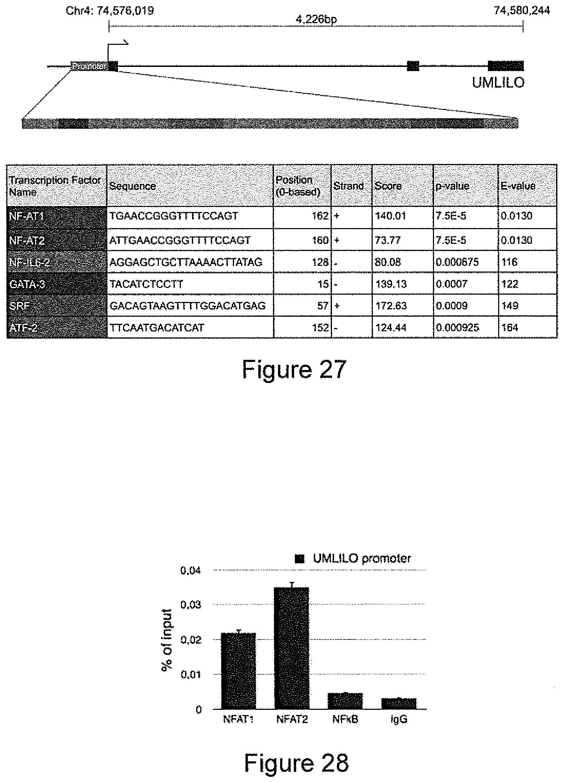

**Figure 27****:** Tacrolimus inhibits UMLILO and IL-8 expression. The LASAGNA (Length-Aware Site Alignment Guided by Nucleotide Association) algorithm for transcription factor binding site (TFBS) alignment (http://biogrid.engr.uconn.edu/ lasagna_search/) was used to analyze the UMLILO promoter for putative TFBS. A high confidence binding site for the calcium dependent transcription factors, NF-AT1 and NF-AT2 was observed near to the TSS of UMLILO.
**Figure 28****:** Tacrolimus inhibits UMLILO and IL-8 expression. CHIP assays confirm that the NF-AT transcription factors occupy the UMLILO promoter.

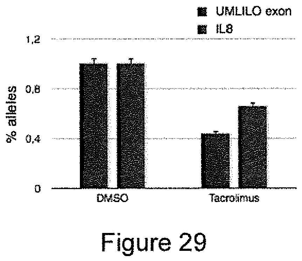

**Figure 29****:** Tacrolimus inhibits UMLILO and IL-8 expression. Tacroliumus, a macrolide calcium inhibitor, leads to a reduction in UMLILO and IL-8 expression.

### DETAILED DESCRIPTION OF THE INVENTION

The present invention will now be described more fully hereinafter with reference to the accompanying drawings, in which some, but not all embodiments of the invention are shown.

The invention as described should not be limited to the specific embodiments disclosed and modifications and other embodiments are intended to be included within the scope of the invention. Although specific terms are employed herein, they are used in a generic and descriptive sense only and not for purposes of limitation.

As used throughout this specification and in the claims which follow, the singular forms "a", "an" and "the" include the plural form, unless the context clearly indicates otherwise.

The terminology and phraseology used herein is for the purpose of description and should not be regarded as limiting. The use of the terms "comprising", "containing", "having" and "including" and variations thereof used herein, are meant to encompass the items listed thereafter and equivalents thereof as well as additional items.

As used herein the term "UMLILO" refers to the Upstream Master LncRNA of the Inflammatory chemokine LOcus, a long non-coding RNA (IncRNA) encoded by the human genome sequence corresponding to the following genomic co-ordinates: chr4;74,576,019-74,580,0244. An exemplary DNA sequence encoding for UMLILO is provided in SEQ ID NO:1. Alternative transcript names for UMLILO include: TCONS_00007551; ENST00000436089; ENST00000436089.1; AC112518.3-001; OTTHUMT00000322 216.2; NONHSAT096866.

The term "immunomodulatory" or "modulating an immune response" as used herein includes immunostimulatory as well as immunosuppressive effects. Immunomodulation, for example, by UMLILO or an inhibitor of UMLILO may cause an increase or decrease in CXC production, respectively, in an individual treated in accordance with the methods of the invention as compared to the absence of treatment. The level of CXC, in turn modulates chemotaxis innate and cellular immune responses by interacting with G protein-linked transmembrane receptors or chemokine receptors, that are selectively found on the surfaces of their target cells e.g. neutrophils, macrophages, T-lymphocytes, mast cells and eosinophils.

The term "microRNA" refers to endogenous or artificial non-coding RNAs that are capable of regulating gene expression. It is believed that miRNAs function via RNA interference. Endogenous (e.g., naturally occurring) miRNAs are typically expressed from RNA polymerase II promoters and are generated from a larger transcript.

The term "siRNA" refers to single-stranded or double-stranded RNA molecules that are capable of inducing RNA interference. siRNA molecules typically have a duplex region that is between 18 and 30 base pairs in length.

The term "piRNA" refers to a class of small RNAs involved in gene silencing. piRNA molecules typically are between 26 and 31 nucleotides in length.

The term "snRNA" refers to a class of small RNAs involved in a variety of processes including RNA splicing and regulation of transcription factors. The subclass of small nucleolar RNAs (snoRNAs) is also included. The term is also intended to include artificial snRNAs, such as antisense derivatives of snRNAs comprising antisense sequences directed against the IncRNA, UMLILO.

The terms "chemokine," "pro-inflammatory chemokine," "CXC chemokine" and "CXC" are interchangeable and refer to cytokines characterised according to behavioural and structural characteristics to induce neutrophil chemotaxis to the site of injury or infection, in particular CXC chemokines having two N-terminal cysteines separated by one amino acid, even more particularly having a specific amino acid sequence of glutamic acid-leucine-arginine (ELR) immediately before the first cysteine of the CXC motif (ELR-positive or ELR+ CXC chemokines). ELR-positive (ELR+) CXC chemokines specifically induce the migration of neutrophils, and interact with chemokine receptors CXCR1 and CXCR2. An example of an ELR-positive CXC chemokine is interleukin-8 (IL-8), which induces neutrophils to leave the bloodstream and enter into the surrounding tissue.

The term "exon" when used in relation to a lncRNA refers to a defined section of nucleic acid that is represented in the mature form of a IncRNA molecule after portions of a pre-processed (or precursor) lncRNA have been removed by splicing.

The term "intron" when used in relation to a IncRNA refers to a nucleic acid region in the precursor IncRNA that is subsequently removed by splicing during formation of the mature lncRNA.

The terms "polynucleotide," "oligonucleotide," "oligo" "nucleic acid" and "nucleic acid molecule" are used herein to include a polymeric form of nucleotides of any length, either ribonucleotides or deoxyribonucleotides. This term refers only to the primary structure of the molecule. Thus, the term includes triple-, double- and single-stranded DNA, as well as triple-, double- and single-stranded RNA. It also includes modifications, such as by methylation and/or by capping, and unmodified forms of the polynucleotide. There is no intended distinction in length between these terms and they are used interchangeably.

The term "pharmaceutically acceptable excipient or carrier" refers to an excipient that may optionally be included in the compositions of the invention and that causes no significant adverse toxicological effects to the patient.

The term "pharmaceutically acceptable salt" includes, but is not limited to, amino acid salts, salts prepared with inorganic acids, such as chloride, sulfate, phosphate, diphosphate, bromide, and nitrate salts, or salts prepared from the corresponding inorganic acid form of any of the preceding, e.g., hydrochloride, etc., or salts prepared with an organic acid, such as malate, maleate, fumarate, tartrate, succinate, ethylsuccinate, citrate, acetate, lactate, methanesulfonate, benzoate, ascorbate, paratoluenesulfonate, palmoate, salicylate and stearate, as well as estolate, gluceptate and lactobionate salts. Similarly salts containing pharmaceutically acceptable cations include, but are not limited to, sodium, potassium, calcium, aluminum, lithium, and ammonium (including substituted ammonium).

The term "pathogen" or "parasite" or "microbe" refers to any virus or organism that spends at least part of its life cycle or reproduces within a host. Intracellular pathogens include, but are not limited to, viruses (e.g., influenza virus, respiratory syncytial virus, hepatitis virus B, hepatitis virus C, herpes virus, papilloma virus, and human immunodeficiency virus), bacteria (e.g., *Listeria, Mycobacteria* (e.g., *Mycobacterium tuberculosis, Mycobacterium leprae), Salmonella* (e.g., *S*. *typhi*), enteropathogenic *Escherichia coli* (EPEC), enterohaemorrhagic *Escherichia coli* (EHEC), *Yersinia, Shigella, Chlamydia, Chlamydophila, Staphylococcus, Legionella*)*,* protozoa (e.g., *Plasmodium* (e.g., *P*. *vivax, P. falciparum, P. ovale,* and *P*. *malariae*)*, Taxoplasma, Leishmania*)*,* and fungi (e.g., *Aspergillus, Blastomyces, Candida*)*.* Eukaryotic intercellular parasites include trematodes (e.g., *Schistosoma, Clonorchis*)*,* hookworms (e.g., *Ancylostoma duodenale* and *Necator americanus*)*,* and tape worms (e.g., *Taenia solium, T. saginata, Diphyllobothrium* spp., *Hymenolepis* spp., *Echinococcus* spp.).

The term "cancer" refers to human cancers and carcinomas, sarcomas, adenocarcinomas, lymphomas, leukemias, etc., including but not limited to solid tumors and lymphoid cancers, kidney, breast, lung, kidney, bladder, colon, ovarian, prostate, pancreas, stomach, brain, head and neck, skin, uterine, testicular, esophagus, and liver cancer, lymphoma, including non-Hodgkin's and Hodgkin's lymphoma, leukemia, and multiple myeloma.

The terms "overexpress," "overexpression" or "overexpressed" interchangeably refer to a gene that is transcribed or translated at a detectably greater level, in comparison to a normal cell. Overexpression therefore refers to both overexpression of protein and/or RNA (due to increased transcription, post transcriptional processing, translation, post translational processing, altered stability, and/or altered protein degradation), as well as local overexpression due to altered protein traffic patterns (increased nuclear localization), and augmented functional activity, e.g., as in an increased enzyme hydrolysis of substrate.

The term "inhibitor" refers to an agent that, by way of non-limiting example, inhibits expression of a IncRNA of the invention or bind to, partially or totally block stimulation or enzymatic activity, decrease, prevent or delay activation, inactivate, desensitize, or down regulate the activity of a IncRNA of the invention, e.g., antagonists.

The terms "identical" or percent "identity," in the context of two or more nucleic acids or polypeptide sequences, refer to two or more sequences or subsequences that are the same or have a specified percentage of amino acid residues or nucleotides that are the same (i.e., about 60% identity, preferably 65%, 70%, 75%, 80%, 85%, 90%, 91%, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters described below, or by manual alignment and visual inspection (see, e.g., NCBI website: ncbi.nlm.nih.gov/BLAST/ or the like). Such sequences are then said to be "substantially identical." This definition also refers to, or may be applied to, the compliment of a test sequence. The definition also includes sequences that have deletions and/or additions, as well as those that have substitutions. As described below, the preferred algorithms can account for gaps and the like. Preferably, identity exists over a region that is at least about 25 amino acids or nucleotides in length, or more preferably over a region that is 50-100 amino acids or nucleotides in length.

The term "up-regulation" refers to the process by which a cell increases the quantity of a cellular component, such as RNA or protein, in response to an internal or external variable or stimulus.

An "effective amount" of UMLILO is an amount sufficient to effect beneficial or desired results, such as an amount that increases production of CXCs. An effective amount can be administered in one or more administrations, applications, or dosages.

An "effective amount" of an UMLILO inhibitor (e.g., microRNA, siRNA, piRNA, snRNA, antisense nucleic acid, ribozyme, or small molecule inhibitor) is an amount sufficient to effect beneficial or desired results, such as an amount that inhibits the activity of UMLILO, for example by interfering with transcription of UMLILO, binding of UMLILO to WDR5, or activation of CXC gene expression. An effective amount can be administered in one or more administrations, applications, or dosages.

### Statistics

p values from two-tailed unpaired Student's t test and Fisher Exact tests were calculated using R (http://www.R-project.org). *p < 0.05, **p < 0.01, and ***p < 0.001.

Bioinformatic analysis of both Hi-C and ChlA-PET data in the CXC TAD identified a 'super-enhancer' region, spanning ∼80kb and forming extensive chromosomal contacts with the proinflammatory genes. CAGE analysis across various primary cell types and cell lines identified UMLILO (upstream master linc of the chemokine locus), a novel lncRNA transcribed from within the super enhancer region in resting immune and non-immune cells. UMLILO is highly conserved in mammals with neutrophil-rich blood, whereas no homolog of either UMLILO or IL-8 exists in rodents and other animals with neutrophil-poor blood. "Super enhancer" drugs targeting BRD4 abrogate chemokine expression, but UMLILO expression remained unaffected. Using genome-editing technologies and siRNA knockdown approaches we show that UMLILO is necessary for chemokine expression. Further, simultaneous intronic FISH revealed that UMLILO transcription precedes the chemokine gene expression. This suggests that UMLILO may be required to establish a nuclear compartment that facilitates the rapid activation of chemokine gene expression. Accordingly, RNA immunopreciptation experiments revealed that UMLILO might coordinate chemokine gene regulation by acting as a scaffold to recruit chromatin remodelers and the WAR complex to the chemokine TAD. Preventing the WDR5/MLL1 interaction using the small molecule inhibitor, MM-102 abrogated chemokine transcription. Therefore, we present a novel approach to alter the transcriptional status of chemokine genes.

The following examples are offered by way of illustration and not by way of limitation.

### EXAMPLE 1

### Cell Culture

Early passage HUVECs from pooled donors (Lonza) were grown to ∼80% confluence in Endothelial Basal Medium-2 (EGM-2) with supplements (Lonza), serum-starved (18hr) in EGM-2 + 0.5% FBS, and treated with TNF (10 ng/ml; Sigma) for up to 24 hr. Prior to transfection cells were grown in antibiotic free EGM-2.

### Hi-C analysis

Hi-C sequencing data were preprocessed (iterative alignment and outlier removal) using the pipeline described by Imakaev and colleagues (Imakaev et al. 2012). The heatmap in the chemokine locus show the Hi-C interactions as paired-read counts between pairs of sliding windows of 50 Kbp in length.

### ChlA-PET analysis

The two Pol II (Ser2/Ser5) ChlA-PET libraries for HUVEC were obtained from NCBI (accession numbers: GSE41553) (Papantonis et al. 2012). One library is obtained 0 min after TNF treatment while another one is obtained 30 min after TNF treatment. Each library yielded about 35 millions paired-end reads. Both libraries were processed by ChlA-PET tool (Li et al. 2010). Briefly, the head and tail ChlA-PET tags are first extracted from the sequences. The ChlA-PET tags were then mapped to the human reference genome (hg19) by Batmis (Tennakoon et al. 2012) and 10.8X10⁶ and 8.8X10⁶ are successfully aligned to hg19 for 0 min and 30 min samples, respectively. From the uniquely mapped paired-end tags (PETs), we extract all intra-chromosomal PETs. Precisely, intra-chromosomal PETs were defined as the head and tail of the PETs mapped onto the same chromosome with a genomic distance at least 5 Kbp. The intra-chromosomal PETs are then clustered to form ChlA-PET interaction clusters.

### Identification of enhancers in the chemokine TAD

We used the web tool PrESSto (Promoter Enhancer Slider Selector Tool, htp://pressto.binf.ku.dk/) to query the atlas of active, *in vivo* bi-directionally transcribed human enhancers identified analysing the FANTOM5 panel of tissue and primary cell samples (Andersson et al. 2014). Across the chemokine locus (chr4: 74,500,000-75,000,000) we searched for enhancers significantly expressed in the "blood vessel endothelial cell" and the "macrophage" facets (% expression ≥ 1, P value < 0.001, Benjamini-Hochberg adjusted FDR < 0.05). Every identified enhancer was associated to its overall % of expression and number of tags per million (tpm) in the facets, as well as its relative tpm in every single sample belonging to the analysed facets.

### Identification of long noncoding RNAs in the chemokine TAD

We used the ENSEMBL mart human database (Release GRCh37.p13) to identify all the IncRNAs mapping to the chemokine locus (chr4:74,500,000-75,000,000). We then queried the FANTOM5 human expression atlas to evaluate the promoter activity associated to the Transcription Start Site (TSS) of every identified lncRNA. The ZENBU omics interactive visualization system (human hg19, Feb 2015 updated release) was used to derive the expression levels of each lncRNA in 1829 human samples (primary cells, cancer cell-lines and post-mortem tissues) profiled either in steady state or time-course experiments.

### CRISPR synthesis

Software developed by the Zhang laboratory (http://www.genome-engineering.org/) was used to identify CRISPR candidate guide sequences (Cong et al. 2013). CRISPRs were generated using the protocol described by Cong et al., briefly, 1 µg of pX330 was digested with Bbsl for 30 min at 37°C, gel purified using QIAquick Gel Extraction Kit and eluted in elution buffer. Oligos were then phosphorylated using T4 PNK (NEB) and annealed using the following parameters: 37 °C for 30 min; 95 °C for 5 min and then ramp down to 25 °C at 5 °C/min. Annealed oligos were then ligated into the BbSI-digested pX330 vector using Quickligase (NEB). The ligation reaction was then treated with PlasmidSafe exonuclease to prevent unwanted recombination products at 37 °C for 30 min. Colony PCR was used on *E.* coli transformants to identify successful CRISPR clones. HUVECs were then microporated with the respective CRISPR using the Neon® Transfection System (Life Technologies) according to manufacturer's instructions. HeLas were transfected with Lipofectamine 2000 according to manufacturer's instructions. Nuclease activity was assessed by the surveyor assay.

### RNA interference

HUVECs and HeLas were transfected as per manufacturer's instructions with siRNAs targeting UMLILO (Dharmacon) using the Neon® Transfection System (Life Technologies) and Lipofectamine 2000 (Invitrogen) respectively. Total RNA was harvested 48-72 hr later using TRIzol (Invitrogen) according to manufacturer's instructions. The following siRNA's were used:
UMLILO siRNA 1: 5' AUC UUA AAU UAG AGG CGA AUU 3' (SEQ ID NO:54)
UMLILO siRNA 2: 5' CAU ACA AAU UCU CGC AGC AUU 3' (SEQ ID NO:55)
UMLILO siRNA 3: 5' AAG AGU UGG UAC ACG GUG AUU 3' (SEQ ID NO:56)
UMLILO siRNA 4: 5' GCA UAU UAA CCC UAC AAG UUU 3' (SEQ ID NO:57)

### RT-qPCR

For all RT-qPCR analyses, whole RNA was extracted using TRIzol (Invitrogen) according to manufacturer's instructions. cDNA was generated using the SuperScript III cDNA kit (Invitrogen). All qPCR primers (Table 1) were verified to produce specific products and to perform efficiently.

qPCR reactions were performed with technical triplicates on a CFX Real Time PCR detection system (Biorad) with SsoFast qPCR Supermix (Biorad). Relative quantification was performed using the delta delta Ct method with HPRT as a reference gene.

**Table 1: QPCR primers**

| **Target** | **Direction** | **Primer sequence** | **SEQ ID NO.** |
|---|---|---|---|
| UMLILO intron 1 | FW | 5' GGAAGGAGGGGAACATGGAA 3' | SEQ ID NO:2 |
| | RW | 5' CTACCTGACTCCCTCCCTCT 3' | SEQ ID NO:3 |
| UMLILO exon 3 | FW | 5' GGTACACGGTGAACATTTGCT 3' | SEQ ID NO:4 |
| | RW | 5' CAGCATCTCTCTGTCCACTGA 3' | SEQ ID NO:5 |
| IL8 gene | FW | 5' AGGGCCAAGAGAATATCCGA 3' | SEQ ID NO:6 |
| | RW | 5' GGACTTGTGGATCCTGGCTA 3' | SEQ ID NO:7 |
| CXCL1 gene | FW | 5' AGCTTGCCTCAATCCTGCAT 3' | SEQ ID NO:8 |
| | RW | 5' CCTCCTCCCTTCTGGTCAGT 3' | SEQ ID NO:9 |
| CXCL2 gene | FW | 5' CACAGTGTGTGGTCAACATTTCTC 3' | SEQ ID NO:10 |
| | RW | 5' ACACAGAGGGAAACACTGCATAA 3' | SEQ ID NO:11 |

### RNA FISH probes

RNA FISH was performed according to the protocol by Raj et al. 2008 using 48 20-mer probes (Biosearch) targeting the following genes: IL-8 intron 1 (SEQ ID NOS:58 - 101), CXCL1 intron 1 (SEQ ID NOs:187 - 227), CXCL2 intron 1 (SEQ ID NOs:149 - 186), UMLILO intron 1 and intron 2 (SEQ ID NOs:260 - 294) and 28 20-mer probes targeting eGFP (SEQ ID NOs:228 - 259). Each 20-mer bares a 3'-amino-modifier C6-dT. The amino group was subsequently conjugated to the following NHS-ester dyes: ATTO-488, ATTO-565, ATTO-647N (ATTO-TEC) or Alexa Fluor 647 (Invitrogen). Briefly, oligonucleotide probes were ethanol precipitated and resuspended in 0.1 M sodium tetraborate (Sigma). Approximately 0.3 mg of the NHS-ester dye (ATTO-TEC) was dissolved in dimethyl sulphoxide (Sigma). The dye solution was added to the probe solution and incubated overnight in the dark at 37°C. Following conjugation reaction, the probes were ethanol precipitated overnight, and resuspended in 0.1 M Triethyl ammonium (TEA, Sigma). Conjugated probes were separated and purified to enrich for dye-conjugated probes by reverse phase HPLC on a C18 column.

### Immuno-RNA FISH

For each experiment, early passage HUVECs on coverslips were grown to -80% confluence, treated with TNF, fixed in 3.7% formaldehyde for 10 mins at room temperature, then washed three times in PBS. Cells were permeabilized in ice-cold 90% methanol for ten minutes, then washed twice with PBS and incubated in blocking buffer (1% BSA/PBS) for 30 minutes at room temperature on an orbital shaker. Cells were then incubated in primary antibody solution (diluted in 1% BSA/PBS) for 1 hr. Double strand breaks were detected with rabbit polyclonal anti-phospho-histone H2A.X (Ser139) (Sigma). Goat polyclonal anti-WDR5 (SantaCruz) was used to detect WDR5 protein. Coverslips were then washed 5 times with wash buffer (0.05% Tween-20/PBS), following incubation with secondary antibodies conjugated to either Atto-488 or Atto-565 for 1 hr. Coverslips were then washed 5 times with wash buffer (0.05% Tween-20/PBS), and post-fixed with 3.7% formaldehyde/PBS for 10 minutes at room temperature, followed by further permeabilization in 70% ethanol overnight. For RNA FISH detection, coverslips were washed twice in PBS and incubated in wash buffer (10% formamide, 2X SCC - 1X SCC is 0.15 M NaCl plus 0.015 M sodium citrate) for 5 min. Cells were then hybridized overnight in a humidified chamber at 37 °C in 50 µl of Hyb buffer (10% dextran sulfate, 1 µg/µl *E. Coli* tRNA, 2 mM Vanadyl ribonucleoside complex, 0.02% RNAse-free BSA, 10% formamide) combined with 50 ng of single molecule FISH probes. Coverslips were then washed 3X (30 min each on the orbital shaker) in wash buffer (10% formamide, 2X SCC). Cells were then incubated in equilibration buffer (0.4% glucose, 2X SCC) for 5 min and counter stained with 1 µg/ml DAPI (4',6-diamidino-2-phenylindole; Life Technologies). Coverslips were mounted in glox buffer (3.7 µg/µl glucose oxidase, 1U catalase) and imaged.

### Image acquisition and processing

Cells were imaged on a custom built Nikon Ti Eclipse widefield TIRF microscope using a 100X N.A. 1.49 Nikon Apochromat TIRF oil immersion objective. Imaging was done using mercury lamp illumination through the appropriate filter sets at low camera gain in each of the fluorescent channels using an Andor iXion897 EMCCD camera cooled to -80°C. The microscope was controlled using µmanager open source microscope management software (NIH and UCSF, USA). A 20 ms exposure time was used for DAPI. Exposure times ranged from 200 to 500 ms for other dyes. Each field of view was captured as a series of images acquired on multiple focal planes through the samples, across a range of 2-10 µm in the axial plane. A 0.2 µm piezo step-size was used for these z-stacks. Chromatic aberration was verified before image capture by alignment of Focal Check Fluorescent Microspheres (Molecular Probes). Signal intensities were measured using Fiji (Schindelin et al., 2012). The contrast of pictures shown was adjusted to fit a 16bit grey scale. To facilitate the comparison between different fields of view on the same coverslip, IDV values were normalized relative to the intensity of fluorescent beads.

### Repair construct

To generate double stranded PCR product harboring the 20 and 18 bp homologous arms, the following primers were used:
(100 nmole and HPLC purification)
FW: 5' TTGAACCGGGTTTTCCAGTCACATATGGTGAGCAAGGGCGA 3' (SEQ ID NO:12)
RW: 5' ACTATGAAGACTCTTGGGTCACTTGTACAGCTCGTCCA 3' (SEQ ID NO:13)

The PCR product was purified by QIAquick PCR Purification Kit (Qiagen) prior to transfection.

### Chromatin immunoprecipitation

For each ChlP experiment, 1x10⁷ cells were grown to ∼80% confluence, fixed in 3.7% formaldehyde for 10 mins at room temperature, then washed three times in PBS. Then, glycine was added to a final concentration of 125 mM to the media and incubated with shaking for 5 min at RT. Cells were rinsed 2X with 10 ml cold PBS, scraped into 5 ml cold PBS and centrifuged for 5 min at 1,000 g. The resultant pellet was resuspended in FA Lysis Buffer (50 mM HEPES-KOH pH 7.5; 140 mM NaCl; 1 mM EDTA pH 8; 1% Triton X-100; 0.1% Sodium Deoxycholate; 0.1% SDS; Protease Inhibitors (add fresh each time). The nuclear pellet was then sonicated with the Covaris S220 Sonicator to an average fragment size of 500 to 1000 bp and centrifuged for 30 seconds at 4 °C, 8,000 g. The supernatant was transferred to a new tube. 50 µl of each sonicated sample was removed as the INPUT to obtain the DNA concentration. 25 µg of protein was used per IP. Protein concentration was calculated using the Bradford assay. Each sample was diluted 1:10 with RIPA buffer (50 mM Tris-HCl pH 8.0; 2 mM EDTA pH 8.0; 1% NP-40; 0.5% Sodium Deoxycholate; 0.1% SDS; Protease Inhibitors (add fresh each time). 2 µg of antibody was added per 25 µg of protein. The following ChlP grade antibodies were used: anti-H3K4me3 (Abcam), MED12 (Bethyl laboratories), anti-RNA Pol II Ser5 (Abeam), anti-WDR5 (H-35 SantaCruz). 20 µl of magnetic protein A/G beads (Resyn Biosciences) (pre-absorbed with sonicated single stranded salmon sperm DNA at 1.5 µg / 20 µl beads) to all samples and incubated at 4 °C overnight with rotation. Beads were washed 3X with 1 ml wash buffer (0.1% SDS; 1% Triton X-100; 2 mM EDTA pH 8; 150 mM NaCl; 20 mM Tris-HCl pH 8) and then 1X with final wash buffer (0.1% SDS; 1% Triton X-100; 2 mM EDTA pH 8; 500 mM NaCl; 20 mM Tris-HCl pH 8). DNA was eluted with 120 µl of Elution Buffer (1% SDS; 100mM NaHCO₃) to the protein A/G beads and incubated at 37 °C for 15 min with rotation. DNA from ChlP and INPUT samples was purified using phenol:chloroform extraction and ethanol precipitated in presence of 10 µl glycogen (5 mg/ml) and taken up in 100 µl H₂O.

**Table 2: ChlP primers**

| **Target** | **Di rection** | **Primer sequence** | **SEQ ID NO.** |
|---|---|---|---|
| IL8 | FW | 5' TGGGCCATCAGTTGCAAATC 3' | SEQ ID NO:14 |
| | RW | 5' AGTGAGATGGTTCCTTCCGG 3' | SEQ ID NO:15 |
| CXCL1 | FW | 5' ACGTGGGTCTAAGGGATCTG 3' | SEQ ID NO:16 |
| | RW | 5' GGGTCTGACTGTCTTGCGTA 3' | SEQ ID NO:17 |
| CXCL2 | FW | 5' CTGTGGTGGTTCTCAGGGAT 3' | SEQ ID NO:18 |
| | RW | 5' TGGACTCTGAGACTCTGGGA 3' | SEQ ID NO:19 |
| CXCL3 | FW | 5' TCTGGAATCCGAGACGATGG 3' | SEQ ID NO:20 |
| | RW | 5' GACAGGAAAGGCACGACTTC 3' | SEQ ID NO:21 |
| SAMD4A | FW | 5' GAGCTTTGGGTGGAGAGAGT 3' | SEQ ID NO:22 |
| | RW | 5' TTACTCCTCCTCCTCCTCCC 3' | SEQ ID NO:23 |
| UMLILO | FW | 5' TGTCCAAATCCACATTGACAGT 3' | SEQ ID NO:24 |
| | RW | 5' GGAGTGTTGCTGCGAGAATT 3' | SEQ ID NO:25 |

### RNA immunoprecipitation with targeted mass spectrophotometry

For each experiment, 1X10⁷ cells were grown to ∼80% confluence, then washed three times in PBS. Briefly, cells were resuspended in Buffer A (10 mM HEPES pH 7.5, 1.5 mM MgCl₂, 10 mM KCl, 0.5 mM DTT, 1.0 mM PMSF), lysed in 0.25% NP40, and fractionated by low speed centrifugation. The nuclear fraction was resuspended and lysed in Buffer C (20 mM HEPES pH 7.5, 10% glycerol, 0.42 M KCl, 4 mM MgCl₂, 0.5 mM DTT, 1.0 mM PMSF). Either the nuclear or cytoplasmic fraction was incubated with biotinylated oligonucleotide probes (SEQ ID NOs:295 - 314) tiling exonic UMLILO in hyb buffer (100 mM Tris-HCI pH 7.0; 500 mM NaCl; 10 mM EDTA pH 8.0; 15% formamide; 1mM DTT; 1% SDS; 0.1 U/µl Superase) at 37 °C for 4 hr. 20 µl of magnetic protein A/G beads (Resyn Biosciences) (pre-absorbed with sonicated single stranded salmon sperm DNA at 1.5 µg / 20 µl beads) was added to all samples and incubated at 37 °C for 1 hr with rotation. Beads were washed 3X with wash buffer (2X SCC; 0.5% SD; 1 mM DTT). Proteins were eluted with SDS loading buffer and subject to SDS-PAGE analysis. Gels were stained using Colloidal Coomasie and protein bands of interest were in-gel trypsin digested as per the protocol described in Shevchenko *et* a/., 2007. In short, gel bands were destained using 50 mM NH₄HCO₃/50% MeOH followed by in-gel protein reduction (50 mM DTT in 25 mM NH₄HCO₃) and alkylation (55 mM iodoacetamide in 25 mM NH₄HCO₃). Proteins were digested overnight at 37 °C using 5-50 µl, 10 ng/µl tryspin depending on the gel piece size. Digests were resuspended in 40 µl, 2% acetonitrile/0.2% formic acid and analysed using a Dionex Ultimate 3000 RSLC system coupled to an AB Sciex 6600 TripleTOF mass spectrometer. Peptides were first desalted on an Acclaim PepMap C18 trap column (75 µm X 2 cm) for 8 min at 5 µl/min using 2% acetonitrile/0.2% formic acid, then separated on Acclain PepMap C18 RSLC column (75 µm X 15 cm, 2 µm particle size). Peptide elution was achieved using a flowrate of 500 nl/min with a gradient of 4-60% B in 30 min (A: 0.1% formic acid; B: 80% acetonitrile/0.1% formic acid). Nano-spray was achieved using a NanoSpray III source assembled with a New Objective, PicoTip emitter. An electrospray voltage of 2.3 kV was applied to the emitter. Precursor spectra were acquired in the range 400 - 1500 m/z (0.25 s accumulation time). Product ion spectra were acquired for 18 precursors reporting on 5 targeted proteins. Product scans were recorded in the mass range 100 - 1500 m/z, high sensitivity mode, with an accumulation time of 0.1 s for a total cycle time of 2.1 s. Data processing was performed in Skyline (Shevchenko et al. 2007). MS1 filtering was performed at 30,000 whilst MS2 at 15,000 resolution with a match tolerance of 0.025 m/z.

### 3C assay

3C experiments were performed as described previously by Hagege et al. with some minor modifications (Hagege et al. 2007). Briefly, a single-cell suspension of 8X10⁶ HeLa cells was crosslinked with 1% formaldehyde in 10% (v/v) FBS/PBS for 10 min at room temperature and quenched by adding 0.125 M glycine and incubating for 5 min on ice. Cells were then pelleted by centrifugation at 225 g for 8 min at 4 °C and lysed on ice in 3C lysis buffer (10 mM Tris-HCl, pH 7.5; 10 mM NaCl; 0.2% NP-40; 1X complete protease inhibitor (Roche) for 30 min and subsequently dounce homogenized. Nuclei were collected by centrifugation at 400 g for 5 min at 4 °C. The pellets were resuspended in 1.2X Buffer R (Thermo Scientific) with 0.3% SDS and incubated at 37 °C for 1 hr while being shaken at 250 rpm. The SDS was sequestered by adding 2% Triton X-100 and incubating this for 1 hr at 37 °C with shaking. 550 U of *Hind*III (10 U/ul) (Thermo Scientific) was then added and incubated overnight at 37 °C with shaking. The digestion was terminated with 1.3% SDS at 65 °C for 20 min. 6.125 ml of 1.15X T4 Ligation Buffer (Thermo Scientific) with 1% Triton X-100 was added and incubated at 37 °C for 1 hr with mild shaking (150 rpm). Samples were allowed to cool to room temperature before 100 U T4 DNA ligase (Thermo Scientific) was added and incubated at 16 °C for 4 hr, with gentle shaking (90 rpm), followed by an additional 30 min at room temperature. Crosslinks were reversed with 300 µg of proteinase K and overnight incubation at 65 °C. The following day, 300 µg of RNase A was added to the sample and incubated at 37 °C for 30 min. Finally, genomic DNA was purified by phenol chloroform extraction followed by isopropanol precipitation. Ligation events were detected using unidirectional, fragment specific primers (Table 3). qPCR reactions were performed with technical triplicates on a CFX Real Time PCR detection system (Biorad) with SsoFast qPCR Supermix (Biorad). A putative genomic interaction was used to correct for variations between 3C library preparations. Interaction frequencies were calculated relative to the random interaction frequencies as determined with a BAC template (RPCI-11 447E20) spanning the genomic region of interest.

**Table 3: 3C primers**

| **Primer** | **Fragment location** | **Sequence** | **SEQ ID NO** |
|---|---|---|---|
| Anchor | chr4:74604292-74606315 | 5' TCCTCTGACATAATGAAAAGATGAGGGTGC 3' | SEQ ID NO:26 |
| 1 | chr4:74499669-74501601 | 5' AAAATAGAAACCCTGAATGTACCGGTAACA 3' | SEQ ID NO:27 |
| 2 | chr4:74511769-74524359 | 5' TCAAATCCGTGATCAGCATTACCAAGCCAT 3' | SEQ ID NO:28 |
| 3 | chr4:74527725-74529421 | 5' ATTGGAAGGCTAAATATACTTACATGGC 3' | SEQ ID NO:29 |
| 4 | chr4:74530103-74534800 | 5' TTTTAAAAGCAGCACTAGTGTATCCGG 3' | SEO ID NO:30 |
| 5 | chr4:74546061-74548099 | 5' AAAGAGCTCAATGTGTGTTACTAAAGAATG 3' | SEQ ID NO:31 |
| 6 | chr4:74549704-74551014 | 5' AAAGGTAAGCAATAATTGGCCCATATCTC 3' | SEQ ID NO:32 |
| 7 | chr4:74553813-74555419 | 5' AAAAGTGATACATGTTCATTGCATTTAAAC 3' | SEQ ID NO:33 |
| 8 | chr4:74578660-74585594 | 5' CGTAAGGCTGGTCAAGGTATGCTGAG 3' | SEQ ID NO:34 |
| 9 | chr4:74585594-74587345 | 5' TTCTTCATTATGCTAGTGTTGTGTATTGTG 3' | SEQ ID NO:35 |
| 10 | chr474587345-74591147 | 5' TGAAATTAGAGAAAAACATGTACTTAGGGA 3' | SEQ ID NO:36 |
| 11 | chr4:74591147-74596339 | 5' CACCCCGCCCATTTGATGAACTGTTT 3' | SEQ ID NO:37 |
| 12 | chr4:74596459-74601876 | 5' ACTTCAAACTCCAAACTCCACCGATTTG 3' | SEQ ID NO:38 |
| 13 | chr4:74601963-74604292 | 5' CCAACATCACTGAAGCAAAGAAACTTGGAG 3' | SEQ ID NO:39 |
| 14 | chr4:74607958-74612686 | 5' TCTGCTTGTACGTAGGTATGTAGATT 3' | SEQ ID NO:40 |
| 15 | chr4:74722664-74725426 | 5' GCTAGCAACCAACTCTTTAAGAATACAGCC 3' | SEQ ID NO:41 |
| 16 | chr4:74736063-74750395 | 5' AGAAACACAACGATACAATGTGAAA 3' | SEQ ID NO:42 |
| 17 | chr4:74751595-74756828 | 5'AGTGTGACTCCAAATAACCTAGTTTGCTA 3' | SEQ ID NO:43 |
| 18 | chr4:74756828-74759327 | 5' ACACAGTCATAATCACAACCCCAGTC 3' | SEQ ID NO:44 |
| 19 | chr4:74899318-74900128 | 5' TCAGACTCATGGGCTCAGTTGATTC 3' | SEQ ID NO:45 |
| 20 | chr4:74901502-74902795 | 5' GGCTGACACATTATGGTCTCCCACTAAATA 3' | SEQ ID NO:46 |
| 21 | chr4:74903866-74908292 | 5' GAAACATGTCAAGAGGCCGTGGACATTT 3' | SEQ ID NO:47 |
| 22 | 74917831-74918715 | 5' AGTGTGAAACAAAACGAGAAGGGAAG 3' | SEQ ID NO:48 |
| 23 | 74964374-74967509 | 5' AAATTATTTGCTTTAGGAAGGGAAGTAGAA 3' | SEQ ID NO:49 |
| 24 | 74972881-74981053 | 5' CATAGGAGAGACTGCGACAGAAATTCCATT 3' | SEQ ID NO:50 |
| 25 | 74981053-74983881 | 5' TTACAACTCCTACAACCGTGCTTGGTACAT 3' | SEQ ID NO:51 |
| GAPDH control F1 | chr12:6639882-6641136 | 5' TGCCAATCTCCTTGTTTTCTAATG 3' | SEQ ID NO:52 |
| GAPDH control F2 | chr12:6641136-6645917 | 5' TATTCCCCCAGGTTTACATGTTC 3' | SEQ ID NO:53 |

### EXAMPLE 2

### The ELR+ CXC chemokines engage in pre-formed chromosomal contact

Recent Hi-C and 5C studies show that several classes of innate immune genes are organized into TADs (Jin et al. 2013). Tumor necrosis factor (TNF) has been shown to strongly induce the expression of the ELR+ CXC chemokines (IL8, CXCL1, CXCL2 and CXCL3; hereafter referred to as chemokines) in fibroblasts and endothelial cells (Paulsen et al. 2013; Jin et al. 2013). Therefore, we utilized previously published Hi-C data to examine the higher order chromatin organization of the chemokine TAD in diploid fibroblasts (IMR90), primary endothelial cells (HUVECs) and other cell types. Analysis of Hi-C data across the proinflammatory chemokine locus revealed that the chemokine TAD spans a region of ∼500 Kbp, and is well defined in unstimulated IMR90s, HUVECs and other immune and non-immune cells (Figure 1). Further, the chemokine TAD in humans may be further divided into two smaller subdomains with the super-enhancer region, IL8 and CXCL1 being located within the same subdomain (∼230 Kbp), whilst CXCL3 and CXCL2 are located within the second sub-domain (∼290 Kbp).

Although Hi-C reveals TAD structure, it does not reveal the status of interacting chromatin at actively transcribing genes. The ChIA-PET technique incorporates a ChIP step, and therefore, enables the detection of chromosomal contacts that are mediated by specific proteins. We analysed ChIA-PET data from a library constructed with an antibody that enriches for actively transcribing RNA Pol II (dual phosphorylated at Ser2/Ser5) (Papantonis et al. 2012). To minimise amplification effects, two or more reads that were either identical, or overlapped by +/- 2bp were classified as a single PET. ChIA-PET analysis confirmed that upon gene activation with TNF for 30 min there were numerous PETs/contacts that occurred between the super-enhancer region and the chemokine genes, forming a putative 'inverted rosette' structure (Figure 2). Further, contacts detected by ChIA-PET between interacting chemokine genes, were confined exclusively to the region encompassing the chemokine TAD. Therefore, the chemokine TAD structure may play a fundamental role in chemokine gene regulation by creating an insulated neighbourhood for transcriptional regulators, chromatin remodelers and transcribed elements. Accordingly, unraveling the mechanism whereby this TAD is regulated could reveal novel mechanisms of chemokine regulation.

A previous report demonstrated that the 5' insulator element of the chemokine TAD engages in chromosomal contact prior to chemokine activation by TNF. Therefore, in order to investigate whether chromosomal contact between UMLILO and the chemokine was influenced by TNF treatment, we performed 3C analysis across the entire chemokine TAD. Corresponding to other innate immune genes examined in the study by Jin et al. we observed that the chemokine TAD structure is formed prior to gene activation by TNF. The transcriptional regulation of chemokines is tightly controlled, permitting chemokine expression only upon receipt of the correct signals. For this reason, in resting cells, the promoters of chemokine genes are primed to allow their swift activation and the immediate mobilization of the neutrophil response. Taken together, this suggested a relationship between pre-formed chromosomal contact and the poised nature of the chemokine genes.

### UMLILO is a super-enhancer resident IncRNA transcribed within the chemokine TAD

TADs containing enhancer-dense regions or super-enhancers (SE) may include co-regulated genes that are exceptionally sensitive to external stimuli, permitting fine-tuned and rapid control of gene regulation. ChIPseq analysis across the chemokine TAD revealed a previously identified SE that is atypically large (∼80 Kbp) and highly enriched for the eRNA chromatin marks, H3K4me1 and H3K27Ac (Figure 3). This region has also been shown to be densely occupied by chromatin regulators including subunits of the Mediator complex and the chromatin reader BRD4 (Brown et al. 2014). Sites enriched for SE chromatin marks corresponded to contact regions identified by ChIA-PET (Figure 3), suggesting that these regions may give rise to eRNA transcription. Analysis of FANTOM 5 CAGE data identified several eRNAs which corresponded to H3K4me1 and H3K27Ac peaks emanating from within the SE (Figure 3). To investigate the existence of novel IncRNAs transcribed within the chemokine TAD, we queried the FANTOM 5 data for evidence of CAGE peaks not associated to any Entrez/GENCODE (coding) genes nor the typical eRNA chromatin marks. Our analysis allowed us to identify a transcript that was expressed from the positive strand and across numerous cell types, that we named UMLILO (Upstream Master LncRNA of the Inflammatory chemokine LOcus). This novel 575 nucleotide (nt) long transcript is a spliced and polyadenylated IncRNA that does not possess the typical eRNA histone modifications of H3K4me1 and H3K27Ac (Figure 3). Though lacking the typical eRNA chromatin marks, ChIA-PET analysis revealed that the region encoding UMLILO formed extensive Pol II-mediated chromosomal contacts with the chemokine genes, particularly its closest chemokine neighbor, IL8 (Figure 3). Together with the 3C data, this suggested that in unstimulated cells, UMLILO might be brought in close proximity to the chemokine genes by pre-formed chromosomal looping (Figure 4). This data demonstrated that UMLILO is a novel IncRNA emanating from the SE, lacking typical eRNA chromatin marks, and revealed its positioning in close proximity to the chemokine genes by pre-configured nuclear architecture.

### EXAMPLE 3

### UMLILO transcription precedes chemokine gene activation

The regulation of chemokine expression occurs predominantly at the transcriptional level. Historically, chemokine transcription is assayed at a population level, which lacks the resolution to reveal the exact site of active chemokine transcriptional activation. Importantly, this can only be achieved through single cell studies able to achieve single molecule resolution. Thus in order to directly observe the site of transcription at a single cell level, we designed single molecule RNA FISH (smFISH) probes to target the introns of the chemokine genes (Raj et al. 2008). As introns are typically spliced and degraded cotranscriptionally, these probes label the transcriptional start site (TSS). Of the four chemokine genes within the TAD, CXCL3 does not possess an intron and was therefore excluded from our single cell analysis experiments. Using intronic smFISH, we were able to show that the chemokines are only induced in HUVECs following TNF induction (Figure 5). Furthermore, the RNA FISH foci of co-expressed chemokine genes always co-localized (Figure 5). This provided supporting evidence to the Hi-C and ChIAPET data that these chemokine genes engage in chromosomal contact, and are organized into TADs. At an early time point following TNF stimulation (30 min) approximately 50% of all alleles displayed chemokine transcriptional activation. The expression frequency of the chemokine increased significantly upon stimulation with TNF for 24 hr, reaching ∼95% for IL8, ∼ 90% for CXCL1 and ∼85% for CXCL2 (Figure 5). Importantly, after 30 min post TNF treatment, even though not every allele of IL8, CXCL1 and CXCL2 was expressed simultaneously, approximately 90% of cells displayed expression of either IL8, CXCL1 or CXCL2. Therefore, since all of the individual chemokines are able to induce neutrophil chemotaxis, this suggests that the transcriptional activation of at least a single chemokine per cell may be sufficient to mount a successful neutrophil response. Interestingly, this single cell perspective revealed that the expression of IL8 was not uniformly accompanied by CXCL1 and CXCL2, implicating IL8 as the dominant chemokine in this TAD. This dominance may be attributed to the proximity of IL8 to the super-enhancer, both of which are located within the same subdomain.

To investigate the transcriptional activation of UMLILO in HUVECs at a single cell level, we designed RNA FISH probes targeting intron 1 and intron 2 of UMLILO. RNA FISH revealed that the signal from both introns consistently colocalized (Figure 6) at the same site of transcription. This suggests that the intronic portions of UMLILO are not redistributed to different subcellular locations. Further, intronic FISH signal was observed in both unstimulated and TNF challenged HUVECs. Although levels of intronic UMLILO were elevated shortly after TNF treatment, they dropped back down to resting UMLILO levels after longer durations of TNF stimulation (Figure 6). This single cell RNA FISH data supported the CAGE data showing UMLILO to be transcribed in unstimulated HUVECs and prior to chemokine gene activation. In addition, we used RNA FISH to visualize the exonic portion of UMLILO. However, as the exonic form of UMLILO is only 575 nt, RNA FISH signals were difficult to detect reliably. Therefore, in order to investigate the stability of UMLILO after TNF treatment we performed qPCR on both an intronic and exonic portion of UMLILO. We observed the same pattern of intronic expression of UMLILO as we did with the RNA FISH approach. Interestingly, we observed that the exonic portion of UMLILO was slightly elevated after both 30 min and 24 hr TNF treatment. Since the level of the intronic portion of UMLILO declined 24 hr post TNF treatment, it suggests that the transcriptional activation of UMLILO is reduced at later time points after TNF treatment, but that the exonic portion of UMLILO is stabilized.

The expression of UMLILO in resting endothelial cells coincides with UMLILO being brought in close proximity to the chemokine gene promoters by pre-formed chromosomal loops (Figure 6). However, genome-wide 3C and transcriptome approaches fail to reveal the temporal regulation of UMLILO vis-à-vis the transcriptional regulation of the chemokine genes. Therefore, we performed simultaneous intronic RNA FISH on UMLILO as well as the chemokine genes in the presence of TNF. Intronic RNA FISH revealed that overlapping co-localization between intronic UMLILO and the chemokine genes was rarely observed, suggesting that UMLILO transcription is decoupled to chemokine gene expression (Figure 7). Interestingly, the expression of UMLlLO and the chemokines was frequently observed to occur on the periphery of the nucleolus. The expression of UMLILO prior to chemokine transcriptional activation suggests a relationship between UMLILO expression and chemokine gene regulation.

### UMLILO is conserved in higher vertebrates, but lacks a homolog in rodents

The rules governing the transcriptional regulation of IncRNAs are poorly understood, especially with respect to IncRNAs that are expressed in unstimulated cells. UMLILO was identified by CAGE to be a low abundant transcript, which is expressed in a wide variety of challenged and unchallenged immune and nonimmune cells, including primary endothelial cells. Due to the fact that most of these cell types are able to induce neutrophil chemotaxis, UMLILO may be an important regulator of the innate immune response. The chemokines comprise a critical component of the innate immune system, which in mammals is highly evolutionarily conserved. Therefore, we investigated the sequence conservation of UMLILO across metazoans. We observed that UMLILO is highly conserved in higher vertebrates, but no homolog of UMLILO exists in mice, a model used for a multitude of inflammatory disorders (Figure 8). In addition to the absence of an UMLILO homolog, mice do not express IL8 (Figure 8). Further, corresponding to the absence of UMLILO and IL8, mice blood has been shown to contain lower levels of neutrophils than human blood. Taken together this indicates that there may exist key differences between ELR+ CXC chemokine-dependent neutrophil chemotaxis across mammals.

### EXAMPLE 4

### UMLILO transcription is impervious to perturbation of super-enhancer transcriptional regulators and is necessary for chemokine transcription

Super-enhancers (SE) are densely occupied by chromatin regulators, including the mediator subunit, MED12, and BRD4 (Figure 9). Therefore, genes regulated by SEs have been shown to be exquisitely sensitive to depletion of either MED12 or abrogation of BRD4 chromatin occupancy by small molecule inhibitors. The expression of SE-resident IncRNAs has been shown to be higher than that of non SE-resident IncRNAs. However, it remains unknown whether the SE directly influences IncRNA transcriptional regulation. UMLILO resides within a SE that is engaged in pre-formed chromosomal contact with the chemokine genes. Therefore, we investigated whether ablating SE function would influence UMLILO expression.

Many critical proinflammatory genes, such as the chemokines, are poised prior to activation, allowing their rapid transcription upon the arrival of signal-dependent transcription factors. The promoters of the chemokine genes are enriched for histone H3 K4 mono-, di- and trimethylation (H3K4me3), which are active chromatin marks catalyzed by the MLL family of methyltransferases. These chromatin modifications permit RNA Pol II to access the promoter, where it awaits further signals to initiate active transcription. The release of "paused" Pol II is facilitated by the recruitment of the transcriptional elongation factor P-TEFb by the chromatin reader, BRD4. In the case of proinflammatory genes, various signals such as TNF or lipopolysaccharide (LPS) induce the nuclear translocation of NF-κB. The p65 subunit of NF-κB then associates directly with BRD4, prompting the release of the "paused" inflammatory genes (Kaikkonen et al. 2013). In endothelial cells, TNF induction has been demonstrated to redistribute the BRD4 payload away from resting SE, to establish new SE domains. Critically, these newly activated SEs are adjacent to canonical inflammatory genes. Therefore, we used the bromodomain inhibitor, JQ1, to investigate how the loading of the super-enhancer with BRD4/NF-κB influenced UMLILO and chemokine expression. Corresponding to a previous report, (+) JQ1 but not its enantiomer (-) JQ1 abrogated chemokine transcriptional activation (Figure 10). Interestingly, JQ1 did not influence UMLILO transcription (Figure 10). This supports the observation that UMLILO is expressed prior to chemokine transcription and therefore, uninfluenced by preventing the release of "paused" pol II. Further, this reveals a possible alternative regulatory mechanism for UMLILO transcription, which is independent to CXC chemokine transcriptional regulation.

Through their interaction with MED12, eRNAs have been proposed to be the architects of chromatin organization and therefore putatively TADs. We found that depleting MED12 abrogates the expression of the CXC chemokines (Figure 11). This suggests that, through the activity of eRNAs, the super-enhancer region in the cytokine TAD may be coordinating chemokine expression. A previous study showed that depleting subunits of the Mediator complex did not after the expression of the activating RNA, ncRNA-a7. Similarly, knockdown of MED12 did not influence UMLILO expression (Figure 11). Collectively, this revealed that although the super-enhancer is clearly important to chemokine transcriptional activation, it does not seem to have an impact on UMLILO transcriptional regulation.

It remains unknown whether IncRNAs arising from within super-enhancers are an artifact produced due to the elevated levels of transcriptional regulators occupying super-enhancers, or if they are functionally necessary for target gene expression. 3C-based approaches had thus far shown that the chemokine TAD assembles into an 'inverted rosette', with pre-formed contact occurring between the chemokines and UMLILO (Figure 2). Further, coinciding with the pre-formed contact was the transcriptional activity of UMLILO in unstimulated cells (Figure 6). Therefore, we sought to investigate whether the expression of UMLILO prior to chemokine activation is necessary to their transcription. We employed small interfering RNAs (siRNA) to knockdown UMLILO RNA and investigated chemokine expression by RT-qPCR. siRNA knockdown of UMLILO, in both HUVECs and HeLas, was sufficient to significantly abrogate chemokine expression, but not another TNF-induced gene located in a different TAD (Figure 12). This revealed UMLILO transcription is not an artifact of the SE, but is indeed necessary for chemokine gene activation. In sum, this revealed that both the super-enhancer and UMLILO act as independent factors to drive chemokine transcription.

### EXAMPLE 5

### UMLILO acts in cis to regulate chemokine expression

The activity of *cis*-acting IncRNAs appears to be dependent on these IncRNAs being transcribed at an exact genomic location. Indeed, ectopic expression of HOTTIP was insufficient to rescue the effects of HOTTIP depletion (Wang et al. 2011). siRNA approaches fail to address whether a IncRNA is acting in *cis* or in *trans.* Therefore, we devised a single cell microscopy-based approach, using the CRISPR-Cas9 system to erase the genomic sequence encoding UMLILO and replace it with an eGFP reporter sequence in primary HUVEC cells (Figure 13). Unlike artificial tethering systems (Wang et al. 2011), this approach incorporates a critical aspect, namely the endogenous nuclear environment. Successful removal of UMLILO from its genomic locus and its replacement with the DNA sequence of eGFP was detected using RNA FISH probes targeting eGFP RNA. eGFP transcription was driven by the endogenous UMLILO promoter (Figure 14). Following repair, we observed distinct eGFP foci in the nucleus of ∼25% of cells, with ∼18% of cells displaying a single focus, and ∼7% of cells displaying dual eGFP foci (Figure 15). Due to the low activity of the endogenous UMLILO promoter, eGFP foci in the cytoplasm were seldom observed. Importantly cells displaying a single eGFP focus were still able to express UMLILO from the opposite allele, whereas UMLILO expression was never observed in cells displaying dual eGFP foci. Therefore, the comparison between cells displaying a single or dual eGFP foci was used as an indication of *cis* or *trans* effects of UMLILO. In agreement with siRNA data, co-localization between eGFP foci and IL8 was never observed (Figure 15). This provided supporting evidence for the siRNA data where the silencing of UMLILO resulted in the significant downregulation of the chemokine genes.

To investigate whether UMLILO exerts its effects in *cis* or *trans,* we compared cells displaying a single eGFP focus but still expressing UMLILO from the intact allele, to cells displaying dual eGFP foci. Interestingly, in cells displaying no eGFP foci, expression of IL8 and CXCL2 is comparable to the mock transfected cells (Figure 16). However, in cells displaying a single eGFP focus, the expression of IL8 and CXCL2 was reduced significantly (Figure 16). Importantly, this is observed despite the fact that these cells are presumed to still be able to express UMLILO from the opposite allele. This suggests that UMLILO is unable to exert its effect in *trans,* but rather exerts its activity from a precise genomic location in *cis* within the chemokine TAD. Strongly supporting the siRNA data, chemokine expression was never observed in cells displaying dual eGFP foci (Figure 16). This definitively proves the necessity of the UMLILO transcript for the transcriptional activation of the chemokine genes.

### EXAMPLE 6

### UMLILO interacts with the WAR complex to maintain H3K4me3 epigenetic regulation of chemokine genes

The data thus far had shown UMLILO to be an enhancer-like *cis*-acting IncRNA (Figures 13, 14, 15 and 16) that is expressed at low abundance in unstimulated cells and brought into close proximity to target genes by chromosomal looping. As HOTTIP shares the same attributes (Wang et al. 2011), we hypothesized that UMLILO would share the same protein binding partners. HOTTIP is a IncRNA of the human *HOXA* locus that binds directly binds to the WDR5 protein to recruit the MLL H3K4 methylase complex to maintain H3K4me3 across the *HoxA* gene promoters (Wang et al. 2011). MLL1 is one of six members of the MLL family, which regulates hematopoiesis through the regulation of H3K4 methylation of the *Hox* genes. MLL1 has been implicated as the H3K4 methyltransferase of the innate immunity genes as it has been found to selectively regulate the activation of TNF- and LPS-induced gene activation (Wang et al. 2012). MLL1 activity is mediated via direct protein-protein interactions with the WAR complex (comprised of WDR5, Ash2L and RbBP5) as well as other regulators.

UMLILO was recovered from the nuclear fraction of cell lysates using biotinylated single stranded DNA probes tiling the exonic portion of the IncRNA. In order to verify the components of the WAR complex we used a targeted Multiple Reaction Monitoring with High Resolution Mass Spectrometry (MRM^{HR}) method (Figure 17). Targeted MS approaches, such as MRM^{HR}, are generally accepted as more sensitive, specific and reproducible compared to discovery Mass Spectrometry (MS) methods. Unlike standard discovery-based approaches, MRM^{HR} allows the quantification of proteins in the samples. Using MRM^{HR}, we confirmed the presence of WDR5, but not Ash2L, RbBP5 or DY30, in cytoplasmic or nuclear-extracted UMLILO pull downs (Figure 17). Consistent with previous studies demonstrating the promiscuity of RNA-binding by the C-terminal of MLL1, we detected its non-specific binding to RNA, including control IncRNA lincRNAp21 as well as UMLILO pulldowns (Wang et al. 2011). Immunofluoresence labeling revealed that WDR5 is a low abundant protein located predominantly in the nucleus in discrete punctate spots. Immuno-RNA FISH revealed that distinct foci of WDR5 colocalized with UMLILO (Figure 17) providing supporting evidence for the UMLILO-WDR5 interaction detected by the mass spectrometry approach.

To investigate whether UMLILO was targeting the WDR5-MLL1 methylase complex to the chemokine promoters, and to interrogate the specificity of the UMLILO-WDR5 interaction, we performed siRNA knockdown of UMLILO followed by WDR5, H3K4me3, Pol II Ser5 and MED12 CHIP analysis of the chemokine promoters. Consistent with the lack of eRNA chromatin marks on UMLILO, siUMLILO did not alter MED12 occupancy on the chemokine promoters (Figure 18). Corresponding to the mass spectrometry and IF-FISH data, we observed that siUMLILO modulates WDR5 binding as well as Pol II-Ser5 and H3K4me3 occupancy on chemokine promoters (Figure 18). This indicated that the loss of chemokine expression observed upon UMLILO knockdown, or CRISPR-mediated deletion, is likely to be due to loss of H3K4me3 across the chemokine promoters.

Activity of MLL1 is particularly reliant on its association with WDR5, a multifunctional adapter protein that identifies H3K4 methylation marks on the promoters of genes as well as binds to MLL1. As a consequence, small molecule inhibitors that prevent the WDR5/MLL1 interaction lead to a significant reduction in MLL1 methyltransferase activity (Cao et al. 2014). We introduced such a small molecule inhibitor, MM102, into HUVECs to prevent the WDR5/MLL1 interaction, and observed a significant reduction in chemokine gene transcription (Figure 19). These data conclusively demonstrated the molecular mechanism by which UMLILO acts. Further, this reveals that the UMLILO-WDR5 interaction is specific and can be targeted to ablate chemokine expression through loss of H3K4me3 across chemokine gene promoters.

### EXAMPLE 7

### Depleting UMLILO does not abrogate chromosomal contact across the chemokine TAD

The mechanism underpinning the pause release of Pol II on the promoters of the primed inflammatory genes is well studied. However, the upstream molecular events that coordinate the poised state of inflammatory genes, such as the chemokines, are poorly understood, Important outstanding questions include; what directs the preinitiation complex to the chemokine promoters to facilitate the methylation of the chemokine genes? And, how does 3D chromatin topology influence this regulation? In a resting state, the chemokine TAD is comprised of pre-formed chromosomal loops. Moreover, through the activity of UMLILO, the promoters of the chemokine genes are marked with active epigenetic marks prior to chemokine gene activation (Figure 18). This suggests a relationship between the preformation of the chemokine TAD and the epigenetic activation of the chemokine genes.

5C analysis revealed that although HOTTIP is brought in close proximity to the *HoxA* genes by chromosomal looping, the higher order chromatin structure of the *HoxA* locus remained unaltered in HOTTIP-depleted cells (Wang et al. 2011). However, this approach lacked the resolution to determine whether intra-TAD contacts were formed prior to *HoxA* gene activation. Therefore, we sought to investigate whether the pre-formed chromosomal contacts occurred upstream of the epigenetic activation of the chemokines. Across three biological replicates, siUMLILO followed by 3C analysis revealed that chromosomal contact across the chemokine TAD remained unaffected in the absence of UMLILO (Figure 20). Notably, chromosomal interactions between UMLILO and IL8 remained unaltered (Figure 21). In sum these chromatin architecture data revealed that stable, or pre-formed, chromosomal interactions in the chemokine TAD occur upstream of H3K4me3 epigenetic activation. Further, the 3D chromatin topology in the chemokine TAD contributes directly to the positioning of UMLILO in the appropriate genomic location, to allow the epigenetic activation of chemokine expression.

### EXAMPLE 8

### The activity of UMLILO can be substituted with a different WDR5-interacting IncRNA

Both HOTTIP and UMLILO use 'pre-configured' chromatin compaction to direct the WDR5-MLL1 complex in close vicinity of their target genes (Figure 20 and 20; Wang et al. 2011). This reveals that interactions between IncRNAs and WDR5 may be a widely used mechanism of gene activation in 3D nuclear compartments. However, to our knowledge, the interchangeability of *cis*-acting IncRNAs with similar molecular functions has never been functionally explored. Therefore, we hypothesized that exchanging HOTTIP with UMLILO, at its endogenous location within the chemokine TAD, may be sufficient to enable successful activation of the chemokine genes.

We generated a repair template that included the sequence of the HOTTIP IncRNA, with homologous arms that flanked UMLILO (Figure 23). By transfecting cells with the repair template as well as a CRISPR that induced a double strand break near to the transcription start site of UMLILO, we aimed to exploit homologous-directed recombination (HDR) to delete the UMLILO DNA sequence, and replace it with the HOTTIP DNA sequence under the control of the endogenous UMLILO promoter. We then used TNF to induce chemokine expression in cells where the recombination event had successfully occurred. We detected the transcription of HOTTIP using RNA FISH probes that bind to its RNA and related the position of HOTTIP transcription to the chemokine genes. Due to HDR with such a long repair construct being a rare event and as we observed very poor transfection efficiency and repair in the primary HUVECs (data not shown), we performed the genome editing experiments in a well-characterized HeLa cell line. In unstimulated, serum-starved HeLas, HOTTIP FISH spots were observed at approximately 25% of alleles (Figure 22). Importantly, in our HeLa cell line only single allelic expression of HOTTIP was ever observed in each cell. Further, co-localization between HOTTIP and the chemokines was never observed in control transfected cells. Strikingly, despite the lack of antibiotic selection, we observed overlapping HOTTIP with IL8 and/or CXCL2 foci in ∼1% of transfected cells (Figure 24). Notably, in several instances, overlapping foci were observed in cells displaying two HOTTIP foci. Remarkably, this suggests that by exchanging IncRNAs with similar molecular functions in the appropriate genomic location, functional substitution is possible. Here we were able to restore chemokine gene activation by substituting one WDR5-binding IncRNA for another. This is, to our knowledge, the very first observation revealing the interoperability of IncRNAs with similar functions. This striking finding demonstrates that IncRNA function is dependent on both the activity encoded in their RNA structure as well as their expression in the appropriate 3D genomic context.

### Discussion

Here we report UMLILO, a new super-enhancer (SE) resident enhancer-like IncRNA that interacts with WDR5 to coordinate the H3K4me3 epigenetic activation of the highly therapeutically relevant chemokine locus. Many characterized IncRNAs are identified based on their strong upregulation by external stimuli, such as LPS or DNA damage. Subsequently, low copy number IncRNAs that are expressed in resting cells and prior to stimulation receive less attention. Uniquely, UMLILO was identified using a 'guilt-by association' approach, by analyzing all IncRNAs transcribed across the chemokine TAD in unchallenged immune and nonimmune cells. The ubiquitous expression of UMLILO across various cell types reveals that it may be an important regulator of the innate immune response.

The functional relationship between 3D chromatin structure and rapidly responding genes remains opaque. Therefore, we used the chemokine TAD as a model to dissect the temporal events that are required to achieve chemokine gene expression. Chromosomal contacts across the TAD were not influenced by TNF induction of the chemokines, indicating that pre-formed chromosomal contact within the chemokine TAD forms upstream of chemokine transcription. In support of this notion, siRNA depletion of UMLILO did not alter the pre-formed chromosomal contacts demonstrating that the formation of the TAD precedes UMLILO-mediated activity (Figure 20, 21 and 25). Although UMLILO arises from within a SE, its transcription is decoupled from SE function (Figure 3, 9, 10, 11 and 12). This demonstrates that the epigenetic activation of the chemokine may proceed independently of the loading of the SE with transcriptional regulators (Figure 25). Thus, upon activation by TNF, the chemokine SE becomes loaded with BRD4 and other transcriptional regulators, to induce the rapid and robust transcriptional induction of the chemokine genes (Figure 25). Therefore, the sequential combination of all three events; preformation of chromosomal contact; epigenetic priming of chemokines; signal-dependent loading of transcription factors on the SE, is required to achieve chemokine transcriptional activation (Figure 25). Intriguingly, we also show that the activity of UMLILO can be substituted with a different WDR5-interacting enhancer-like IncRNA. This reveals that the pre-configured compartment is a critical factor in constraining the location of *cis*-acting IncRNAs, which in turn, coordinates the activation of genes located within the same compartment. This study is to our knowledge, the first demonstration of the temporal sequence of how 3D nuclear architecture and epigenetic gene priming influence chemokine gene transcription.

Owing to the delays that occur due to the assembly of the preinitiation complex and eviction of inhibitory nucleosomes at the promoter, genes that lack poised Pol II may exhibit more stochastic patterns of gene expression. However, stochasticity in innate immune gene expression may be detrimental to mounting a successful immune response. Therefore, by maintaining the promoters of the innate immune genes in an active state, eukaryotic organisms may have evolved to reduce gene-intrinsic noise. Indeed, the "poised" innate immune genes in humans, such as the CXC chemokines, need to respond immediately to external stimuli, and therefore, exhibit a robust transcriptional response upon activation across nearly all cells (Figure 5). Therefore, we speculate that genes displaying fast, nonstochastic gene expression exhibit a distinct TAD nuclear architecture and may also be assisted in transcriptional activation by enhancer-like IncRNAs such as UMLILO. In addition, they may be located within a TAD that may contain a SE, and engage in pre-formed chromosomal contact with similar epigenetic priming. This preformation of nuclear architecture around the SE may create a domain of IncRNAs, chromatin remodelling proteins and other transcriptional regulators in close vicinity to target genes. As a consequence, target gene promoters would overcome noise associated with stochastic promoter activation, and therefore, upon the arrival of signal-dependent transcription factors would be able to achieve rapid transcriptional activation. Contrastingly, genes that are located in a TAD lacking pre-formed contact, may be subject to dynamic loop-mediated regulation and exhibit stochastic gene expression, evoking spatially variegated gene expression patterns. This suggests that the assembly of co-regulated genes into a pre-formed "inverted rosette" may be a general feature of poised genes, such as immune genes, facilitating their rapid activation.

TNF is an established mediator of sepsis-associated inflammatory processes. Therefore, a detailed understanding of TNF-induced transcriptional responses is vital to develop new therapies that curb the excessive cytokine release that occurs during sepsis. Despite being used as a model for various inflammatory disorders, mice have been reported to be highly resistant to inflammatory stimuli when compared with humans. Indeed, early *in vivo* studies revealed that the lethal dose of LPS in half of the population of mice was 1,000-10,000 times the dose that is associated with septic shock and severe disease in humans. One explanation may be that mice lack several components involved in innate immune signaling; notably IL8 (Figure 8). Furthermore, although mice do possess orthologs for CXCL1, CXCL2 and CXCL3, they possess neutrophil-poor blood when compared to higher vertebrates. A curious observation from this study is the lack of any UMLILO homolog in mice, despite its clear role in human chemokine regulation. Interestingly, despite lacking UMLILO, Cxcl2 and Cxcl5 levels were significantly altered in MII1 (-/-) knockout mice (Wang et al. 2012). As NeST is a IncRNA that acts in *trans* to direct WDR5 and MLL1 to target *IFN-y* in humans and mice (Gomez et al. 2013), we cannot rule out that a *trans*-acting IncRNA regulates chemokine expression in mice. However, as neutrophil chemotaxis is a critical component of innate immune signaling in higher vertebrates, we speculate that *cis*-acting IncRNAs may have evolved to ensure more robust chemokine expression.

Historically, upstream steps that regulate chemokine signal transduction are targeted for therapeutic intervention. Unfortunately, due to broad off-target effects, these approaches have been largely unsuccessful. More recently, agonists targeting the CXC chemokine receptors, CXCR1 and CXCR2, are thought to be a viable option to attenuate chemokine signalling. However, there is concern that these global approaches may lead to immunosuppression upon extended treatment. Therefore, there is a great need to develop highly specific small molecule inhibitors that are able to more discretely influence chemokine transcriptional regulation. As UMLILO is essential to the epigenetic activation of the chemokines, it represents a novel approach to drug the chemokine transcriptional response. Indeed, a small molecule inhibitor that inhibits the WDR5/MLL1 interaction led to a significant reduction in chemokine expression (Figure 19). As aberrant expression of these chemokines and other cytokines underlies multiple disease states, adjustment of chemokine levels by altering the activity of UMLILO, may represent a valuable therapeutic strategy.

### REFERENCES

Andersson, R, et al. (2014). An atlas of active enhancers across human cell types and tissues. Nature 507, 455-461.
Cao, F, et al. (2014). Targeting MLL1 H3K4 methyltransferase activity in mixed-lineage leukemia. Mol Cell. 53, 247-261.
Cong, L, et al. (2013). Multiplex genome engineering using CRISPR/Cas systems. Science 339, 819-23.
Grebien, F, et al. (2015). Pharmacological targeting of the Wdr5-MLL interaction in C/EBPa N-terminal leukemia. Nat Chem Biol. 11, 571-8.
Hagege, H, et al. (2007). Quantitative analysis of chromosome conformation capture assays (3C-qPCR). Nat. Protoc. 2, 1722-1733.
Gomez, J A, et al. (2013). The NeST long ncRNA controls microbial susceptibility and epigenetic activation of the interferon-g locus. Cell 152, 743-754.
Jin, F. (2013). A high-resolution map of the three- dimensional chromatin interactome in human cells. Nature 503, 290-294.
Li, G, et al. (2010). ChIA-PET tool for comprehensive chromatin interaction analysis with paired-end tag sequencing. Genome Biol. 11, R22.
Papantonis, A, et al. (2012). TNFα signals through specialized factories where responsive coding and miRNA genes are transcribed. EMBO J. 31, 4404-4414.
Paulsen, M T, et al. (2013). Coordinated regulation of synthesis and stability of RNA during the acute TNF-induced proinflammatory response. Proc Natl Acad Sci U S A. 110, 2240-5.
Raj, A, et al. (2008). Imaging individual mRNA molecules using multiple singly labeled probes. Nat. Methods 5, 877-879.
Shevchenko, A, et al. (2007). In-gel digestion for mass spectrometric characterization of proteins and proteomes. Nature Protocols. 1, 2856-2860.
Tennakoon, C, et al. (2012). BatMis: a fast algorithm for k-mismatch mapping. Bioinformatics 28, 2122-8.
Wang, K C, et al. (2011). A long non-coding RNA maintains active chromatin to coordinate homeotic gene expression. Nature 472, 120-124.
Wang, X, et al. (2012). MLL1, a H3K4 methyltransferase, regulates the TNFα-stimulated activation of genes downstream of NF-κB. J Cell Sci. 125, 4058-4066.

## Claims

1. An UMLILO IncRNA inhibitor for use in a method of treating an inflammatory condition, an autoimmune disorder or cancer in a subject by decreasing the production of ELR+ proinflammatory chemokines in the subject, the method comprising administering an effective amount of the UMLILO IncRNA inhibitor to the subject, wherein the UMLILO IncRNA is encoded by the sequence provided in SEQ ID NO:1, wherein the UMLILO IncRNA inhibitor is selected from the group consisting of antisense oligonucleotides, miRNAs, siRNAs, piRNAs, snRNAs, CAS9/CRISPR, TALENS, zinc finger nucleases and BUD1 nucleases.

2. The UMLILO IncRNA inhibitor for use of claim 1, wherein the ELR+ proinflammatory chemokines are IL8, CXCL1, CXCL2 and CXCL3.

3. The UMLILO IncRNA inhibitor for use of claim 1 or 2, wherein the siRNA is an siRNA comprising a sequence selected from the group consisting of SEQ ID NOs:54 to 57.

4. The UMLILO IncRNA inhibitor for use of any one of claims 1 to 3, wherein the UMLILO IncRNA inhibitor is provided by a recombinant polynucleotide comprising a promoter operably linked to a polynucleotide encoding the UMLILO IncRNA inhibitor.

5. The UMLILO IncRNA inhibitor for use of claim 4, wherein the UMLILO IncRNA inhibitor is provided by a lentiviral vector-carrying short hairpin RNA (shRNA) and wherein the shRNA comprises a sequence selected from the group consisting of SEQ ID NOs:54 to 57.

6. The UMLILO IncRNA inhibitor for use of any one of claims 1 to 5, wherein the subject displays reduced inflammation after treatment with the UMLILO IncRNA inhibitor.

7. The UMLILO IncRNA inhibitor for use of any one of claims 1 to 6, wherein the inflammatory condition is selected from the group consisting of allergy, acne vulgaris, appendicitis, asthma, atherosclerosis, bursitis, cancer, celiac disease, chronic prostatitis, colitis, cystitis, dermatitis, glomerulonephritis, hay fever, viral infection, bacterial infection, fungal infection, archeal infection, inflammatory bowel disease, pelvic inflammatory disease, periodontitis, phlebitis, reperfusion injury, rhinitis, rheumatoid arthritis, sarcoidosis, sepsis, tendonitis, tonsillitis, transplant rejection, type 1 hypersensitivity and vasculitis.

8. The UMLILO IncRNA inhibitor for use of any one of claims 1 to 6, wherein the autoimmune disorder is selected from the group consisting of acute disseminated encephalomyelitis, acute motor axonal neuropathy, Addison's disease, adiposis dolorosa, adult-onset still's disease, alopecia areata, ankylosing spondylitis, anti-glomerular basement membrane nephritis, anti-neutrophil cytoplasmic antibody-associated vasculitis, anti-N-methyl-D-aspartate receptor encephalitis, antiphospholipid syndrome, antisynthetase syndrome, aplastic anemia, autoimmune angioedema, autoimmune enteropathy, autoimmune hemolytic anemia, autoimmune hepatitis, autoimmune inner ear disease, autoimmune lymphoproliferative syndrome, autoimmune neutropenia, autoimmune oophoritis, autoimmune orchitis, autoimmune pancreatitis, autoimmune polyendocrine syndrome, autoimmune polyendocrine syndrome type 2, autoimmune polyendocrine syndrome type 3, autoimmune progesterone dermatitis, autoimmune retinopathy, autoimmune thrombocytopenic purpura, autoimmune thyroiditis, autoimmune urticarial, autoimmune uveitis, balo concentric sclerosis, Behçet's disease, Bickerstaff's encephalitis, bullous pemphigoid, celiac disease, chronic inflammatory demyelinating polyneuropathy, Churg-Strauss syndrome, cicatricial pemphigoid, Cogan syndrome, cold agglutinin disease, crest syndrome, Crohn's disease, dermatitis herpetiformis, dermatomyositis, diabetes mellitus type 1, discoid lupus erythematosus, drug-induced lupus, endometriosis, enthesitis-related arthritis, eosinophilic fasciitis, epidermolysis bullosa acquisita, erythema nodosum, essential mixed cryoglobulinemia, Evans syndrome, Felty syndrome, fibromyalgia, gestational pemphigoid, giant cell arteritis, Graves' disease, Graves ophthalmopathy, Guillain-Barre syndrome, Hashimoto's encephalopathy, Henoch-Schonlein purpura, hidradenitis suppurativa, idiopathic inflammatory demyelinating diseases, Igg4-related systemic disease, inclusion body myositis, intermediate uveitis, interstitial cystitis, juvenile arthritis, Kawasaki's disease, Lambert-Eaton myasthenic syndrome, leukocytoclastic vasculitis, lichen planus, lichen sclerosus, ligneous conjunctivitis, linear IgA disease, lupus nephritis, lupus vasculitis, Lyme disease, Ménière's disease, microscopic colitis, microscopic polyangiitis, mixed connective tissue disease, Mooren's ulcer, morphea, Mucha-Habermann disease, multiple sclerosis, myasthenia gravis, myocarditis, myositis, neuromyelitis optica, neuromyotonia, opsoclonus myoclonus syndrome, optic neuritis, Ord's thyroiditis, palindromic rheumatism, paraneoplastic cerebellar degeneration, paroxysmal nocturnal hemoglobinuria, Parry Romberg syndrome, Parsonage-Turner syndrome, pediatric autoimmune neuropsychiatric disorder associated with streptococcus, pemphigus vulgaris, pernicious anemia, pityriasis lichenoides et varioliformis acuta, polyarteritis nodosa, polymyalgia rheumatica, polymyositis, postmyocardial infarction syndrome, primary biliary cirrhosis, primary sclerosing cholangitis, progressive inflammatory neuropathy, psoriasis, psoriatic arthritis, pure red cell aplasia, reactive arthritis, relapsing polychondritis, restless leg syndrome, retroperitoneal fibrosis, rheumatic fever, rheumatoid arthritis, rheumatoid vasculitis, sarcoidosis, Schnitzler syndrome, scleritis, Sjogren's syndrome, stiff person syndrome, subacute bacterial endocarditis, Susac's syndrome, Sydenham chorea, sympathetic ophthalmia, systemic lupus erythematosus, systemic scleroderma, thrombocytopenia, Tolosa-Hunt syndrome, transverse myelitis, ulcerative colitis, undifferentiated connective tissue disease, urticarial vasculitis, vasculitis and vitiligo.

9. An UMLILO IncRNA for use in a method of treating an immunodeficiency in a subject by increasing the production of ELR+ proinflammatory chemokines in the subject, the method comprising administering an effective amount of an UMLILO IncRNA to the subject, wherein transcription of the ELR+ proinflammatory chemokines is initiated in a cell when WRD5 is present with the UMLILO IncRNA, wherein the UMLILO IncRNA is encoded by the sequence provided in SEQ ID NO:1.

10. The UMLILO IncRNA for use of claim 9, wherein the ELR+ proinflammatory chemokines are IL8, CXCL1, CXCL2 and CXCL3.

11. The UMLILO IncRNA for use of claim 9 or 10, wherein the UMLILO IncRNA is provided by a recombinant polynucleotide comprising a promoter operably linked to a polynucleotide encoding the UMLILO IncRNA.

12. The UMLILO IncRNA for use of any one of claims 9 to 11, wherein the immunodeficiency results in the subject having an increased risk of a pathogenic infection.

13. The UMLILO IncRNA for use of claim 12, wherein the pathogenic infection is a viral, bacterial, fungal or parasitic infection.

## Patentansprüche

1. UMLILO lncRNA-Inhibitor zur Verwendung in einem Verfahren zur Behandlung eines Entzündungszustands, einer Autoimmunerkrankung oder von Krebs bei einem Patienten durch Verminderung der Produktion von ELR+-proinflammatorischen Chemokinen in dem Patienten, wobei das Verfahren die Verabreichung einer wirksamen Menge des UMLILO lncRNA-Inhibitors an den Patienten umfasst, wobei die UMLILO lncRNA durch die in SEQ-ID NO: 1 bereitgestellte Sequenz kodiert wird, wobei der UMLILO lncRNA-Inhibitor ausgewählt ist aus der Gruppe bestehend aus Antisense-Oligonukleotiden, miRNAs, siRNAs, piRNAs, snRNAs, CAS9/CRISPR, TALENS, Zinkfinger-Nukleasen und BUD1-Nukleasen.

2. UMLILO lncRNA-Inhibitor zur Verwendung nach Anspruch 1, wobei es sich bei den ELR+-proinflammatorischen Chemokinen um IL8, CXCL1, CXCL2 und CXCL3 handelt.

3. UMLILO lncRNA-Inhibitor zur Verwendung nach Anspruch 1 oder 2, wobei es sich bei der siRNA um eine siRNA handelt, die eine Sequenz ausgewählt aus der Gruppe bestehend aus SEQ-ID NO:54 bis 57 umfasst.

4. UMLILO lncRNA-Inhibitor zur Verwendung nach einem der Ansprüche 1 bis 3, wobei der UMLILO lncRNA-Inhibitor durch ein rekombinantes Polynucleotid bereitgestellt wird, das einen Promotor umfasst, der funktionsfähig an ein Polynucleotid gebunden ist, das den UMLILO lncRNA-Inhibitor kodiert.

5. UMLILO lncRNA-Inhibitor zur Verwendung nach Anspruch 4, wobei der UMLILO lncRNA-Inhibitor durch eine lentivirale vektortragende Kurzhaarnadel-RNA (shRNA) bereitgestellt wird und wobei die shRNA eine Sequenz umfasst, die ausgewählt ist aus der Gruppe bestehend aus SEQ-ID NO:54 bis 57.

6. UMLILO lncRNA-Inhibitor zur Verwendung nach einem der Ansprüche 1 bis 5, wobei der Patient nach der Behandlung mit dem UMLILO lncRNA-Inhibitor eine reduzierte Entzündung aufweist.

7. UMLILO lncRNA-Inhibitor zur Verwendung nach einem der Ansprüche 1 bis 6, wobei der entzündliche Zustand ausgewählt ist aus der Gruppe bestehend aus Allergie, Akne vulgaris, Blinddarmentzündung, Asthma, Atherosklerose, Schleimbeutelentzündung, Krebs, Zöliakie, chronischer Prostatitis, Colitis, Blasenentzündung, Dermatitis, Glomerulonephritis, Heuschnupfen, Virusinfektion, bakterielle Infektion, Pilzinfektion, Archaeeninfektion, entzündliche Darmerkrankung, Beckenentzündung, Parodontitis, Phlebitis, Reperfusionsverletzung, Rhinitis, rheumatoide Arthritis, Sarkoidose, Sepsis, Sehnenentzündung, Mandelentzündung, Transplantatabstoßung, Überempfindlichkeit vom Typ 1 und Vaskulitis.

8. UMLILO lncRNA-Inhibitor zur Verwendung nach einem der Ansprüche 1 bis 6, wobei die Autoimmunerkrankung ausgewählt ist aus der Gruppe bestehend aus akuter disseminierter Enzephalomyelitis, akuter motorischer axonaler Neuropathie, Addison-Krankheit, Adiposis dolorosa, Morbus Parkinson im Erwachsenenalter, Alopecia areata, Spondylitis ankylosans, antiglomerulärer Basalmembran-Nephritis, mit antineutrophilen zytoplasmatischen Antikörpern assoziierter Vaskulitis, Anti-N-Methyl-D-Aspartat-Rezeptor-Enzephalitis, Antiphospholipid-Syndrom, Antisynthetase-Syndrom, aplastischer Anämie, Autoimmunangiödem, Autoimmunenteropathie, autoimmunhämolytischer Anämie, Autoimmunhepatitis, Autoimmunerkrankung des Innenohrs, autoimmunem lymphoproliferativem Syndrom, Autoimmun-Neutropenie, Autoimmun-Oophoritis, Autoimmun-Orchitis, Autoimmun-Pankreatitis, autoimmun-polyendokrinem Syndrom, autoimmun-polyendokrinem Syndrom Typ 2, autoimmun-polyendokrinem Syndrom Typ 3, Autoimmun-Progesteron-Dermatitis, autoimmune Retinopathie, autoimmuner thrombozytopenischer Purpura, autoimmuner Thyreoiditis, autoimmuner Urtikaria, autoimmuner Uveitis, konzentrischer Sklerose Baló, Morbus Behçet, Bickerstaff-Enzephalitis, bullösem Pemphigoid, Zöliakie, chronisch inflammatorischer demyelinisierender Polyneuropathie, Churg-Strauss-Syndrom, Narbenpemphigoid, Cogan-Syndrom, Kälteagglutinin-Krankheit, CREST-Syndrom, Morbus Crohn, Dermatitis herpetiformis, Dermatomyositis, Diabetes mellitus Typ 1, diskoidaler Lupus erythematodes, medikamenteninduzierter Lupus, Endometriose, Enthesitis-bezogener Arthritis, eosinophiler Fasziitis, Epidermolysis bullosa acquisita, Erythema nodosum, essentieller gemischter Kryoglobulinämie, Evans-Syndrom, Felty-Syndrom, Fibromyalgie, Gestationspemphigoid, Riesenzellarteriitis, Morbus Basedow, endokriner Orbitopathie, Guillain-Barre-Syndrom, Hashimoto-Enzephalopathie, Purpura Schönlein-Henoch, Hidradenitis suppurativa, idiopathisch entzündlichen demyelinisierenden Krankheiten, Igg4-bezogener Systemerkrankung, Einschlusskörpermyositis, intermediärer Uveitis, interstitieller Zystitis, juveniler Arthritis, Kawasaki-Syndrom, Lambert-Eaton-Myasthenie-Syndrom, leukozytoklastischer Vaskulitis, Flechtenplanus, Flechtensklerosus, Conjunktivitis lignosa, linearer IgA-Krankheit, Lupusnephritis, Lupusvaskulitis, Lyme-Krankheit, Morbus Meniere, mikroskopischer Kolitis, mikroskopischer Polyangiitis, Sharp-Syndrom, Mooren-Geschwür, Morphea, Mucha-Habermann-Krankheit, Multipler Sklerose, Myasthenia gravis, Myokarditis, Myositis, Neuromyelitis optica, Neuromyotonie, Opsoklonus-Myoklonus-Syndrom, Sehnerventzündung, Ord-Thyreoiditis, palindromischem Rheumatismus, paraneoplastischer Kleinhirndegeneration, paroxysmaler nächtlicher Hämoglobinurie, Parry-Romberg-Syndrom, Parsonage-Turner-Syndrom, pädiatrischer neuropsychiatrischer Autoimmunerkrankung im Zusammenhang mit Streptokokken-Infektionen, Pemphigus vulgaris, perniziöser Anämie, Pityriasis lichenoides et varioliformis acuta, Polyarteritis nodosa, Polymyalgia rheumatica, Polymyositis, Postmyokardinfarkt-Syndrom, primär biliärer Zirrhose, primär sklerosierender Cholangitis, progressiver entzündlicher Neuropathie, Psoriasis, psoriatischer Arthritis, erworbener isolierter aplastischer Anämie, reaktiver Arthritis, rezidivierender Polychondritis, Restless-Legs-Syndrom, retroperitonealer Fibrose, rheumatischem Fieber, rheumatoider Arthritis, rheumatischer Vaskulitis, Sarkoidose, Schnitzler-Syndrom, Skleritis, Sjögren-Syndrom, Stiff-Person-Syndrom, subakuter bakterieller Endokarditis, Susac-Syndrom, Chorea minor (Sydenham), sympathischer Ophthalmie, systemischer Lupus erythematodes, systemischer Sklerodermie, Thrombozytopenie, Tolosa-Hunt-Syndrom, transversaler Myelitis, Colitis ulcerosa, undifferenzierter Bindegewebserkrankung, Urtikaria-Vaskulitis, Vaskulitis und Vitiligo.

9. UMLILO lncRNA zur Verwendung in einem Verfahren zur Behandlung einer Immunschwäche bei einem Patienten durch Erhöhung der Produktion von ELR+-proinflammatorischen Chemokinen bei dem Patienten, wobei das Verfahren die Verabreichung einer wirksamen Menge einer UMLILO lncRNA an den Patienten umfasst, wobei die Transkription der ELR+-proinflammatorischen Chemokine in einer Zelle initiiert wird, wenn WRD5 mit der UMLILO lncRNA vorliegt, wobei die UMLILO lncRNA durch die in SEQ-ID NO:1 bereitgestellte Sequenz kodiert wird.

10. UMLILO lncRNA zur Verwendung nach Anspruch 9, wobei es sich bei den ELR+-proinflammatorischen Chemokinen um IL8, CXCL1, CXCL2 und CXCL3 handelt.

11. UMLILO lncRNA zur Verwendung nach Anspruch 9 oder 10, wobei die UMLILO lncRNA durch ein rekombinantes Polynucleotid bereitgestellt wird, das einen Promotor umfasst, der funktionsfähig mit einem Polynucleotid verbunden ist, das die UMLILO lncRNA kodiert.

12. UMLILO lncRNA zur Verwendung nach einem der Ansprüche 9 bis 11, wobei die Immunschwäche dazu führt, dass der Patient ein erhöhtes Risiko einer pathogenen Infektion aufweist.

13. UMLILO lncRNA zur Verwendung nach Anspruch 12, wobei es sich bei der pathogenen Infektion um eine virale, bakterielle, pilzliche oder parasitäre Infektion handelt.

## Revendications

1. Inhibiteur d'ARNlnc UMLILO destiné à être utilisé dans une méthode de traitement d'une affection inflammatoire, d'un trouble auto-immun ou d'un cancer chez un sujet par diminution de la production de chimiokines pro-inflammatoires ELR+ chez le sujet, la méthode comprenant l'administration d'une quantité efficace de l'inhibiteur d'ARNlnc UMLILO au sujet, l'ARNlnc UMILO étant codé par la séquence fournie dans SEQ ID n° : 1, l'inhibiteur d'ARNlnc UMLILO étant choisi dans le groupe constitué par les oligonucléotides antisens, les miARN, les pARNi, les ARNpi, les pARNn, CAS9/CRISPR, TALENS, les nucléases à doigts de zinc et les nucléases BUD1.

2. Inhibiteur d'ARNlnc UMLILO destiné à être utilisé selon la revendication 1, les chimiokines pro-inflammatoires ELR+ étant IL8, CXCL1, CXCL2 et CXCL3.

3. Inhibiteur d'ARNlnc UMLILO destiné à être utilisé selon la revendication 1 ou 2, le pARNi étant un pARNi comprenant une séquence choisie dans le groupe constitué par les SEQ ID n^{os} : 54 à 57.

4. Inhibiteur d'ARNlnc UMLILO destiné à être utilisé selon l'une quelconque des revendications 1 à 3, l'inhibiteur d'ARNlnc UMLILO étant fourni par un polynucléotide recombiné comprenant un promoteur lié de manière fonctionnelle à un polynucléotide codant pour l'inhibiteur d'ARNlnc UMLILO.

5. Inhibiteur d'ARNlnc UMLILO destiné à être utilisé selon la revendication 4, l'inhibiteur d'ARNlnc UMLILO étant fourni par un vecteur lentiviral portant un ARN court en épingle à cheveux (ARNsh) et l'ARNsh comprenant une séquence choisie dans le groupe constitué par les SEQ ID n^{os} : 54 à 57.

6. Inhibiteur d'ARNlnc UMLILO destiné à être utilisé selon l'une quelconque des revendications 1 à 5, le sujet présentant une inflammation réduite après traitement avec l'inhibiteur d'ARNlnc UMLILO.

7. Inhibiteur d'ARNlnc UMLILO destiné à être utilisé selon l'une quelconque des revendications 1 à 6, l'affection inflammatoire étant choisie dans le groupe constitué par une allergie, l'acné vulgaire, une appendicite, l'asthme, l'athérosclérose, une bursite, un cancer, la maladie cœliaque, une prostatite chronique, une colite, une cystite, une dermatite, la glomérulonéphrite, le rhume des foins, une infection virale, une infection bactérienne, une infection fongique, une infection par des archées, une maladie intestinale inflammatoire, une maladie pelvienne inflammatoire, une parodontite, une phlébite, une lésion de reperfusion, une rhinite, la polyarthrite rhumatoïde, la sarcoïdose, le sepsis, une tendinite, une amygdalite, un rejet de greffe, une hypersensibilité de type 1 et une vascularite.

8. Inhibiteur d'ARNlnc UMLILO destiné à être utilisé selon l'une quelconque des revendications 1 à 6, le trouble auto-immun étant choisi dans le groupe constitué par l'encéphalomyélite aiguë disséminée, une neuropathie axonale motrice aiguë, la maladie d'Addison, l'adipose douloureuse, la maladie de Still de l'adulte, la pelade, la spondylarthrite ankylosante, une néphrite anti-membrane basale glomérulaire, une vascularite associée aux anticorps anti-cytoplasme des neutrophiles, l'encéphalite à anticorps anti-récepteur N-méthyl-D-aspartate, le syndrome des antiphospholipides, le syndrome des anti-synthétases, l'anémie aplasique, un angio-œdème auto-immun, une entéropathie auto-immune, l'anémie hémolytique auto-immune, une hépatite auto-immune, une maladie de l'oreille interne auto-immune, un syndrome lymphoprolifératif auto-immun, une neutropénie auto-immune, une ovarite auto-immune, une orchite auto-immune, une pancréatite auto-immune, un syndrome polyendocrinien auto-immun, un syndrome polyendocrinien auto-immun de type 2, un syndrome polyendocrinien auto-immun de type 3, une dermatite auto-immune à la progestérone, une rétinopathie auto-immune, le purpura thrombopénique auto-immun, une thyroïdite auto-immune, un urticaire auto-immun, une uvéite auto-immune, la sclérose concentrique de Balo, la maladie de Behçet, l'encéphalite de Bickerstaff, la pemphigoïde bulleuse, la maladie cœliaque, une polyneuropathie démyélinisante inflammatoire chronique, le syndrome de Churg-Strauss, la pemphigoïde cicatricielle, le syndrome de Cogan, la maladie des agglutinines froides, le syndrome CREST, la maladie de Crohn, la dermatite herpétiforme, la dermatomyosite, le diabète sucré de type 1, le lupus érythémateux discoïde, un lupus d'origine médicamenteuse, une endométriose, l'arthrite liée à une enthésite, la fasciite à éosinophiles, l'épidermolyse bulleuse acquise, l'érythème noueux, une cryoglobulinémie mixte essentielle, le syndrome d'Evans, le syndrome de Felty, une fibromyalgie, la pemphigoïde de la grossesse, l'artérite à cellules géantes, la maladie de Graves, l'ophtalmopathie de Graves, le syndrome de Guillain-Barré, l'encéphalopathie de Hashimoto, le purpura de Henoch-Schonlein, l'hidradénite suppurée, les maladies démyélinisantes inflammatoires idiopathiques, une maladie systémique associée aux IgG4, une myosite à corps d'inclusion, une uvéite intermédiaire, la cystite interstitielle, l'arthrite juvénile, la maladie de Kawasaki, le syndrome myasthénique de Lambert-Eaton, une vascularite leucocytoclasique, le lichen plan, le lichen scléreux, la conjonctivite ligneuse, une maladie à IgA linéaire, une néphrite lupique, une vascularite lupique, la maladie de Lyme, la maladie de Ménière, une colite microscopique, la polyangéite microscopique, une maladie mixte du tissu conjonctif, l'ulcère de Mooren, la morphée, la maladie de Mucha-Habermann, la sclérose en plaques, la myasthénie grave, une myocardite, une myosite, la neuromyélite optique, une neuromyotonie, le syndrome opsomyoclonique, une névrite optique, la thyroïdite d'Ord, le rhumatisme palindromique, la dégénérescence cérébelleuse paranéoplasique, l'hémoglobinurie paroxystique nocturne, le syndrome de Parry-Romberg, le syndrome de Parsonage-Turner, un trouble neuropsychiatrique auto-immun pédiatrique associé à une infection à streptocoque, le pemphigus vulgaire, l'anémie pernicieuse, le pityriasis lichénoïde et varioliforme aigu, la polyartérite noueuse, une polymyalgie rhumatismale, une polymyosite, un syndrome post-infarctus du myocarde, la cirrhose biliaire primitive, la cholangite sclérosante primitive, une neuropathie inflammatoire progressive, le psoriasis, l'arthrite psoriasique, l'aplasie pure des globules rouges, une arthrite réactionnelle, la polychondrite récidivante, le syndrome des jambes sans repos, une fibrose rétropéritonéale, une fièvre rhumatismale, la polyarthrite rhumatoïde, une vascularite rhumatoïde, la sarcoïdose, le syndrome de Schnitzler, une sclérite, le syndrome de Sjögren, le syndrome de l'homme raide, une endocardite bactérienne subaiguë, le syndrome de Susac, la chorée de Sydenham, l'ophtalmie sympathique, le lupus érythémateux disséminé, une sclérodermie systémique, une thrombocytopénie, le syndrome de Tolosa-Hunt, la myélite transverse, la rectocolite hémorragique, une maladie du tissu conjonctif non différenciée, une vascularite urticarienne, une vascularite et le vitiligo.

9. ARNlnc UMLILO destiné à être utilisé dans une méthode de traitement d'une immunodéficience chez un sujet par augmentation de la production de chimiokines pro-inflammatoires ELR+ chez le sujet, la méthode comprenant l'administration d'une quantité efficace d'un ARNlnc UMLILO au sujet, la transcription des chimiokines pro-inflammatoires ELR+ étant amorcée dans une cellule lorsque WRD5 est présent avec l'ARNlnc UMLILO, l'ARNlnc UMLILO étant codé par la séquence fournie dans SEQ ID n° : 1.

10. ARNlnc UMLILO destiné à être utilisé selon la revendication 9, les chimiokines pro-inflammatoires ELR+ étant IL8, CXCL1, CXCL2 et CXCL3.

11. ARNlnc UMLILO destiné à être utilisé selon la revendication 9 ou 10, l'ARNlnc UMLILO étant fourni par un polynucléotide recombiné comprenant un promoteur lié de manière fonctionnelle à un polynucléotide codant pour l'ARNlnc UMLILO.

12. ARNlnc UMLILO destiné à être utilisé selon l'une quelconque des revendications 9 à 11, l'immunodeficience résultant en ce que le sujet a un risque accru d'infection par un pathogène.

13. ARNlnc UMLILO destiné à être utilisé selon la revendication 12, l'infection par un pathogène étant une infection virale, bactérienne, fongique ou parasitaire.


### SEQUENCE LISTING

<110> CSIR
<120> IMMUNOMODULATION BY CONTROLLING ELR+ PROINFLAMMATORY CHEMOKINE LEVELS WITH THE LONG NON-CODING RNA UMLILO
<130> PA162147/PCT
<150> ZA 2014/09351
   <151> 2014-12-18
<160> 314
<170> PatentIn version 3.5
<210> 1
   <211> 575
   <212> DNA
   <213> Homo sapiens
<400> 1
   atacatgtgg agattaagac ccataataac aatgacaaca ctttcataac agttcatctg 60
   tgttaacata caaattctcg cagcaacact ccagggcgct ttatgtgtgg atctttttta 120
   gtctgcatat taaccctaca agttggaaat ggctcctctc aaacactgga gatagagcag 180
   cccaaatgta tctgctactg tggtgccttc cataatgcaa aactctctga ggagctgaga 240
   atatgtctac tgctaccaaa attgtaaccc ccatcatcta gtaaagagtt ggtacacggt 300
   gaacatttgc tgtgggaatg tattctgctt cattccagag gcctgccaat tcttaatctc 360
   actataggct gaagagctgc tcacatagaa tacttgtagt gacttccatt ttcaccagtt 420
   tagatcagtg gacagagaga tgctgaatta ctgctcaaga agtatagatc cacatgcctt 480
   caacttcaga atcttaaatt agaggcgaat gttgagtcta ctaaactgta tagtctgtaa 540
   aggcaggaac tgtatttatc tcagtcatat ttaat 575
<210> 2
   <211> 20
   <212> DNA
   <213> Artificial sequence
<220>
   <223> UMLILO intron 1 qPCR forward primer
<400> 2
   ggaaggaggg gaacatggaa 20
<210> 3
   <211> 20
   <212> DNA
   <213> Artificial sequence
<220>
   <223> UMLILO intron 1 qPCR reverse primer
<400> 3
   ctacctgact ccctccctct 20
<210> 4
   <211> 21
   <212> DNA
   <213> Artificial sequence
<220>
   <223> UMLILO exon 3 qPCR forward primer
<400> 4
   ggtacacggt gaacatttgc t 21
<210> 5
   <211> 21
   <212> DNA
   <213> Artificial sequence
<220>
   <223> UMLILO exon 3 qPCR reverse primer
<400> 5
   cagcatctct ctgtccactg a 21
<210> 6
   <211> 20
   <212> DNA
   <213> Artificial sequence
<220>
   <223> IL8 qPCR forward primer
<400> 6
   agggccaaga gaatatccga 20
<210> 7
   <211> 20
   <212> DNA
   <213> Artificial sequence
<220>
   <223> IL8 qPCR reverse primer
<400> 7
   ggacttgtgg atcctggcta 20
<210> 8
   <211> 20
   <212> DNA
   <213> Artificial sequence
<220>
   <223> CXCL1 gene qPCR forward primer
<400> 8
   agcttgcctc aatcctgcat 20
<210> 9
   <211> 20
   <212> DNA
   <213> Artificial sequence
<220>
   <223> CXCL1 gene qPCR reverse primer
<400> 9
   cctcctccct tctggtcagt 20
<210> 10
   <211> 24
   <212> DNA
   <213> Artificial sequence
<220>
   <223> CXCL2 gene qPCR forward primer
<400> 10
   cacagtgtgt ggtcaacatt tctc 24
<210> 11
   <211> 23
   <212> DNA
   <213> Artificial sequence
<220>
   <223> CXCL2 gene qPCR reverse primer
<400> 11
   acacagaggg aaacactgca taa 23
<210> 12
   <211> 41
   <212> DNA
   <213> Artificial sequence
<220>
   <223> Repair construct forward primer
<400> 12
   ttgaaccggg ttttccagtc acatatggtg agcaagggcg a 41
<210> 13
   <211> 38
   <212> DNA
   <213> Artificial sequence
<220>
   <223> Repair construct reverse primer
<400> 13
   actatgaaga ctcttgggtc acttgtacag ctcgtcca 38
<210> 14
   <211> 20
   <212> DNA
   <213> Artificial sequence
<220>
   <223> ChIP IL8 forward primer
<400> 14
   tgggccatca gttgcaaatc 20
<210> 15
   <211> 20
   <212> DNA
   <213> Artificial sequence
<220>
   <223> ChIP IL8 reverse primer
<400> 15
   agtgagatgg ttccttccgg 20
<210> 16
   <211> 20
   <212> DNA
   <213> Artificial sequence
<220>
   <223> ChIP CXCL1 forward primer
<400> 16
   acgtgggtct aagggatctg 20
<210> 17
   <211> 20
   <212> DNA
   <213> Artificial sequence
<220>
   <223> ChIP CXCL1 reverse primer
<400> 17
   gggtctgact gtcttgcgta 20
<210> 18
   <211> 20
   <212> DNA
   <213> Artificial sequence
<220>
   <223> ChIP CXCL2 forward primer
<400> 18
   ctgtggtggt tctcagggat 20
<210> 19
   <211> 20
   <212> DNA
   <213> Artificial sequence
<220>
   <223> ChIP CXCL2 reverse primer
<400> 19
   tggactctga gactctggga 20
<210> 20
   <211> 20
   <212> DNA
   <213> Artificial sequence
<220>
   <223> ChIP CXCL3 forward primer
<400> 20
   tctggaatcc gagacgatgg 20
<210> 21
   <211> 20
   <212> DNA
   <213> Artificial sequence
<220>
   <223> ChIP CXCL3 reverse primer
<400> 21
   gacaggaaag gcacgacttc 20
<210> 22
   <211> 20
   <212> DNA
   <213> Artificial sequence
<220>
   <223> ChIP SAMD4A forward primer
<400> 22
   gagctttggg tggagagagt 20
<210> 23
   <211> 20
   <212> DNA
   <213> Artificial sequence
<220>
   <223> ChIP SAMD4A reverse primer
<400> 23
   ttactcctcc tcctcctccc 20
<210> 24
   <211> 22
   <212> DNA
   <213> Artificial sequence
<220>
   <223> ChIP UMLILO forward primer
<400> 24
   tgtccaaatc cacattgaca gt 22
<210> 25
   <211> 20
   <212> DNA
   <213> Artificial sequence
<220>
   <223> ChIP UMLILO reverse primer
<400> 25
   ggagtgttgc tgcgagaatt 20
<210> 26
   <211> 30
   <212> DNA
   <213> Artificial sequence
<220>
   <223> 3C anchor
<400> 26
   tcctctgaca taatgaaaag atgagggtgc 30
<210> 27
   <211> 30
   <212> DNA
   <213> Artificial sequence
<220>
   <223> 3C primer 1
<400> 27
   aaaatagaaa ccctgaatgt accggtaaca 30
<210> 28
   <211> 30
   <212> DNA
   <213> Artificial sequence
<220>
   <223> 3C primer 2
<400> 28
   tcaaatccgt gatcagcatt accaagccat 30
<210> 29
   <211> 28
   <212> DNA
   <213> Artificial sequence
<220>
   <223> 3C primer 3
<400> 29
   attggaaggc taaatatact tacatggc 28
<210> 30
   <211> 27
   <212> DNA
   <213> Artificial sequence
<220>
   <223> 3C primer 4
<400> 30
   ttttaaaagc agcactagtg tatccgg 27
<210> 31
   <211> 30
   <212> DNA
   <213> Artificial sequence
<220>
   <223> 3C primer 5
<400> 31
   aaagagctca atgtgtgtta ctaaagaatg 30
<210> 32
   <211> 29
   <212> DNA
   <213> Artificial sequence
<220>
   <223> 3C primer 6
<400> 32
   aaaggtaagc aataattggc ccatatctc 29
<210> 33
   <211> 30
   <212> DNA
   <213> Artificial sequence
<220>
   <223> 3C primer 7
<400> 33
   aaaagtgata catgttcatt gcatttaaac 30
<210> 34
   <211> 26
   <212> DNA
   <213> Artificial sequence
<220>
   <223> 3C primer 8
<400> 34
   cgtaaggctg gtcaaggtat gctgag 26
<210> 35
   <211> 30
   <212> DNA
   <213> Artificial sequence
<220>
   <223> 3C primer 9
<400> 35
   ttcttcatta tgctagtgtt gtgtattgtg 30
<210> 36
   <211> 30
   <212> DNA
   <213> Artificial sequence
<220>
   <223> 3C primer 10
<400> 36
   tgaaattaga gaaaaacatg tacttaggga 30
<210> 37
   <211> 26
   <212> DNA
   <213> Artificial sequence
<220>
   <223> 3C primer 11
<400> 37
   caccccgccc atttgatgaa ctgttt 26
<210> 38
   <211> 28
   <212> DNA
   <213> Artificial sequence
<220>
   <223> 3C primer 12
<400> 38
   acttcaaact ccaaactcca ccgatttg 28
<210> 39
   <211> 30
   <212> DNA
   <213> Artificial sequence
<220>
   <223> 3C primer 13
<400> 39
   ccaacatcac tgaagcaaag aaacttggag 30
<210> 40
   <211> 26
   <212> DNA
   <213> Artificial sequence
<220>
   <223> 3C primer 14
<400> 40
   tctgcttgta cgtaggtatg tagatt 26
<210> 41
   <211> 30
   <212> DNA
   <213> Artificial sequence
<220>
   <223> 3C primer 15
<400> 41
   gctagcaacc aactctttaa gaatacagcc 30
<210> 42
   <211> 25
   <212> DNA
   <213> Artificial sequence
<220>
   <223> 3C primer 16
<400> 42
   agaaacacaa cgatacaatg tgaaa 25
<210> 43
   <211> 29
   <212> DNA
   <213> Artificial sequence
<220>
   <223> 3C primer 17
<400> 43
   agtgtgactc caaataacct agtttgcta 29
<210> 44
   <211> 26
   <212> DNA
   <213> Artificial sequence
<220>
   <223> 3C primer 18
<400> 44
   acacagtcat aatcacaacc ccagtc 26
<210> 45
   <211> 25
   <212> DNA
   <213> Artificial sequence
<220>
   <223> 3C primer 19
<400> 45
   tcagactcat gggctcagtt gattc 25
<210> 46
   <211> 30
   <212> DNA
   <213> Artificial sequence
<220>
   <223> 3C primer 20
<400> 46
   ggctgacaca ttatggtctc ccactaaata 30
<210> 47
   <211> 28
   <212> DNA
   <213> Artificial sequence
<220>
   <223> 3C primer 21
<400> 47
   gaaacatgtc aagaggccgt ggacattt 28
<210> 48
   <211> 26
   <212> DNA
   <213> Artificial sequence
<220>
   <223> 3C primer 22
<400> 48
   agtgtgaaac aaaacgagaa gggaag 26
<210> 49
   <211> 30
   <212> DNA
   <213> Artificial sequence
<220>
   <223> 3C primer 23
<400> 49
   aaattatttg ctttaggaag ggaagtagaa 30
<210> 50
   <211> 30
   <212> DNA
   <213> Artificial sequence
<220>
   <223> 3C primer 24
<400> 50
   cataggagag actgcgacag aaattccatt 30
<210> 51
   <211> 30
   <212> DNA
   <213> Artificial sequence
<220>
   <223> 3C primer 25
<400> 51
   ttacaactcc tacaaccgtg cttggtacat 30
<210> 52
   <211> 24
   <212> DNA
   <213> Artificial sequence
<220>
   <223> 3C GAPDH control F1
<400> 52
   tgccaatctc cttgttttct aatg 24
<210> 53
   <211> 23
   <212> DNA
   <213> Artificial sequence
<220>
   <223> 3C GAPDH control F2
<400> 53
   tattccccca ggtttacatg ttc 23
<210> 54
   <211> 21
   <212> RNA
   <213> Artificial sequence
<220>
   <223> UMLILO siRNA 1
<400> 54
   aucuuaaauu agaggcgaau u 21
<210> 55
   <211> 21
   <212> RNA
   <213> Artificial sequence
<220>
   <223> UMLILO siRNA 2
<400> 55
   cauacaaauu cucgcagcau u 21
<210> 56
   <211> 21
   <212> RNA
   <213> Artificial sequence
<220>
   <223> UMLILO siRNA 3
<400> 56
   aagaguuggu acacggugau u 21
<210> 57
   <211> 21
   <212> RNA
   <213> Artificial sequence
<220>
   <223> UMLILO siRNA 4
<400> 57
   gcauauuaac ccuacaaguu u 21
<210> 58
   <211> 20
   <212> DNA
   <213> Artificial sequence
<220>
   <223> IL-8 smFISH probe 1
<400> 58
   ttgtcctaga agcttgtgtg 20
<210> 59
   <211> 20
   <212> DNA
   <213> Artificial sequence
<220>
   <223> IL-8 smFISH probe 2
<400> 59
   cacagtgaga tggttccttc 20
<210> 60
   <211> 20
   <212> DNA
   <213> Artificial sequence
<220>
   <223> IL-8 smFISH probe 3
<400> 60
   cagcttggaa gtcatgttta 20
<210> 61
   <211> 20
   <212> DNA
   <213> Artificial sequence
<220>
   <223> IL-8 smFISH probe 4
<400> 61
   gaaatcagga aggctgccaa 20
<210> 62
   <211> 20
   <212> DNA
   <213> Artificial sequence
<220>
   <223> IL-8 smFISH probe 5
<400> 62
   cttaccttca cacagagctg 20
<210> 63
   <211> 20
   <212> DNA
   <213> Artificial sequence
<220>
   <223> IL-8 smFISH probe 6
<400> 63
   acgctgtagg tcagaaagat 20
<210> 64
   <211> 20
   <212> DNA
   <213> Artificial sequence
<220>
   <223> IL-8 smFISH probe 7
<400> 64
   ggatcacatt tagacatagg 20
<210> 65
   <211> 20
   <212> DNA
   <213> Artificial sequence
<220>
   <223> IL-8 smFISH probe 8
<400> 65
   caagaatagc tttgctatct 20
<210> 66
   <211> 20
   <212> DNA
   <213> Artificial sequence
<220>
   <223> IL-8 smFISH probe 9
<400> 66
   aggatgtttg ttaccaaagc 20
<210> 67
   <211> 20
   <212> DNA
   <213> Artificial sequence
<220>
   <223> IL-8 smFISH probe 10
<400> 67
   ctttatactt tcctaagtgt 20
<210> 68
   <211> 20
   <212> DNA
   <213> Artificial sequence
<220>
   <223> IL-8 smFISH probe 11
<400> 68
   atatatgcat gctacatggt 20
<210> 69
   <211> 20
   <212> DNA
   <213> Artificial sequence
<220>
   <223> IL-8 smFISH probe 12
<400> 69
   tataggtaac cgtggttctc 20
<210> 70
   <211> 20
   <212> DNA
   <213> Artificial sequence
<220>
   <223> IL-8 smFISH probe 13
<400> 70
   ccttgctcaa ctcaatatta 20
<210> 71
   <211> 20
   <212> DNA
   <213> Artificial sequence
<220>
   <223> IL-8 smFISH probe 14
<400> 71
   aggagtggaa ttgtctgagt 20
<210> 72
   <211> 20
   <212> DNA
   <213> Artificial sequence
<220>
   <223> IL-8 smFISH probe 15
<400> 72
   gacaaatctg aggcttgtca 20
<210> 73
   <211> 20
   <212> DNA
   <213> Artificial sequence
<220>
   <223> IL-8 smFISH probe 16
<400> 73
   gggtatcttt aaaccagaca 20
<210> 74
   <211> 20
   <212> DNA
   <213> Artificial sequence
<220>
   <223> IL-8 smFISH probe 17
<400> 74
   aagttataca tgcctggtct 20
<210> 75
   <211> 20
   <212> DNA
   <213> Artificial sequence
<220>
   <223> IL-8 smFISH probe 18
<400> 75
   gcatagaaat tgccttcaga 20
<210> 76
   <211> 20
   <212> DNA
   <213> Artificial sequence
<220>
   <223> IL-8 smFISH probe 19
<400> 76
   gtaggcaagc taagactctc 20
<210> 77
   <211> 20
   <212> DNA
   <213> Artificial sequence
<220>
   <223> IL-8 smFISH probe 20
<400> 77
   gtgataccac agtgttattt 20
<210> 78
   <211> 20
   <212> DNA
   <213> Artificial sequence
<220>
   <223> IL-8 smFISH probe 21
<400> 78
   ggtcaatatt gcataatcct 20
<210> 79
   <211> 20
   <212> DNA
   <213> Artificial sequence
<220>
   <223> IL-8 smFISH probe 22
<400> 79
   tcaacatctt catggtattt 20
<210> 80
   <211> 20
   <212> DNA
   <213> Artificial sequence
<220>
   <223> IL-8 smFISH probe 23
<400> 80
   gttagagttc tttttgtaca 20
<210> 81
   <211> 20
   <212> DNA
   <213> Artificial sequence
<220>
   <223> IL-8 smFISH probe 24
<400> 81
   acaacattga acgacttcct 20
<210> 82
   <211> 20
   <212> DNA
   <213> Artificial sequence
<220>
   <223> IL-8 smFISH probe 25
<400> 82
   cctcagttag ttctttgttt 20
<210> 83
   <211> 20
   <212> DNA
   <213> Artificial sequence
<220>
   <223> IL-8 smFISH probe 26
<400> 83
   tgaatattct cctagccctt 20
<210> 84
   <211> 20
   <212> DNA
   <213> Artificial sequence
<220>
   <223> IL-8 smFISH probe 27
<400> 84
   catgtttcta gtgaactcat 20
<210> 85
   <211> 20
   <212> DNA
   <213> Artificial sequence
<220>
   <223> IL-8 smFISH probe 28
<400> 85
   atttggagac tatggaaggc 20
<210> 86
   <211> 20
   <212> DNA
   <213> Artificial sequence
<220>
   <223> IL-8 smFISH probe 29
<400> 86
   ctacttcctt tctaattcca 20
<210> 87
   <211> 20
   <212> DNA
   <213> Artificial sequence
<220>
   <223> IL-8 smFISH probe 30
<400> 87
   tttatcaaca ggcacagctc 20
<210> 88
   <211> 20
   <212> DNA
   <213> Artificial sequence
<220>
   <223> IL-8 smFISH probe 31
<400> 88
   cacctgttgg gggaaaaagt 20
<210> 89
   <211> 20
   <212> DNA
   <213> Artificial sequence
<220>
   <223> IL-8 smFISH probe 32
<400> 89
   ttagcactcc ttggcaaaac 20
<210> 90
   <211> 20
   <212> DNA
   <213> Artificial sequence
<220>
   <223> IL-8 smFISH probe 33
<400> 90
   gtctttatgc actgacatct 20
<210> 91
   <211> 20
   <212> DNA
   <213> Artificial sequence
<220>
   <223> IL-8 smFISH probe 34
<400> 91
   tttggggtgg aaaggtttgg 20
<210> 92
   <211> 20
   <212> DNA
   <213> Artificial sequence
<220>
   <223> IL-8 smFISH probe 35
<400> 92
   tctcaatcac tctcagttct 20
<210> 93
   <211> 20
   <212> DNA
   <213> Artificial sequence
<220>
   <223> IL-8 smFISH probe 36
<400> 93
   cataatttct gtgttggcgc 20
<210> 94
   <211> 20
   <212> DNA
   <213> Artificial sequence
<220>
   <223> IL-8 smFISH probe 37
<400> 94
   ttccaccttc cttaatttta 20
<210> 95
   <211> 20
   <212> DNA
   <213> Artificial sequence
<220>
   <223> IL-8 smFISH probe 95
<400> 95
   cacctggaac tttcctaaat 20
<210> 96
   <211> 20
   <212> DNA
   <213> Artificial sequence
<220>
   <223> IL-8 smFISH probe 39
<400> 96
   tcatttacta ctgtaatcct 20
<210> 97
   <211> 20
   <212> DNA
   <213> Artificial sequence
<220>
   <223> IL-8 smFISH probe 40
<400> 97
   tagtagttgt ggaagctacc 20
<210> 98
   <211> 20
   <212> DNA
   <213> Artificial sequence
<220>
   <223> IL-8 smFISH probe 41
<400> 98
   aggcataaag tctaagtcca 20
<210> 99
   <211> 20
   <212> DNA
   <213> Artificial sequence
<220>
   <223> IL-8 smFISH probe 42
<400> 99
   gcattcaaat catgattcct 20
<210> 100
   <211> 20
   <212> DNA
   <213> Artificial sequence
<220>
   <223> IL-8 smFISH probe 43
<400> 100
   tctcttccat cagaaagctt 20
<210> 101
   <211> 20
   <212> DNA
   <213> Artificial sequence
<220>
   <223> IL-8 smFISH probe 44
<400> 101
   aaaacttctc cacaaccctc 20
<210> 102
   <211> 20
   <212> DNA
   <213> Artificial sequence
<220>
   <223> CCL20 smFISH probe 1
<400> 102
   aagcagccag gagcaaactc 20
<210> 103
   <211> 20
   <212> DNA
   <213> Artificial sequence
<220>
   <223> CCL20 smFISH probe 2
<400> 103
   tggagtagca gcactgacat 20
<210> 104
   <211> 20
   <212> DNA
   <213> Artificial sequence
<220>
   <223> CCL20 smFISH probe 3
<400> 104
   ggaaagagcg acacttacct 20
<210> 105
   <211> 20
   <212> DNA
   <213> Artificial sequence
<220>
   <223> CCL20 smFISH probe 4
<400> 105
   atagtctttc ttctgtgcta 20
<210> 106
   <211> 20
   <212> DNA
   <213> Artificial sequence
<220>
   <223> CCL20 smFISH probe 5
<400> 106
   cagttgagct caaggctaaa 20
<210> 107
   <211> 20
   <212> DNA
   <213> Artificial sequence
<220>
   <223> CCL20 smFISH probe 6
<400> 107
   taacagtttt ccatccctta 20
<210> 108
   <211> 20
   <212> DNA
   <213> Artificial sequence
<220>
   <223> CCL20 smFISH probe 7
<400> 108
   tcactacctc ttgtatactt 20
<210> 109
   <211> 20
   <212> DNA
   <213> Artificial sequence
<220>
   <223> CCL20 smFISH probe 8
<400> 109
   caccatgggc agacaacttc 20
<210> 110
   <211> 20
   <212> DNA
   <213> Artificial sequence
<220>
   <223> CCL20 smFISH probe 9
<400> 110
   cagcagtaac tcttctctcc 20
<210> 111
   <211> 20
   <212> DNA
   <213> Artificial sequence
<220>
   <223> CCL20 smFISH probe 10
<400> 111
   gcagagagga aagcctgatc 20
<210> 112
   <211> 20
   <212> DNA
   <213> Artificial sequence
<220>
   <223> CCL20 smFISH probe 11
<400> 112
   cccaaatctg ctgagattga 20
<210> 113
   <211> 20
   <212> DNA
   <213> Artificial sequence
<220>
   <223> CCL20 smFISH probe 12
<400> 113
   tagggatctc taagagccca 20
<210> 114
   <211> 20
   <212> DNA
   <213> Artificial sequence
<220>
   <223> CCL20 smFISH probe 13
<400> 114
   actttggctt tatcctgaag 20
<210> 115
   <211> 20
   <212> DNA
   <213> Artificial sequence
<220>
   <223> CCL20 smFISH probe 14
<400> 115
   ttatatcact cccagaaaca 20
<210> 116
   <211> 20
   <212> DNA
   <213> Artificial sequence
<220>
   <223> CCL20 smFISH probe 15
<400> 116
   tctactggta acattacaca 20
<210> 117
   <211> 20
   <212> DNA
   <213> Artificial sequence
<220>
   <223> CCL20 smFISH probe 16
<400> 117
   tctacatttc aaaatggccc 20
<210> 118
   <211> 20
   <212> DNA
   <213> Artificial sequence
<220>
   <223> CCL20 smFISH probe 17
<400> 118
   agttggggac acaggaggat 20
<210> 119
   <211> 20
   <212> DNA
   <213> Artificial sequence
<220>
   <223> CCL20 smFISH probe 18
<400> 119
   ctattaagtt gggaggtacg 20
<210> 120
   <211> 20
   <212> DNA
   <213> Artificial sequence
<220>
   <223> CCL20 smFISH probe 19
<400> 120
   gggatgttga tgtcaggtat 20
<210> 121
   <211> 20
   <212> DNA
   <213> Artificial sequence
<220>
   <223> CCL20 smFISH probe 20
<400> 121
   aaagttggca agcaatgtcc 20
<210> 122
   <211> 20
   <212> DNA
   <213> Artificial sequence
<220>
   <223> CCL20 smFISH probe 21
<400> 122
   acatgctgta aggtaaggga 20
<210> 123
   <211> 20
   <212> DNA
   <213> Artificial sequence
<220>
   <223> CCL20 smFISH probe 22
<400> 123
   aatacttgga cattcccgag 20
<210> 124
   <211> 20
   <212> DNA
   <213> Artificial sequence
<220>
   <223> CCL20 smFISH probe 23
<400> 124
   gtgttgtgtg actattgagc 20
<210> 125
   <211> 20
   <212> DNA
   <213> Artificial sequence
<220>
   <223> CCL20 smFISH probe 24
<400> 125
   gcatttttag gaatgcacct 20
<210> 126
   <211> 20
   <212> DNA
   <213> Artificial sequence
<220>
   <223> CCL20 smFISH probe 25
<400> 126
   ctggctacta agtattgaga 20
<210> 127
   <211> 20
   <212> DNA
   <213> Artificial sequence
<220>
   <223> CCL20 smFISH probe 26
<400> 127
   tgcttgaagg aacccttatg 20
<210> 128
   <211> 20
   <212> DNA
   <213> Artificial sequence
<220>
   <223> CCL20 smFISH probe 27
<400> 128
   ttcaagaata tgagcagccc 20
<210> 129
   <211> 20
   <212> DNA
   <213> Artificial sequence
<220>
   <223> CCL20 smFISH probe 28
<400> 129
   gtgggatatc ttcagtaaga 20
<210> 130
   <211> 20
   <212> DNA
   <213> Artificial sequence
<220>
   <223> CCL20 smFISH probe 29
<400> 130
   gagaaacaca ggacttctca 20
<210> 131
   <211> 20
   <212> DNA
   <213> Artificial sequence
<220>
   <223> CCL20 smFISH probe 30
<400> 131
   aacagtttgt gtgccgagaa 20
<210> 132
   <211> 20
   <212> DNA
   <213> Artificial sequence
<220>
   <223> CCL20 smFISH probe 31
<400> 132
   ggtcgttgag aaccaatcta 20
<210> 133
   <211> 20
   <212> DNA
   <213> Artificial sequence
<220>
   <223> CCL20 smFISH probe 32
<400> 133
   aatgtcttgg aaacctttgt 20
<210> 134
   <211> 20
   <212> DNA
   <213> Artificial sequence
<220>
   <223> CCL20 smFISH probe 33
<400> 134
   aaccacttag aggaggagtt 20
<210> 135
   <211> 20
   <212> DNA
   <213> Artificial sequence
<220>
   <223> CCL20 smFISH probe 34
<400> 135
   acttatcctg tgattggtat 20
<210> 136
   <211> 20
   <212> DNA
   <213> Artificial sequence
<220>
   <223> CCL20 smFISH probe 35
<400> 136
   aggtattgga tccacatgaa 20
<210> 137
   <211> 20
   <212> DNA
   <213> Artificial sequence
<220>
   <223> CCL20 smFISH probe 36
<400> 137
   gacagcagtc aaagttgctt 20
<210> 138
   <211> 20
   <212> DNA
   <213> Artificial sequence
<220>
   <223> CCL20 smFISH probe 37
<400> 138
   agaatacggt ctgtgtatcc 20
<210> 139
   <211> 20
   <212> DNA
   <213> Artificial sequence
<220>
   <223> CCL20 smFISH probe 38
<400> 139
   cgtgtgaagc ccacaataaa 20
<210> 140
   <211> 20
   <212> DNA
   <213> Artificial sequence
<220>
   <223> CCL20 smFISH probe 39
<400> 140
   gatagcattg atgtcacagc 20
<210> 141
   <211> 20
   <212> DNA
   <213> Artificial sequence
<220>
   <223> CCL20 smFISH probe 40
<400> 141
   agtttctagg tattcctgat 20
<210> 142
   <211> 20
   <212> DNA
   <213> Artificial sequence
<220>
   <223> CCL20 smFISH probe 41
<400> 142
   tttgccacat catttctttc 20
<210> 143
   <211> 20
   <212> DNA
   <213> Artificial sequence
<220>
   <223> CCL20 smFISH probe 42
<400> 143
   aaaggttttg ccttattcca 20
<210> 144
   <211> 20
   <212> DNA
   <213> Artificial sequence
<220>
   <223> CCL20 smFISH probe 43
<400> 144
   actagttccg tttgaccaaa 20
<210> 145
   <211> 20
   <212> DNA
   <213> Artificial sequence
<220>
   <223> CCL20 smFISH probe 44
<400> 145
   tagaaagtga tttttgccct 20
<210> 146
   <211> 20
   <212> DNA
   <213> Artificial sequence
<220>
   <223> CCL20 smFISH probe 45
<400> 146
   atcttcccta ttttcctaat 20
<210> 147
   <211> 20
   <212> DNA
   <213> Artificial sequence
<220>
   <223> CCL20 smFISH probe 46
<400> 147
   ctttctggta gaagatgctc 20
<210> 148
   <211> 20
   <212> DNA
   <213> Artificial sequence
<220>
   <223> CCL20 smFISH probe 47
<400> 148
   agattttact tttggtctca 20
<210> 149
   <211> 20
   <212> DNA
   <213> Artificial sequence
<220>
   <223> CXCL2 smFISH probe 1
<400> 149
   agggaatctc gtgagacagg 20
<210> 150
   <211> 20
   <212> DNA
   <213> Artificial sequence
<220>
   <223> CXCL2 smFISH probe 2
<400> 150
   cattcttgag tgtggctctg 20
<210> 151
   <211> 20
   <212> DNA
   <213> Artificial sequence
<220>
   <223> CXCL2 smFISH probe 3
<400> 151
   gggttgagac aagctttctg 20
<210> 152
   <211> 20
   <212> DNA
   <213> Artificial sequence
<220>
   <223> CXCL2 smFISH probe 4
<400> 152
   tttcttaacc atgggcgatg 20
<210> 153
   <211> 20
   <212> DNA
   <213> Artificial sequence
<220>
   <223> CXCL2 smFISH probe 5
<400> 153
   ctttttcagc atcttttcga 20
<210> 154
   <211> 20
   <212> DNA
   <213> Artificial sequence
<220>
   <223> CXCL2 smFISH probe 6
<400> 154
   cgcctgtgta catggaaatt 20
<210> 155
   <211> 20
   <212> DNA
   <213> Artificial sequence
<220>
   <223> CXCL2 smFISH probe 7
<400> 155
   gtatttctga ccaacagctc 20
<210> 156
   <211> 20
   <212> DNA
   <213> Artificial sequence
<220>
   <223> CXCL2 smFISH probe 8
<400> 156
   actttttagg gggcagacgc 20
<210> 157
   <211> 20
   <212> DNA
   <213> Artificial sequence
<220>
   <223> CXCL2 smFISH probe 9
<400> 157
   taaccctggg ttttcctgat 20
<210> 158
   <211> 20
   <212> DNA
   <213> Artificial sequence
<220>
   <223> CXCL2 smFISH probe 10
<400> 158
   ataatttttt cagtcctgca 20
<210> 159
   <211> 20
   <212> DNA
   <213> Artificial sequence
<220>
   <223> CXCL2 smFISH probe 11
<400> 159
   ccttaatggc aactttgtga 20
<210> 160
   <211> 20
   <212> DNA
   <213> Artificial sequence
<220>
   <223> CXCL2 smFISH probe 12
<400> 160
   tctggcacca gaacagatta 20
<210> 161
   <211> 20
   <212> DNA
   <213> Artificial sequence
<220>
   <223> CXCL2 smFISH probe 13
<400> 161
   aggagggaag aacctgggtg 20
<210> 162
   <211> 20
   <212> DNA
   <213> Artificial sequence
<220>
   <223> CXCL2 smFISH probe 14
<400> 162
   gcagttttac ctacattcac 20
<210> 163
   <211> 20
   <212> DNA
   <213> Artificial sequence
<220>
   <223> CXCL2 smFISH probe 15
<400> 163
   tccaatgaga ccttacatga 20
<210> 164
   <211> 20
   <212> DNA
   <213> Artificial sequence
<220>
   <223> CXCL2 smFISH probe 16
<400> 164
   cacttcagca caactggcaa 20
<210> 165
   <211> 20
   <212> DNA
   <213> Artificial sequence
<220>
   <223> CXCL2 smFISH probe 17
<400> 165
   gtgtttttta acaggtggaa 20
<210> 166
   <211> 20
   <212> DNA
   <213> Artificial sequence
<220>
   <223> CXCL2 smFISH probe 18
<400> 166
   ggaactaggg gaaatgaagc 20
<210> 167
   <211> 20
   <212> DNA
   <213> Artificial sequence
<220>
   <223> CXCL2 smFISH probe 19
<400> 167
   gcttttcagg tctgaaagtc 20
<210> 168
   <211> 20
   <212> DNA
   <213> Artificial sequence
<220>
   <223> CXCL2 smFISH probe 20
<400> 168
   tttacaaaaa accagggggt 20
<210> 169
   <211> 20
   <212> DNA
   <213> Artificial sequence
<220>
   <223> CXCL2 smFISH probe 21
<400> 169
   attgctcttt gtcatactgg 20
<210> 170
   <211> 20
   <212> DNA
   <213> Artificial sequence
<220>
   <223> CXCL2 smFISH probe 22
<400> 170
   ctggctaaaa cagccagagc 20
<210> 171
   <211> 20
   <212> DNA
   <213> Artificial sequence
<220>
   <223> CXCL2 smFISH probe 23
<400> 171
   agtggcagtc tacactgttt 20
<210> 172
   <211> 20
   <212> DNA
   <213> Artificial sequence
<220>
   <223> CXCL2 smFISH probe 24
<400> 172
   tcttttagga atgggggtga 20
<210> 173
   <211> 20
   <212> DNA
   <213> Artificial sequence
<220>
   <223> CXCL2 smFISH probe 25
<400> 173
   tgtgtggcaa ggacctctag 20
<210> 174
   <211> 20
   <212> DNA
   <213> Artificial sequence
<220>
   <223> CXCL2 smFISH probe 26
<400> 174
   aagtgggtac tgcctgtgac 20
<210> 175
   <211> 20
   <212> DNA
   <213> Artificial sequence
<220>
   <223> CXCL2 smFISH probe 27
<400> 175
   gttttctgaa tgcagtttcc 20
<210> 176
   <211> 20
   <212> DNA
   <213> Artificial sequence
<220>
   <223> CXCL2 smFISH probe 28
<400> 176
   cacaacagtc ttctcctgtc 20
<210> 177
   <211> 20
   <212> DNA
   <213> Artificial sequence
<220>
   <223> CXCL2 smFISH probe 29
<400> 177
   taggtgctca ggaaagctga 20
<210> 178
   <211> 20
   <212> DNA
   <213> Artificial sequence
<220>
   <223> CXCL2 smFISH probe 30
<400> 178
   aatgagaaaa tgggtgccct 20
<210> 179
   <211> 20
   <212> DNA
   <213> Artificial sequence
<220>
   <223> CXCL2 smFISH probe 31
<400> 179
   cttctggtca gttggatttg 20
<210> 180
   <211> 20
   <212> DNA
   <213> Artificial sequence
<220>
   <223> CXCL2 smFISH probe 32
<400> 180
   aacagccacc aataagcttc 20
<210> 181
   <211> 20
   <212> DNA
   <213> Artificial sequence
<220>
   <223> CXCL2 smFISH probe 33
<400> 181
   ctcttctgtt cctgtaaggg 20
<210> 182
   <211> 20
   <212> DNA
   <213> Artificial sequence
<220>
   <223> CXCL2 smFISH probe 34
<400> 182
   aacacattag gcgcaatcca 20
<210> 183
   <211> 20
   <212> DNA
   <213> Artificial sequence
<220>
   <223> CXCL2 smFISH probe 35
<400> 183
   agacttctcc taagtgatgc 20
<210> 184
   <211> 20
   <212> DNA
   <213> Artificial sequence
<220>
   <223> CXCL2 smFISH probe 36
<400> 184
   gggagagtgt gcaagtagat 20
<210> 185
   <211> 20
   <212> DNA
   <213> Artificial sequence
<220>
   <223> CXCL2 smFISH probe 37
<400> 185
   actaacttgg gtttgaccta 20
<210> 186
   <211> 20
   <212> DNA
   <213> Artificial sequence
<220>
   <223> CXCL2 smFISH probe 38
<400> 186
   ctgcaaacct tctatcttca 20
<210> 187
   <211> 20
   <212> DNA
   <213> Artificial sequence
<220>
   <223> CXCL1 smFISH probe 1
<400> 187
   tcactgactg agcgaggctg 20
<210> 188
   <211> 20
   <212> DNA
   <213> Artificial sequence
<220>
   <223> CXCL1 smFISH probe 2
<400> 188
   acgctcctag ggaagaagag 20
<210> 189
   <211> 20
   <212> DNA
   <213> Artificial sequence
<220>
   <223> CXCL1 smFISH probe 3
<400> 189
   atgttcttgg ggtgaattcc 20
<210> 190
   <211> 20
   <212> DNA
   <213> Artificial sequence
<220>
   <223> CXCL1 smFISH probe 4
<400> 190
   ggacttcacg ttcacacttt 20
<210> 191
   <211> 20
   <212> DNA
   <213> Artificial sequence
<220>
   <223> CXCL1 smFISH probe 5
<400> 191
   ggacttacat gacttcggtt 20
<210> 192
   <211> 20
   <212> DNA
   <213> Artificial sequence
<220>
   <223> CXCL1 smFISH probe 6
<400> 192
   agggaaggga atctcgtgag 20
<210> 193
   <211> 20
   <212> DNA
   <213> Artificial sequence
<220>
   <223> CXCL1 smFISH probe 7
<400> 193
   cattcttgag tgtggctctg 20
<210> 194
   <211> 20
   <212> DNA
   <213> Artificial sequence
<220>
   <223> CXCL1 smFISH probe 8
<400> 194
   caggattgag gcaagctttc 20
<210> 195
   <211> 20
   <212> DNA
   <213> Artificial sequence
<220>
   <223> CXCL1 smFISH probe 9
<400> 195
   attttcttaa ctatggggga 20
<210> 196
   <211> 20
   <212> DNA
   <213> Artificial sequence
<220>
   <223> CXCL1 smFISH probe 10
<400> 196
   taactcacct gttcagcatc 20
<210> 197
   <211> 20
   <212> DNA
   <213> Artificial sequence
<220>
   <223> CXCL1 smFISH probe 11
<400> 197
   tcgcctgtgt acatggaaac 20
<210> 198
   <211> 20
   <212> DNA
   <213> Artificial sequence
<220>
   <223> CXCL1 smFISH probe 12
<400> 198
   cagtatttct gaccaacggc 20
<210> 199
   <211> 20
   <212> DNA
   <213> Artificial sequence
<220>
   <223> CXCL1 smFISH probe 13
<400> 199
   ttttattttt agggggcaca 20
<210> 200
   <211> 20
   <212> DNA
   <213> Artificial sequence
<220>
   <223> CXCL1 smFISH probe 14
<400> 200
   caactaaccc ctgggttttc 20
<210> 201
   <211> 20
   <212> DNA
   <213> Artificial sequence
<220>
   <223> CXCL1 smFISH probe 15
<400> 201
   ataatcccaa tttctagtcc 20
<210> 202
   <211> 20
   <212> DNA
   <213> Artificial sequence
<220>
   <223> CXCL1 smFISH probe 16
<400> 202
   gcaccagagc agtaattatc 20
<210> 203
   <211> 20
   <212> DNA
   <213> Artificial sequence
<220>
   <223> CXCL1 smFISH probe 17
<400> 203
   caggcattgg gaatatcctc 20
<210> 204
   <211> 20
   <212> DNA
   <213> Artificial sequence
<220>
   <223> CXCL1 smFISH probe 18
<400> 204
   cgaaggaaga accagggtgg 20
<210> 205
   <211> 20
   <212> DNA
   <213> Artificial sequence
<220>
   <223> CXCL1 smFISH probe 19
<400> 205
   gttttaccta cattcattgg 20
<210> 206
   <211> 20
   <212> DNA
   <213> Artificial sequence
<220>
   <223> CXCL1 smFISH probe 20
<400> 206
   tactgggcct caaatgaagg 20
<210> 207
   <211> 20
   <212> DNA
   <213> Artificial sequence
<220>
   <223> CXCL1 smFISH probe 21
<400> 207
   aaacagccag aacctgttgc 20
<210> 208
   <211> 20
   <212> DNA
   <213> Artificial sequence
<220>
   <223> CXCL1 smFISH probe 22
<400> 208
   tctccactgt actattggat 20
<210> 209
   <211> 20
   <212> DNA
   <213> Artificial sequence
<220>
   <223> CXCL1 smFISH probe 23
<400> 209
   cttttaggaa tgggggtggg 20
<210> 210
   <211> 20
   <212> DNA
   <213> Artificial sequence
<220>
   <223> CXCL1 smFISH probe 24
<400> 210
   ggacctctaa gcttgggatg 20
<210> 211
   <211> 20
   <212> DNA
   <213> Artificial sequence
<220>
   <223> CXCL1 smFISH probe 25
<400> 211
   tatgacagct gtgctgtgtg 20
<210> 212
   <211> 20
   <212> DNA
   <213> Artificial sequence
<220>
   <223> CXCL1 smFISH probe 26
<400> 212
   tggcaaccaa gtggctactg 20
<210> 213
   <211> 20
   <212> DNA
   <213> Artificial sequence
<220>
   <223> CXCL1 smFISH probe 27
<400> 213
   agagttttcc gaatgcagtt 20
<210> 214
   <211> 20
   <212> DNA
   <213> Artificial sequence
<220>
   <223> CXCL1 smFISH probe 28
<400> 214
   attgcacaac actcttctcc 20
<210> 215
   <211> 20
   <212> DNA
   <213> Artificial sequence
<220>
   <223> CXCL1 smFISH probe 29
<400> 215
   gagtaggtgc tcgggaaagc 20
<210> 216
   <211> 20
   <212> DNA
   <213> Artificial sequence
<220>
   <223> CXCL1 smFISH probe 30
<400> 216
   gcaatgagaa aatgggtgcc 20
<210> 217
   <211> 20
   <212> DNA
   <213> Artificial sequence
<220>
   <223> CXCL1 smFISH probe 31
<400> 217
   cttctggtca gttggatttg 20
<210> 218
   <211> 20
   <212> DNA
   <213> Artificial sequence
<220>
   <223> CXCL1 smFISH probe 32
<400> 218
   ttcaggaaca gccaccagtg 20
<210> 219
   <211> 20
   <212> DNA
   <213> Artificial sequence
<220>
   <223> CXCL1 smFISH probe 33
<400> 219
   ctcttctgtt cctataaggg 20
<210> 220
   <211> 20
   <212> DNA
   <213> Artificial sequence
<220>
   <223> CXCL1 smFISH probe 34
<400> 220
   acacattagg cacaatccag 20
<210> 221
   <211> 20
   <212> DNA
   <213> Artificial sequence
<220>
   <223> CXCL1 smFISH probe 35
<400> 221
   agaagacttc tcctaagcga 20
<210> 222
   <211> 20
   <212> DNA
   <213> Artificial sequence
<220>
   <223> CXCL1 smFISH probe 36
<400> 222
   cccttaatta ttttacacct 20
<210> 223
   <211> 20
   <212> DNA
   <213> Artificial sequence
<220>
   <223> CXCL1 smFISH probe 37
<400> 223
   ataggacagt gtgcaggtag 20
<210> 224
   <211> 20
   <212> DNA
   <213> Artificial sequence
<220>
   <223> CXCL1 smFISH probe 38
<400> 224
   acttggggtt gacatttcaa 20
<210> 225
   <211> 20
   <212> DNA
   <213> Artificial sequence
<220>
   <223> CXCL1 smFISH probe 39
<400> 225
   gcaggctcct cagaaatatt 20
<210> 226
   <211> 20
   <212> DNA
   <213> Artificial sequence
<220>
   <223> CXCL1 smFISH probe 40
<400> 226
   ctatcacagt ggctggcatg 20
<210> 227
   <211> 20
   <212> DNA
   <213> Artificial sequence
<220>
   <223> CXCL1 smFISH probe 41
<400> 227
   atgatctcat tggccatttg 20
<210> 228
   <211> 20
   <212> DNA
   <213> Artificial sequence
<220>
   <223> eGFP smFISH probe 1
<400> 228
   tcctcgccct tgctcaccat 20
<210> 229
   <211> 20
   <212> DNA
   <213> Artificial sequence
<220>
   <223> eGFP smFISH probe 2
<400> 229
   atgggcacca ccccggtgaa 20
<210> 230
   <211> 20
   <212> DNA
   <213> Artificial sequence
<220>
   <223> eGFP smFISH probe 3
<400> 230
   ttacgtcgcc gtccagctcg 20
<210> 231
   <211> 20
   <212> DNA
   <213> Artificial sequence
<220>
   <223> eGFP smFISH probe 4
<400> 231
   gacacgctga acttgtggcc 20
<210> 232
   <211> 20
   <212> DNA
   <213> Artificial sequence
<220>
   <223> eGFP smFISH probe 5
<400> 232
   ggcatcgccc tcgccctcgc 20
<210> 233
   <211> 20
   <212> DNA
   <213> Artificial sequence
<220>
   <223> eGFP smFISH probe 6
<400> 233
   tcagggtcag cttgccgtag 20
<210> 234
   <211> 20
   <212> DNA
   <213> Artificial sequence
<220>
   <223> eGFP smFISH probe 7
<400> 234
   ttgccggtgg tgcagatgaa 20
<210> 235
   <211> 20
   <212> DNA
   <213> Artificial sequence
<220>
   <223> eGFP smFISH probe 8
<400> 235
   ggtgggccag ggcacgggca 20
<210> 236
   <211> 20
   <212> DNA
   <213> Artificial sequence
<220>
   <223> eGFP smFISH probe 9
<400> 236
   cgtaggtgaa ggtggtcacg 20
<210> 237
   <211> 20
   <212> DNA
   <213> Artificial sequence
<220>
   <223> eGFP smFISH probe 10
<400> 237
   tagcggctga agcactgcac 20
<210> 238
   <211> 20
   <212> DNA
   <213> Artificial sequence
<220>
   <223> eGFP smFISH probe 11
<400> 238
   gtgctgcttc atgtggtcgg 20
<210> 239
   <211> 20
   <212> DNA
   <213> Artificial sequence
<220>
   <223> eGFP smFISH probe 12
<400> 239
   gcatggcgga cttgaagaag 20
<210> 240
   <211> 20
   <212> DNA
   <213> Artificial sequence
<220>
   <223> eGFP smFISH probe 13
<400> 240
   cgctcctgga cgtagccttc 20
<210> 241
   <211> 20
   <212> DNA
   <213> Artificial sequence
<220>
   <223> eGFP smFISH probe 14
<400> 241
   gtcgtccttg aagaagatgg 20
<210> 242
   <211> 20
   <212> DNA
   <213> Artificial sequence
<220>
   <223> eGFP smFISH probe 15
<400> 242
   cggcgcgggt cttgtagttg 20
<210> 243
   <211> 20
   <212> DNA
   <213> Artificial sequence
<220>
   <223> eGFP smFISH probe 16
<400> 243
   gtgtcgccct cgaacttcac 20
<210> 244
   <211> 20
   <212> DNA
   <213> Artificial sequence
<220>
   <223> eGFP smFISH probe 17
<400> 244
   ttcagctcga tgcggttcac 20
<210> 245
   <211> 20
   <212> DNA
   <213> Artificial sequence
<220>
   <223> eGFP smFISH probe 18
<400> 245
   cgtcctcctt gaagtcgatg 20
<210> 246
   <211> 20
   <212> DNA
   <213> Artificial sequence
<220>
   <223> eGFP smFISH probe 19
<400> 246
   agcttgtgcc ccaggatgtt 20
<210> 247
   <211> 20
   <212> DNA
   <213> Artificial sequence
<220>
   <223> eGFP smFISH probe 20
<400> 247
   gtggctgttg tagttgtact 20
<210> 248
   <211> 20
   <212> DNA
   <213> Artificial sequence
<220>
   <223> eGFP smFISH probe 21
<400> 248
   tgtcggccat gatatagacg 20
<210> 249
   <211> 20
   <212> DNA
   <213> Artificial sequence
<220>
   <223> eGFP smFISH probe 22
<400> 249
   accttgatgc cgttcttctg 20
<210> 250
   <211> 20
   <212> DNA
   <213> Artificial sequence
<220>
   <223> eGFP smFISH probe 23
<400> 250
   tgttgtggcg gatcttgaag 20
<210> 251
   <211> 20
   <212> DNA
   <213> Artificial sequence
<220>
   <223> eGFP smFISH probe 24
<400> 251
   agctgcacgc tgccgtcctc 20
<210> 252
   <211> 20
   <212> DNA
   <213> Artificial sequence
<220>
   <223> eGFP smFISH probe 25
<400> 252
   tgttctgctg gtagtggtcg 20
<210> 253
   <211> 20
   <212> DNA
   <213> Artificial sequence
<220>
   <223> eGFP smFISH probe 26
<400> 253
   cacggggccg tcgccgatgg 20
<210> 254
   <211> 20
   <212> DNA
   <213> Artificial sequence
<220>
   <223> eGFP smFISH probe 27
<400> 254
   ctcaggtagt ggttgtcggg 20
<210> 255
   <211> 20
   <212> DNA
   <213> Artificial sequence
<220>
   <223> eGFP smFISH probe 28
<400> 255
   ctttgctcag ggcggactgg 20
<210> 256
   <211> 20
   <212> DNA
   <213> Artificial sequence
<220>
   <223> eGFP smFISH probe 29
<400> 256
   tgatcgcgct tctcgttggg 20
<210> 257
   <211> 20
   <212> DNA
   <213> Artificial sequence
<220>
   <223> eGFP smFISH probe 30
<400> 257
   cacgaactcc agcaggacca 20
<210> 258
   <211> 20
   <212> DNA
   <213> Artificial sequence
<220>
   <223> eGFP smFISH probe 31
<400> 258
   cgagagtgat cccggcggcg 20
<210> 259
   <211> 20
   <212> DNA
   <213> Artificial sequence
<220>
   <223> eGFP smFISH probe 32
<400> 259
   cacttgtaca gctcgtccat 20
<210> 260
   <211> 20
   <212> DNA
   <213> Artificial sequence
<220>
   <223> UMLILO intron 1 smFISH probe 1
<400> 260
   gctgcacata tatttatgtc 20
<210> 261
   <211> 20
   <212> DNA
   <213> Artificial sequence
<220>
   <223> UMLILO intron 1 smFISH probe 2
<400> 261
   gcctagaatg cttatgttgt 20
<210> 262
   <211> 20
   <212> DNA
   <213> Artificial sequence
<220>
   <223> UMLILO intron 1 smFISH probe 3
<400> 262
   ggacagtaca catccatttt 20
<210> 263
   <211> 20
   <212> DNA
   <213> Artificial sequence
<220>
   <223> UMLILO intron 1 smFISH probe 4
<400> 263
   atcttcctct ttatacgggg 20
<210> 264
   <211> 20
   <212> DNA
   <213> Artificial sequence
<220>
   <223> UMLILO intron 1 smFISH probe 5
<400> 264
   aggtttcatc agtgtttgga 20
<210> 265
   <211> 20
   <212> DNA
   <213> Artificial sequence
<220>
   <223> UMLILO intron 1 smFISH probe 6
<400> 265
   gaactgcaga gtatgttgtt 20
<210> 266
   <211> 20
   <212> DNA
   <213> Artificial sequence
<220>
   <223> UMLILO intron 1 smFISH probe 7
<400> 266
   attctaggta ggaacgatct 20
<210> 267
   <211> 20
   <212> DNA
   <213> Artificial sequence
<220>
   <223> UMLILO intron 1 smFISH probe 8
<400> 267
   agtcttgttt gcacattctt 20
<210> 268
   <211> 20
   <212> DNA
   <213> Artificial sequence
<220>
   <223> UMLILO intron 1 smFISH probe 9
<400> 268
   catatgcctc atttttccaa 20
<210> 269
   <211> 20
   <212> DNA
   <213> Artificial sequence
<220>
   <223> UMLILO intron 1 smFISH probe 10
<400> 269
   tgaagctcat gactgtgcat 20
<210> 270
   <211> 20
   <212> DNA
   <213> Artificial sequence
<220>
   <223> UMLILO intron 1 smFISH probe 11
<400> 270
   caagtgactt agtgttcccc 20
<210> 271
   <211> 20
   <212> DNA
   <213> Artificial sequence
<220>
   <223> UMLILO intron 1 smFISH probe 12
<400> 271
   accagagttc ctttctcatg 20
<210> 272
   <211> 20
   <212> DNA
   <213> Artificial sequence
<220>
   <223> UMLILO intron 1 smFISH probe 13
<400> 272
   ctctgggaaa ggacaggaca 20
<210> 273
   <211> 20
   <212> DNA
   <213> Artificial sequence
<220>
   <223> UMLILO intron 1 smFISH probe 14
<400> 273
   cttagaagtg ttaggatccc 20
<210> 274
   <211> 20
   <212> DNA
   <213> Artificial sequence
<220>
   <223> UMLILO intron 1 smFISH probe 15
<400> 274
   aactagaggc taccaatggc 20
<210> 275
   <211> 20
   <212> DNA
   <213> Artificial sequence
<220>
   <223> UMLILO intron 1 smFISH probe 16
<400> 275
   ttagcggaca aacagttctg 20
<210> 276
   <211> 20
   <212> DNA
   <213> Artificial sequence
<220>
   <223> UMLILO intron 1 smFISH probe 17
<400> 276
   tcagcatcat tttgctgtta 20
<210> 277
   <211> 20
   <212> DNA
   <213> Artificial sequence
<220>
   <223> UMLILO intron 1 smFISH probe 18
<400> 277
   tctcaacaca catcctgagc 20
<210> 278
   <211> 20
   <212> DNA
   <213> Artificial sequence
<220>
   <223> UMLILO intron 1 smFISH probe 19
<400> 278
   tgacaaaacc cagttctgtc 20
<210> 279
   <211> 20
   <212> DNA
   <213> Artificial sequence
<220>
   <223> UMLILO intron 1 smFISH probe 20
<400> 279
   tgggtgtgaa taacggcttg 20
<210> 280
   <211> 20
   <212> DNA
   <213> Artificial sequence
<220>
   <223> UMLILO intron 1 smFISH probe 21
<400> 280
   caaaccttgg cctaggtgac 20
<210> 281
   <211> 20
   <212> DNA
   <213> Artificial sequence
<220>
   <223> UMLILO intron 1 smFISH probe 22
<400> 281
   cctcatttca cagttgaagt 20
<210> 282
   <211> 20
   <212> DNA
   <213> Artificial sequence
<220>
   <223> UMLILO intron 1 smFISH probe 23
<400> 282
   tttctatggc ctcttgattt 20
<210> 283
   <211> 20
   <212> DNA
   <213> Artificial sequence
<220>
   <223> UMLILO intron 1 smFISH probe 24
<400> 283
   ctcatgcagt aatgctgtga 20
<210> 284
   <211> 20
   <212> DNA
   <213> Artificial sequence
<220>
   <223> UMLILO intron 1 smFISH probe 25
<400> 284
   gccagatatt atgctgtttt 20
<210> 285
   <211> 20
   <212> DNA
   <213> Artificial sequence
<220>
   <223> UMLILO intron 1 smFISH probe 26
<400> 285
   aacaagcttt acctgcagtg 20
<210> 286
   <211> 20
   <212> DNA
   <213> Artificial sequence
<220>
   <223> UMLILO intron 1 smFISH probe 27
<400> 286
   cattttgctt tgagaagtcc 20
<210> 287
   <211> 20
   <212> DNA
   <213> Artificial sequence
<220>
   <223> UMLILO intron 1 smFISH probe 28
<400> 287
   gatgtcctga tattagcaga 20
<210> 288
   <211> 20
   <212> DNA
   <213> Artificial sequence
<220>
   <223> UMLILO intron 1 smFISH probe 29
<400> 288
   agagtctatc ttaagctttt 20
<210> 289
   <211> 20
   <212> DNA
   <213> Artificial sequence
<220>
   <223> UMLILO intron 1 smFISH probe 30
<400> 289
   gattttctca ttactacctt 20
<210> 290
   <211> 20
   <212> DNA
   <213> Artificial sequence
<220>
   <223> UMLILO intron 1 smFISH probe 31
<400> 290
   ctccttctag ctattcttta 20
<210> 291
   <211> 20
   <212> DNA
   <213> Artificial sequence
<220>
   <223> UMLILO intron 1 smFISH probe 32
<400> 291
   ctgattttct cattattacc 20
<210> 292
   <211> 20
   <212> DNA
   <213> Artificial sequence
<220>
   <223> UMLILO intron 1 smFISH probe 33
<400> 292
   ccttctagct atcctttaat 20
<210> 293
   <211> 20
   <212> DNA
   <213> Artificial sequence
<220>
   <223> UMLILO intron 1 smFISH probe 34
<400> 293
   cttgttcttt tcctttcatc 20
<210> 294
   <211> 20
   <212> DNA
   <213> Artificial sequence
<220>
   <223> UMLILO intron 1 smFISH probe 35
<400> 294
   gagcgtgtca cacagagtaa 20
<210> 295
   <211> 20
   <212> DNA
   <213> Artificial sequence
<220>
   <223> UMLILO exon smFISH probe 1
<400> 295
   ggtcttaatc tccacatgta 20
<210> 296
   <211> 20
   <212> DNA
   <213> Artificial sequence
<220>
   <223> UMLILO exon smFISH probe 2
<400> 296
   gagtgttgct gcgagaattt 20
<210> 297
   <211> 20
   <212> DNA
   <213> Artificial sequence
<220>
   <223> UMLILO exon smFISH probe 3
<400> 297
   aagatccaca cataaagcgc 20
<210> 298
   <211> 20
   <212> DNA
   <213> Artificial sequence
<220>
   <223> UMLILO exon smFISH probe 4
<400> 298
   gtagggttaa tatgcagact 20
<210> 299
   <211> 20
   <212> DNA
   <213> Artificial sequence
<220>
   <223> UMLILO exon smFISH probe 5
<400> 299
   tgtttgagag gagccatttc 20
<210> 300
   <211> 20
   <212> DNA
   <213> Artificial sequence
<220>
   <223> UMLILO exon smFISH probe 6
<400> 300
   tacatttggg ctgctctatc 20
<210> 301
   <211> 20
   <212> DNA
   <213> Artificial sequence
<220>
   <223> UMLILO exon smFISH probe 7
<400> 301
   tggaaggcac cacagtagca 20
<210> 302
   <211> 20
   <212> DNA
   <213> Artificial sequence
<220>
   <223> UMLILO exon smFISH probe 8
<400> 302
   tcctcagaga gttttgcatt 20
<210> 303
   <211> 20
   <212> DNA
   <213> Artificial sequence
<220>
   <223> UMLILO exon smFISH probe 9
<400> 303
   ggtagcagta gacatattct 20
<210> 304
   <211> 20
   <212> DNA
   <213> Artificial sequence
<220>
   <223> UMLILO exon smFISH probe 10
<400> 304
   actagatgat gggggttaca 20
<210> 305
   <211> 20
   <212> DNA
   <213> Artificial sequence
<220>
   <223> UMLILO exon smFISH probe 11
<400> 305
   aatgttcacc gtgtaccaac 20
<210> 306
   <211> 20
   <212> DNA
   <213> Artificial sequence
<220>
   <223> UMLILO exon smFISH probe 12
<400> 306
   agcagaatac attcccacag 20
<210> 307
   <211> 20
   <212> DNA
   <213> Artificial sequence
<220>
   <223> UMLILO exon smFISH probe 13
<400> 307
   aattggcagg cctctggaat 20
<210> 308
   <211> 20
   <212> DNA
   <213> Artificial sequence
<220>
   <223> UMLILO exon smFISH probe 14
<400> 308
   ttcagcctat agtgagatta 20
<210> 309
   <211> 20
   <212> DNA
   <213> Artificial sequence
<220>
   <223> UMLILO exon smFISH probe 15
<400> 309
   aagtattcta tgtgagcagc 20
<210> 310
   <211> 20
   <212> DNA
   <213> Artificial sequence
<220>
   <223> UMLILO exon smFISH probe 16
<400> 310
   gtccactgat ctaaactggt 20
<210> 311
   <211> 20
   <212> DNA
   <213> Artificial sequence
<220>
   <223> UMLILO exon smFISH probe 17
<400> 311
   agcagtaatt cagcatctct 20
<210> 312
   <211> 20
   <212> DNA
   <213> Artificial sequence
<220>
   <223> UMLILO exon smFISH probe 18
<400> 312
   ggcatgtgga tctatacttc 20
<210> 313
   <211> 20
   <212> DNA
   <213> Artificial sequence
<220>
   <223> UMLILO exon smFISH probe 19
<400> 313
   actcaacatt cgcctctaat 20
<210> 314
   <211> 20
   <212> DNA
   <213> Artificial sequence
<220>
   <223> UMLILO exon smFISH probe 20
<400> 314
   gataaataca gttcctgcct 20